# EUROPEAN PATENT SPECIFICATION

(11) **EP 4 426 270 B1**
(45) Date of publication and mention of the grant of the patent: **23.07.2025**
(21) Application number: 22813505.9
(22) Date of filing: 02.11.2022
(51) Int. Cl.: A61K 9/51, C07D 241/04, A61K 47/24, A61K 47/22, A61K 47/28, C07D 295/15

(54) **LIPID NANOPARTICLES FOR OLIGONUCLEOTIDE DELIVERY**
LIPIDNANOPARTIKEL ZUR OLIGONUKLEOTIDABGABE
NANOPARTICULES LIPIDIQUES POUR L'ADMINISTRATION D'OLIGONUCLÉOTIDES

(30) Priority: 02.11.2021 EP 21205904; 16.06.2022 EP 22179399
(43) Date of publication of application: 11.09.2024
(73) Proprietor: ZIPHIUS NV, 9052 Zwijnaarde (BE)
(72) Inventor: HAQUE, AKM, Ashiqul, 8020 Oostkamp (BE); VALEMBOIS, Sophie, 8020 Oostkamp (BE); MC CAFFERTY, Séan, 8020 Oostkamp (BE); SAHU, Itishri, 8020 Oostkamp (BE); CARDON, Christiaan, 8020 Oostkamp (BE)
(74) Representative: Brantsandpatents bv
(86) International application number: PCT/EP2022/080581
(87) International publication number: WO 2023/078950

(56) References cited:
- WO-A1-2014/028487
- WO-A1-2018/232120
- BLAKNEY ANNA K. ET AL: "The Skin You Are In: Design-of-Experiments Optimization of Lipid Nanoparticle Self-Amplifying RNA Formulations in Human Skin Explants", vol. 13, no. 5, 2 May 2019 (2019-05-02), US, pages 5920 - 5930, XP055905690, ISSN: 1936-0851, Retrieved from the Internet <URL:http://pubs.acs.org/doi/pdf/10.1021/acsnano.9b01774> DOI: 10.1021/acsnano.9b01774

## Description

### FIELD OF THE INVENTION

The present invention relates to novel lipids and lipid nanoparticles (LNPs) comprising these novel lipids/lipidoids in combination with other lipidic components that can be used for the delivery of oligonucleotides, especially for the delivery of RNA such as self-amplifying RNA (saRNA). The present invention also relates to therapeutic products and their use.

### BACKGROUND

There are many challenges associated with the delivery of nucleic acids to effect a desired response in a biological system. The covid-19 pandemic and the vaccines that were developed as a response thereof has shown that nucleic acid based prophylactic vaccines have enormous potential. mRNA or saRNA used as vaccines, or even in a therapeutic context, however, face the problem that they are susceptible to nuclease digestion in plasma, interstitium and lymph. In addition, free RNAs have limited ability to gain access to the intracellular compartment where the relevant translation machinery resides.

Lipid nanoparticles (LNPs) formed from cationic lipids with other lipid components, such as neutral lipids, cholesterol, PEG, PEGylated lipids have been used to prevent degradation of the RNAs in plasma and facilitate the cellular uptake of the oligonucleotides. The currently known LNPs in the art are specifically designed and optimized for the delivery of conventional mRNA or siRNA. Meanwhile, next-generation oligonucleotide-based therapeutics are being developed, that focus on the use of larger RNA constructs such as self-amplifying RNA (saRNA). SaRNA have the advantage that the RNA comprises a mechanism that allows its self-amplification, which in turn allows for the use of a lower concentration of RNA in the therapeutic. Because saRNA is typically a long and negatively charged molecule, it requires a good delivery system. These LNPs must able to protect the oligonucleotides from the action of nucleases and to deliver it into cells by interacting with the negatively charged cell membrane.

The encapsulation of negatively charged RNA in LNPs largely depends on the interaction with positively charged amino lipids. The selection of the lipids thus of the LNP is of major importance as they aid in the entrapment of RNA molecules and in facilitating endosomal escape. As several studies have shown, some cationic lipids with a permanent positive charge seem to be less efficient and more toxic, the use of other lipids is recommended. Lipids or lipid-like compounds according to the invention, will be protonated at low pH, but will display a relatively neutral surface charge at physiological pH. This has two advantages: it prevents nonspecific lipid-protein interactions such as massive binding of charged (macro)molecules in biological fluids (e.g., albumin) and it promotes the endosomal escape of the RNA due to the protonation of the compounds within the acidic environment of the endosomes. It has been shown that acidification leads to the disruption of the membrane and the endosomal escape of the RNA. Hence, these compounds contribute to an efficient encapsulation and delivery of RNA.

LNPs are commonly formulated with two or more further excipients: (i) a sterol, which enhances the stability of the LNP bilayer and promotes membrane fusion; (ii) optionally a phospholipid, which fortifies the LNP bilayer structure and also aids in endosomal escape; and (iii) a lipid-polyethylene glycol (PEG) conjugate, which inserts into the LNP bilayer and provides a PEG coating that reduces LNP aggregation, reduces nonspecific binding of proteins due to sterically hindrance, and reduces nonspecific endocytosis by immune cells.

US 9 439 968 discloses compositions and methods for the preparation, manufacture and therapeutic use of LNPs comprising lipidoids prepared from the conjugate addition of alkylamines to acrylates. Some of said lipidoids comprise the - CH₂CH₂C(=O)OR^{B} moiety, whereby for each of the specified lipidoids R^{B} is a straight chain alkyl. These lipidoids were designed for the delivery of small interfering RNA constructs, i.e. short stranded RNA.

Blakney et al, 2019 as well as WO2018/232120 A1 and WO2014/028487 discuss LNP formulations suited for saRNA. There remains nonetheless a need for other, improved ionizable lipids and lipid nanoparticles for the delivery of oligonucleotides such as RNA and in particular for saRNA or other large RNA constructs. Preferably, these lipid nanoparticles would provide optimal drug: lipid ratios, protect the nucleic acid from degradation and clearance in serum, be suitable for systemic delivery, and provide intracellular delivery of the nucleic acid. In addition, these lipid-nucleic acid particles should be well-tolerated and provide an adequate prophylactic/therapeutic index, such that subject administration/patient treatment at an effective dose of the nucleic acid is not associated with unacceptable toxicity and/or risk to the patient. The present invention provides these and/or related advantages.

### SUMMARY OF THE INVENTION

The present invention and embodiments thereof serve to provide a solution to one or more of above-mentioned problems and meets one or more of the desired characteristics. To this end, the present invention relates to ionizable lipid-like compound according to Formula (I) or pharmaceutically acceptable salt, tautomer, or stereoisomer thereof, 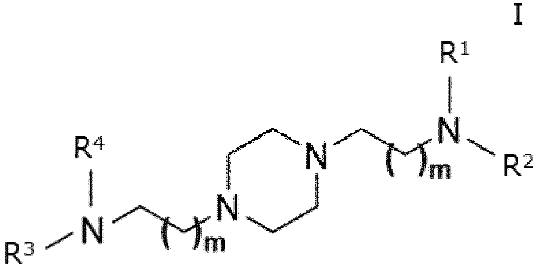 wherein m, R¹, R², R³ and R⁴ are as defined herein.

In a second aspect, the present invention also relates to lipid nanoparticles encapsulating oligonucleotides, in particular RNA, and comprising at least one of the ionizable lipids according to Formula I or pharmaceutically acceptable salt, tautomer, or stereoisomer thereof.

In a further aspect, a pharmaceutical composition, including vaccine, is provided comprising at least one lipid nanoparticle with at least one nucleic acid according to the second aspect. Said pharmaceutical composition or vaccine can be used to treat or prevent disease, such as an infectious disease. Such use comprises administering to a subject an effective amount of an RNA construct encoding a gene of interest, e.g. in the form of a self-replicating RNA molecule, encapsulated or formulated in lipid nanoparticles as described herein, and/or using the composition according to the invention. For example, the invention provides for the use of encapsulated self-replicating RNA molecules of the invention that encode an antigen for inducing an immune response in a subject, or, the use of encapsulated RNA in RNA based protein replacement therapy. The pharmaceutical composition or vaccine can further comprise a pharmaceutically acceptable carrier.

In another aspect, the invention relates to a compound suited for the delivery of an oligonucleotide such as saRNA, wherein said compound is the ionizable lipid-like compound according to Formula (I) as defined in any of the embodiments of the first aspect or a pharmaceutically acceptable salt, tautomer, or stereoisomer thereof.

Further embodiments of the aspects of the invention are provided in the detailed description and claims.

### DEFINITIONS

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "mol%" throughout the description unless otherwise defined, refers to the relative amount of moles of the respective component based on the overall formulation. A mol is defined as exactly 6.02214076×10^23 particles, which may be atoms, molecules, ions, or electrons.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The term "lipid" refers to a group of organic compounds that comprise, but are not limited to, esters of branched or unbranched fatty acids and are generally characterized by being poorly soluble in water, but soluble in many organic solvents. Lipids are usually divided into at least three classes: (1) "simple lipids," which include fats and oils as well as waxes; (2) "compound lipids," which include phospholipids or glycolipids; and (3) "derived lipids" such as steroids.

In the context of the present invention, the term "sterol", also known as steroid alcohol, is a subgroup of steroids that occur naturally in plants, animal and fungi, or can be produced by some bacteria.

The term "neutral lipid" refers to any of a number of lipid species that exist either in an uncharged or neutral zwitterionic form at a selected pH. At physiological pH, such lipids include, but are not limited to, phosphotidylcholines such as 1,2-Distearoyl-sn-glycero-3-phosphocholine (DSPC), 1,2-Dipalmitoyl-sn-glycero-3-phosphocholine (DPPC), 1,2-Dimyristoyl-sn-glycero-3-phosphocholine (DMPC), 1-Palmitoyl-2-oleoyl-sn-glycero-3-phosphocholine (POPC), 1,2-dioleoyl-sn-glycero-3-phosphocholine (DOPC), phophatidylethanolamines such as 1,2-Dioleoyl-sn-glycero-3-phosphoethanolamine (DOPE), sphingomyelins (SM), ceramides, steroids such as sterols and their derivatives. Neutral lipids may be synthetic or naturally derived.

The term "charged lipid" refers to any of a number of lipid species that exist in either a positively charged form, i.e. a "cationic lipid", or negatively charged form, ie. "anionic lipid", at all pH values from pH 3 to pH 9. Charged lipids may be synthetic or naturally derived. Examples of charged lipids include phosphatidylserines, phosphatidic acids, phosphatidylglycerols, phosphatidylinositols, sterol hemisuccinates, dialkyl trimethylammonium-propanes, (e.g. DOTAP, DOTMA), dialkyl dimethylaminopropanes, ethyl phosphocholines, dimethylaminoethane carbamoyl sterols (e.g. DC-Chol).

The term "ionizable" as used herein, for example in "ionizable lipid", "ionizable lipid-like structure" or "ionizable amino lipid" or "ionizable compound" refers to the characteristic that depending on the pH a compound is neutral or charged. Typically in the context of the present invention, the ionizable lipid is an ionizable cationic lipid and comprises (a) primary, secondary or tertiary amino group(s) which is(are) only protonated when exposed to a pH below a certain value. Different nitrogens within a single ionizable amino lipid according to Formula I may be protonated at different pH (i.e. different nitrogens may have a different pKa). Depending on a higher number of ionizable nitrogens in such ionizable cationic lipid, such lipid can have a higher cationic charge available for complexing RNA.

The term "lipid nanoparticle" refers to a particle having at least one dimension in the order of nanometers (e.g., 1-1,000 nm) and comprises a plurality of lipid molecules physically associated with each other by intermolecular forces. The lipid nanoparticles may be, e.g., microspheres (including unilamellar and multilamellar vesicles, e.g., liposomes), a dispersed phase in an emulsion, micelles or an internal phase in a suspension. An active agent or therapeutic agent, such as a nucleic acid, is encapsulated in the lipid portion of the lipid nanoparticle or an aqueous space enveloped by some or all of the lipid portion of the lipid nanoparticle, thereby protecting it from enzymatic degradation or other undesirable effects induced by the mechanisms of the host organism or cells e.g., an adverse immune response.

As used herein, "lipid encapsulated" refers to a lipid nanoparticle that provides an active agent or therapeutic agent, such as a nucleic acid (e.g., mRNA), with full encapsulation or partial encapsulation: the nucleic acid may be fully incorporated within the nanoparticle or (in part) associated with the nanoparticle surface. In an embodiment, the nucleic acid (e.g., saRNA or mRNA) is fully encapsulated in the lipid nanoparticle.

The term "oligonucleotide" or "polynucleotide" as used herein refers to a polymer containing at least two deoxyribonucleotides or ribonucleotides in either single- or double-stranded form and includes DNA, RNA, and hybrids thereof. DNA may be in the form of antisense molecules, plasmid DNA, cDNA, PCR products, or vectors. RNA may be in the form of self-amplifying RNA (saRNA), small hairpin RNA (shRNA), messenger RNA (mRNA), antisense RNA, miRNA, micRNA, multivalent RNA, dicer substrate RNA or viral RNA (vRNA), guide RNA (gRNA), and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (e.g., degenerate codon substitutions), alleles, orthologs, single nucleotide polymorphisms, and complementary sequences as well as the sequence explicitly indicated. Specifically, degenerate codon substitutions may be achieved by generating sequences in which the third position of one or more selected (or all) codons is substituted with mixed-base and/or deoxyinosine residues. "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

As used herein, "buffering agents" include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, d-gluconic acid, calcium glycerophosphate, calcium lactate, calcium lactobionate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, amino-sulfonate buffers (e.g. HEPES), magnesium hydroxide, aluminum hydroxide, alginic acid, pyrogen-free water, isotonic saline, Ringer's solution, ethyl alcohol, and/or combinations thereof.

As used herein, the term "N/P ratio" (or N:P ratio) is the molar ratio of nitrogen atoms in a complexing lipid to phosphate groups in an RNA. This ratio describes the interaction between the cationic charge of the ionized amino (N+) group in the ionizable amino-lipid to the anionic charge of the phosphate (PO4 -) groups in the backbone of nucleic acids and is the basis of the complexation of RNA with the ionizable amino-lipid. To determine the N/P ratio of a lipid nanoparticle containing ionizable lipids such as the lipids of formula I, the number of positively charged nitrogen atoms at the pH at which the LNP is formulated is considered. The N/P ratio of a lipid/nucleic acid (e.g. lipid/RNA) complex can potentially influence other properties such as its net surface charge, size, and stability of the LNP comprising the lipid/nucleic acid complex.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the administrations such as EMA and/or United States Food and Drug Administration as being acceptable for use in humans or domestic animals.

"Pharmaceutically acceptable salt" includes both acid and base addition salts.

A "pharmaceutical composition" refers to a formulation of a compound of the invention and a medium generally accepted in the art for the delivery of the biologically active compound to mammals, e.g., humans. Such a medium includes all pharmaceutically acceptable carriers, diluents or excipients therefor.

"Effective amount" or "therapeutically effective amount" refers to that amount of a compound of the invention, or a lipid nanoparticle comprising the same, which, when administered to an animal, preferably a mammal, more preferably a human, is sufficient to effect treatment in the mammal, preferably a human. The amount of a lipid nanoparticle of the invention which constitutes a "therapeutically effective amount" will vary depending on the compound, the condition and its severity, the manner of administration, and the age of the animal to be treated, but can be determined routinely by one of ordinary skill in the art having regard to his own knowledge and to this disclosure.

The term "self-replicating and "self-amplifying" as used herein are used interchangeably and relate to molecules such as RNA comprising within their sequence specific signals or signature sequences that allow the self-replication or self-amplification of said molecule.

"Treating" or "treatment" as used herein covers the treatment of the disease or condition of interest in a mammal, preferably a human, having the disease or condition of interest, and includes:
(i) preventing from occurring, or reducing the probability of occurrence or the severity (of the symptoms) of the disease or condition in a mammal, in particular, when such mammal is predisposed to the disease or condition but has not yet been diagnosed as having it;
(ii) inhibiting the disease or condition, i.e., slowing down or arresting its development;
(iii) relieving the disease or condition, i.e., causing regression of the disease or condition; or
(iv) relieving the symptoms resulting from the disease or condition, e.g. relieving pain without addressing the underlying disease or condition. Hereby included is thus prophylactic treatment, such as vaccination, whereby disease through infection by a pathogen, e.g. viral or bacterial, is prevented, or, reduced in occurrence and/or severity. As used herein, the terms "disease" and "condition" may be used interchangeably or may be different in that the particular disease or condition may not have a known causative agent (so that etiology has not yet been worked out) and it is therefore not yet recognized as a disease but only as an undesirable condition or syndrome, wherein a more or less specific set of symptoms have been identified by clinicians.

A "stereoisomer" refers to a compound made up of the same atoms bonded by the same bonds but having different three-dimensional structures, which are not interchangeable. Embodiments of the present invention contemplates various stereoisomers and mixtures thereof and includes "enantiomers", which refers to two stereoisomers whose molecules are non-superimposable mirror images of one another.

A "tautomer" refers to a proton shift from one atom of a molecule to another atom of the same molecule. Embodiments of the present invention include tautomers of any said compounds.

"Alkyl" refers to a unbranched (also referred to as straight or linear) or branched hydrocarbon chain radical consisting solely of carbon and hydrogen atoms, which is saturated or unsaturated (i.e., contains one or more double and/or triple bonds), having, for example, from one to four carbon atoms (C1-C4 alkyl), four to twenty carbon atoms (C4-C20 alkyl), six to sixteen carbon atoms (C6-C16 alkyl), six to nine carbon atoms (C6-C9 alkyl), eleven to twenty carbon atoms (C11-C20 alkyl), one to twelve carbon atoms (C1-C12 alkyl), two to six carbon atoms (C2-C6 alkyl) or one to six carbon atoms (C1-C6 alkyl) and which is attached to the rest of the molecule by a single bond, e.g., methyl, ethyl, n-propyl, 1-methylethyl (iso-propyl), n-butyl, n-pentyl, 1,1-dimethylethyl (t-butyl), 3-methylhexyl, 2-methylhexyl, ethenyl, prop-1-enyl, but-1-enyl, pent-1-enyl, penta-1,4-dienyl, ethynyl, propynyl, butynyl, pentynyl, hexynyl, and the like. Unless stated otherwise specifically in the specification, an alkyl group is optionally substituted.

"Branched alkyl" is given its ordinary meaning in the art and typically refers to otherwise straight chain alkyl groups having one or more alkyl substituents. Such alkyl groups thus may comprise a secondary carbon radical (a secondary carbon radical is a carbon radical bound to two other carbon atoms), a tertiary carbon (a tertiary carbon is a carbon atom bound to three other carbon atoms), or a quaternary carbon (a quaternary carbon is a carbon atom bound to four other carbon atoms). In a branched alkyl chain with a secondary carbon radical, the point of attachment of the alkyl chain to the rest of the molecule is not through a carbon atom which is covalently bound to only one other carbon atom. A branched alkyl as used herein can have, for example, from four to thirty carbon atoms (C4-C30 alkyl), wherein the point of attachment to the rest of the molecule is through the second, third, fourth, fifth, sixth, seventh, eighth, ninth, tenth, eleventh, twelfth, thirteenth, fourteenth or fifteenth carbon atom, for example heptadecan-8-yl. A branched alkyl can have, for example, from four to thirty carbon atoms (C4-C30 alkyl), comprising a carbon atom bound to three other carbon atoms. A branched alkyl can have, for example, from four to thirty carbon atoms (C4-C30 alkyl), comprising a carbon atom bound to four other carbon atoms. For example, 3,5,5-trimethyl-hexylphenyl is an alkyl group (hexyl) having three methyl branches (hence, one tertiary and one quaternary carbon) and thus is a branched alkyl bound to a phenyl group. Unless otherwise indicated a branched alkyl includes all isomers thereof.

"Alkylene" or "alkylene chain" refers to a unbranched or branched divalent hydrocarbon chain linking the rest of the molecule to a radical group, consisting solely of carbon and hydrogen, which is saturated or unsaturated (i.e., contains one or more double and/or triple bonds), and having, for example, from one to twenty-four carbon atoms (C1-C24 alkylene), one to fifteen carbon atoms (C1-C15 alkylene),one to twelve carbon atoms (C1-C12 alkylene), one to eight carbon atoms (C1-C8 alkylene), two to six carbon atoms (C2-C6 alkylene), two to four carbon atoms (C2-C4 alkylene), one to two carbon atoms (C1-C2 alkylene), e.g., methylene, ethylene, propylene, n-butylene, ethenylene, propenylene, n-butenylene, propynylene, n-butynylene, and the like. The points of attachment of the alkylene chain to the rest of the molecule and to the radical group can be through one carbon or any two carbons within the chain. Unless stated otherwise specifically in the specification, an alkylene chain may be optionally substituted.

The term "substituted" used herein means any of the above groups (e.g., alkyl, alkylene or heterocyclyl) wherein at least one hydrogen atom (e.g., 1, 2 ,3 or all hydrogen atoms) is replaced by a bond to a non-hydrogen atom such as, but not limited to: a halogen atom such as F, Cl, Br, or I; oxo groups (=O); hydroxyl groups (-OH); C1-C12 alkyl groups; cycloalkyl groups; -(C=O)OR'; -O(C=O)R'; - C(=O)R' ; -OR'; -S(O)ₓR'; -S-SR'; -C(=O)SR'; -SC(=O)R'; -NR'R'; - NR'C(=O)R'; -C(=O)R'; -C(=O)NR'R'; -NR' C(=O)NR'R'; -OC(=O)NR'R'; - NR'C(=O)OR'; -NR'S(O)ₓNR'R'; -NR'S(O)ₓR'; and -S(O)ₓNR'R', wherein: R' is, at each occurrence, independently H, C1-C20 alkyl or cycloalkyl, and x is 0, 1 or 2.

An ester or amide functional group is defined herein as a -C(=O)O- or -OC(=O)- or -NHC(=O)- or -C(=O)NH-. As used herein, the ester or amide functional group can be positioned next to the R^{A} group, according to: -C(=O)OR^{A} or -OC(=O)R^{A} or -NHC(=O)R^{A} or -C(=O)NHR^{A}, or, next to the R^{B} group, according to: -C(=O)OR^{B} or -OC(=O)R^{B} or -NHC(=O)R^{B} or -C(=O)NHR^{B}, or, next to the R^{c} group, according to: -C(=O)OR^{C} or -OC(=O)R^{C} or -NHC(=O)R^{C} or -C(=O)NHR^{C}.

### FIGURES

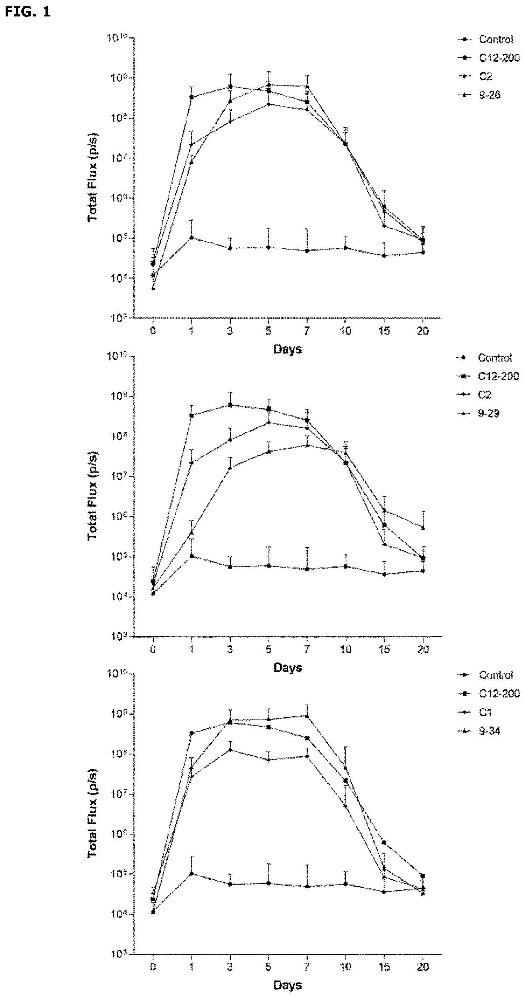

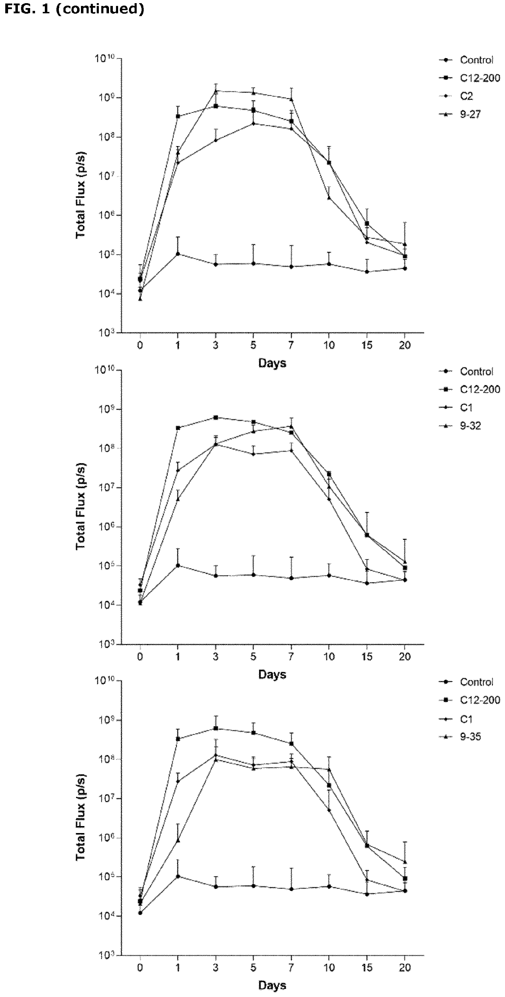

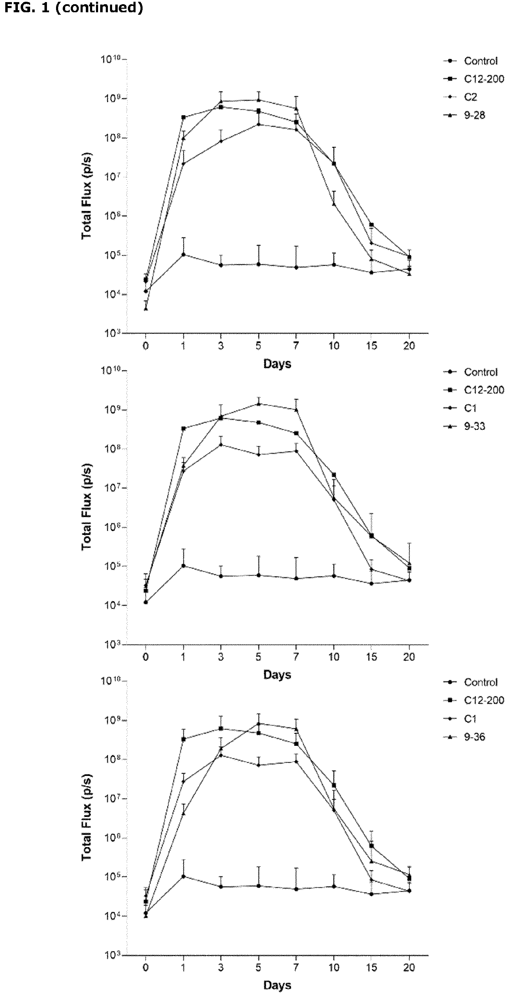

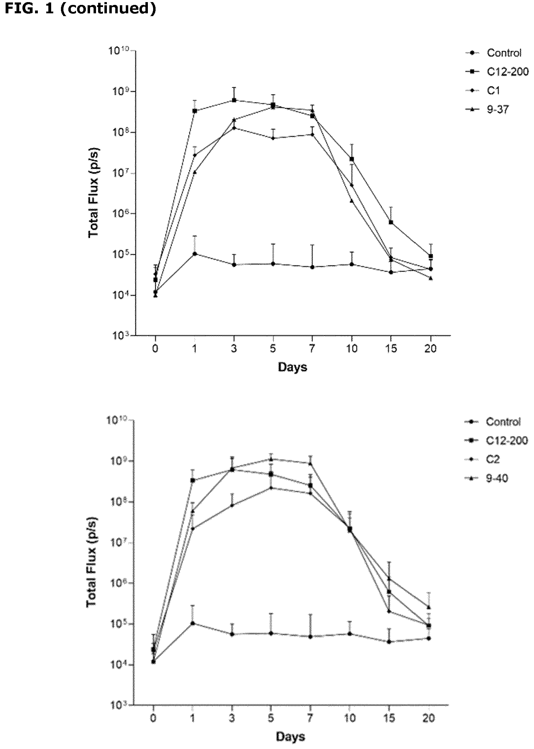

Figure 1 illustrates the *in vivo* expression over time of a luciferase encoding saRNA administered to mice as described in example 2 using exemplary compounds of the invention (triangle graphs), compared to negative control (circle graph), C12-200 (square graph) and comparator compound C1 or C2 (diamond graph).

### DETAILED DESCRIPTION OF THE INVENTION

In a first aspect, the invention relates ionizable lipid-like compounds according to Formula (I) or pharmaceutically acceptable salt, tautomer, or stereoisomer thereof, 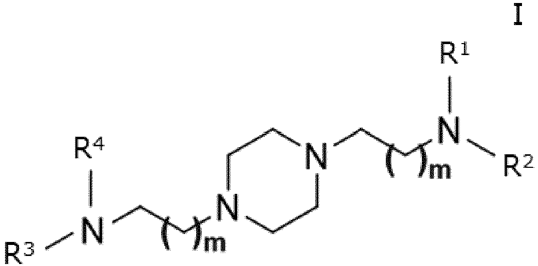 wherein:
each m is, independently, 1, 2, 3, 4 or 5; and
R¹ is -L¹N[L²F¹R^{A}]₂ or -L³F²R^{B} and R², R³ and R⁴ are each -L⁴F³R^{C},
wherein:
   each L¹, L², L³ and L⁴ are, independently, C2-C10 alkylene, each F¹, F² and F³ are, independently, ester or amide functional group and each R^{A}, R^{B} and R^{C} are, independently, branched C4-C30 alkyl.

It was found that the compounds according to formula I, characterized amongst others by a high number of ionizable nitrogens in combination with branched lipidic moieties, allow for efficient encapsulation of RNA molecules in lipid nanoparticles, in particular large RNA constructs (e.g. having 5000nt or more) such as self-amplifying RNA constructs, and that said lipid nanoparticles are proficient in delivering such RNA cargo to the cell.

In an embodiment, for compounds of formula I, each of F¹, F² and F³ is an ester. In an embodiment, for compounds of formula I, each of -F¹R^{A}, -F²R^{B} and -F³R^{C} has the structure -O-C(=O)-R wherein R is R^{A}, R^{B} and R^{C} respectively.

In an embodiment, the ionizable lipid-like compound or pharmaceutically acceptable salt, tautomer, or stereoisomer thereof according to Formula (I) comprises at least one amide functional group. In an embodiment, the ionizable lipid-like compound or pharmaceutically acceptable salt, tautomer, or stereoisomer thereof according to Formula (I) comprises at least two amide functional groups. In an embodiment, the ionizable lipid-like compound or pharmaceutically acceptable salt, tautomer, or stereoisomer thereof according to Formula (I) comprises at least three amide functional groups. In an embodiment, the ionizable lipid-like compound or pharmaceutically acceptable salt, tautomer, or stereoisomer thereof according to Formula (I) comprises at least four amide functional groups.

In an embodiment, the ionizable lipid-like compound or pharmaceutically acceptable salt, tautomer, or stereoisomer thereof according to Formula (I) comprises at least one ester functional group -CH₂C(=O)OR^{A}, wherein R^{A} is the alkyl chain. In an embodiment, the ionizable lipid-like compound or pharmaceutically acceptable salt, tautomer, or stereoisomer thereof according to Formula (I) comprises at least one ester functional group -CH₂OC(=O)R^{A}, wherein R^{A} is the alkyl chain. In an embodiment, the ionizable lipid-like compound or pharmaceutically acceptable salt, tautomer, or stereoisomer thereof according to Formula (I) comprises at least two ester functional groups -CH₂C(=O)OR^{A}, wherein R^{A} is the alkyl chain. In an embodiment, the ionizable lipid-like compound or pharmaceutically acceptable salt, tautomer, or stereoisomer thereof according to Formula (I) comprises at least two ester functional groups -CH₂OC(=O)R^{A}, wherein R^{A} is the alkyl chain. In an embodiment, the ionizable lipid-like compound or pharmaceutically acceptable salt, tautomer, or stereoisomer thereof according to Formula (I) comprises at least three ester functional groups. In an embodiment, the ionizable lipid-like compound or pharmaceutically acceptable salt, tautomer, or stereoisomer thereof according to Formula (I) comprises at least four ester functional groups.

In an embodiment, each branched alkyl side chain R^{A}, R^{B} and R^{C} is a C8-C30 alkyl chain. In an embodiment, each branched alkyl side chain R^{A}, R^{B} and R^{C} is a C8-C23 alkyl chain. In an embodiment, each branched alkyl side chain R^{A}, R^{B} and R^{C} is a C8-C20 alkyl chain. In an embodiment, each branched alkyl side R^{A}, R^{B} and R^{C} is a C11-C30 alkyl chain. In an embodiment, each branched alkyl side chain R^{A}, R^{B} and R^{C} is a C11-C23 alkyl chain. In an embodiment, each branched alkyl side chain R^{A}, R^{B} and R^{C} is a C11-C20 alkyl chain. In an embodiment, each branched alkyl side chain R^{A}, R^{B} and R^{C} is a C13-C30 alkyl chain, a C13-C23 alkyl chain, or a C13-C20 alkyl chain. In an embodiment, each branched alkyl side chain R^{A}, R^{B} and R^{C} is a C15-C30 alkyl chain, a C15-C23 alkyl chain, or a C15-C20 alkyl chain. In an embodiment, each branched alkyl side chain R^{A}, R^{B} and R^{C} is selected from C11, C12, C13, C14, C15, C16, C17, C18, C19 and C20 alkyl. In an embodiment, each branched alkyl side chain R^{A}, R^{B} and R^{C} is selected from C11, C12, C13, C14 and C15 alkyl. In an embodiment, each R^{A}, R^{B} and R^{C} are the same. In an embodiment, R^{A}, R^{B} and R^{C} are unsubstituted branched alkyl groups.

In an embodiment, each branched alkyl side chain R^{A}, R^{B} and R^{C} is, independently, selected from: henicosan-2-yl, docosan-2-yl, tricosan-2-yl, tetracosan-2-yl, pentacosan-2-yl, hexacosan-2-yl, heptacosan-2-yl, octacosan-2-yl, nonacosan-2-yl, triacontan-2-yl, henicosan-3-yl, docosan-3-yl, tricosan-3-yl, tetracosan-3-yl, pentacosan-3-yl, hexacosan-3-yl, heptacosan-3-yl, octacosan-3-yl, nonacosan-3-yl, triacontan-3-yl, henicosan-4-yl, docosan-4-yl, tricosan-4-yl, tetracosan-4-yl, pentacosan-4-yl, hexacosan-4-yl, heptacosan-4-yl, octacosan-4-yl, nonacosan-4-yl, triacontan-4-yl, henicosan-5-yl, docosan-5-yl, tricosan-5-yl, tetracosan-5-yl, pentacosan-5-yl, hexacosan-5-yl, heptacosan-5-yl, octacosan-5-yl, nonacosan-5-yl, triacontan-5-yl, henicosan-6-yl, docosan-6-yl, tricosan-6-yl, tetracosan-6-yl, pentacosan-6-yl, hexacosan-6-yl, heptacosan-6-yl, octacosan-6-yl, nonacosan-6-yl, triacontan-6-yl, henicosan-7-yl, docosan-7-yl, tricosan-7-yl, tetracosan-7-yl, pentacosan-7-yl, hexacosan-7-yl, heptacosan-7-yl, octacosan-7-yl, nonacosan-7-yl, triacontan-7-yl, henicosan-8-yl, docosan-8-yl, tricosan-8-yl, tetracosan-8-yl, pentacosan-8-yl, hexacosan-8-yl, heptacosan-8-yl, octacosan-8-yl, nonacosan-8-yl, triacontan-8-yl, henicosan-9-yl, docosan-9-yl, tricosan-9-yl, tetracosan-9-yl, pentacosan-9-yl, hexacosan-9-yl, heptacosan-9-yl, octacosan-9-yl, nonacosan-9-yl, triacontan-9-yl, henicosan-10-yl, docosan-10-yl, tricosan-10-yl, tetracosan-10-yl, pentacosan-10-yl, hexacosan-10-yl, heptacosan-10-yl, octacosan-10-yl, nonacosan-10-yl, triacontan-10-yl, docosan-11-yl, tricosan-11-yl, tetracosan-11-yl, pentacosan-11-yl, hexacosan-11-yl, heptacosan-11-yl, octacosan-11-yl, nonacosan-11-yl, triacontan-11-yl, tetracosan-12-yl, pentacosan-12-yl, hexacosan-12-yl, heptacosan-12-yl, octacosan-12-yl, nonacosan-12-yl, triacontan-12-yl, hexacosan-13-yl, heptacosan-13-yl, octacosan-13-yl, nonacosan-13-yl, triacontan-13-yl, octacosan-14-yl, nonacosan-14-yl, triacontan-14-yl, triacontan-15-yl, butan-2-yl, pentan-2-yl, hexan-2-yl, heptan-2-yl, octan-2-yl, nonan-2-yl, decan-2-yl, hexan-3-yl, heptan-3-yl, octan-3-yl, nonan-3-yl, decan-3-yl, octan-4-yl, nonan-4-yl, decan-4-yl, decan-5-yl, undecan-2-yl, dodecan-2-yl, tridecan-2-yl, tetradecan-2-yl, pentadecan-2-yl, hexadecan-2-yl, heptadecan-2-yl, octadecan-2-yl, nonadecan-2-yl, eicosan-2-yl, undecan-3-yl, dodecan-3-yl, tridecan-3-yl, tetradecan-3-yl, pentadecan-3-yl, hexadecan-3-yl, heptadecan-3-yl, octadecan-3-yl, nonadecan-3-yl, eicosan-3-yl, undecan-4-yl, dodecan-4-yl, tridecan-4-yl, tetradecan-4-yl, pentadecan-4-yl, hexadecan-4-yl, heptadecan-4-yl, octadecan-4-yl, nonadecan-4-yl, eicosan-4-yl, undecan-5-yl, dodecan-5-yl, tridecan-5-yl, tetradecan-5-yl, pentadecan-5-yl, hexadecan-5-yl, heptadecan-5-yl, octadecan-5-yl, nonadecan-5-yl, eicosan-5-yl, dodecan-6-yl, tridecan-6-yl, tetradecan-6-yl, pentadecan-6-yl, hexadecan-6-yl, heptadecan-6-yl, octadecan-6-yl, nonadecan-6-yl, eicosan-6-yl, tetradecan-7-yl, pentadecan-7-yl, hexadecan-7-yl, heptadecan-7-yl, octadecan-7-yl, nonadecan-7-yl, eicosan-7-yl, hexadecan-8-yl, heptadecan-8-yl, octadecan-8-yl, nonadecan-8-yl, eicosan-8-yl, octadecan-9-yl, nonadecan-9-yl and eicosan-9-yl. In an embodiment, the point of attachment of the C4-C30 alkyl chain (R^{A}, R^{B} and R^{C}) to the rest of the molecule can be through the third, fourth, fifth, sixth, seventh, eighth, ninth, tenth, eleventh, twelfth, thirteenth, fourteenth or fifteenth carbon atom within the alkyl chain. In an embodiment, the lipid nanoparticle comprises at least one ionizable lipid-like structure according to Formula (I) or pharmaceutically acceptable salt, tautomer, or stereoisomer thereof, wherein each R^{A}, R^{B} and R^{C} are, independently, selected from: undecan-5-yl, tridecan-6-yl, pentadecan-7-yl, heptadecan-8-yl, and nonadecan-9-yl. In an embodiment, each branched alkyl side chain R^{A}, R^{B} and R^{C} is selected from: heptadecan-8-yl and nonadecan-9-yl. In an embodiment, each R^{A}, R^{B} and R^{C} are pentadecan-7-yl. In an embodiment, the branched alkyl side chain R^{A}, R^{B} and R^{C} is substituted with one or more alkyl groups. In an embodiment, the branched alkyl side chain R^{A}, R^{B} and R^{C} is substituted with one or more methyl or ethyl groups. In an embodiment, the branched alkyl side chain R^{A}, R^{B} and R^{C} is substituted with any of the isomers of propyl, butyl or pentyl. In an embodiment, the branched alkyl side chain R^{A}, R^{B} and R^{C} is substituted with any of the isomers of hexyl, heptyl, octyl or nonyl. These alkyl chains provide a large apolar zone and good spherical positioning of the lipid-like compounds for the encapsulation of large oligonucleotides.

In an embodiment, each R^{A}, R^{B} and R^{C} are, independently, selected from: undecan-5-yl, tridecan-6-yl, pentadecan-7-yl, heptadecan-8-yl, and nonadecan-9-yl. In an embodiment, each R^{A}, R^{B} and R^{C} are selected from: undecan-5-yl, tridecan-6-yl, pentadecan-7-yl, heptadecan-8-yl, and nonadecan-9-yl. In an embodiment, each R^{A}, R^{B} and R^{C} are pentadecan-7-yl. In an embodiment, each R^{A}, R^{B} and R^{C} are undecan-5-yl. In an embodiment, each R^{A}, R^{B} and R^{C} are tridecan-6-yl. In an embodiment, each R^{A}, R^{B} and R^{C} are pentadecan-7-yl.

In an embodiment, each L¹, L², L³ and L⁴ are, independently, C2-C8 alkylene. In an embodiment, each L¹, L², L³ and L⁴ are, independently, C2-C6 alkylene. In an embodiment, each L¹, L², L³ and L⁴ are, independently, selected from: ethylene, propylene, butylene, pentylene, hexylene, heptylene, octylene, nonylene and decylene. In an embodiment, L¹, L², L³ and/or L⁴ are unsubstituted. In a further embodiment, L¹, L², L³ and/or L⁴ are unsubstituted straight chain alkylene. In an embodiment, L¹, L², L³ and/or L⁴ comprise cyclopropane, cyclobutane, cyclopentane, cyclohexane, cycloheptane or cyclooctane. In an embodiment, L¹, L², L³ and/or L⁴ are substituted with a halogen atom such as F, Cl, Br, or I; oxo groups (=O); hydroxyl groups (-OH); C1-C8 alkyl groups; cycloalkyl groups; -(C=O)OR'; - O(C=O)R'; -C(=O)R' ; -OR'; -S(O)ₓR'; -S-SR'; -C(=O)SR'; -SC(=O)R'; - NR'R'; -NRC(=O)R; -C(=O)R'; -C(=O)NR'R'; -NR' C(=O)NR'R'; - OC(=O)NR'R'; -NR'C(=O)OR'; -NR'S(O)ₓNR'R'; -NR'S(O)ₓR'; and/or - S(O)ₓNR'R'.

In an embodiment, -R², -R³, -R⁴ are, independently, of the structure -L⁴F³R^{C}, wherein L⁴ is C2-C6 alkylene, F³ is ester or amide functional group and R^{C} is selected from: undecan-5-yl, tridecan-6-yl, pentadecan-7-yl, heptadecan-8-yl, and nonadecan-9-yl. Examples of structures are: -CH₂CH₂C(=O)NHR^{C}, -CH₂CH₂CH₂C(=O)NHR^{C}, -CH₂CH₂CH₂CH₂C(=O)NHR^{C}, -CH₂CH₂CH₂CH₂CH₂C(=O)NHR^{C}, -CH₂CH₂CH₂CH₂CH₂CH₂C(=O)NHR^{C}, -CH₂CH₂NHC(=O)R^{C}, -CH₂CH₂CH₂NHC(=O)R^{C}, -CH₂CH₂CH₂CH₂NHC(=O)R^{C}, -CH₂CH₂CH₂CH₂CH₂NHC(=O)R^{C}, -CH₂CH₂CH₂CH₂CH₂CH₂NHC(=O)R^{C}, -CH₂CH₂OC(=O)R^{C}, -CH₂CH₂CH₂OC(=O)R^{C}, -CH₂CH₂CH₂CH₂OC(=O)R^{C}, -CH₂CH₂CH₂CH₂CH₂OC(=O)R^{C}, -CH₂CH₂CH₂CH₂CH₂CH₂OC(=O)R^{C}, -CH₂CH₂C(=O)OR^{C}, -CH₂CH₂CH₂C(=O)OR^{C}, -CH₂CH₂CH₂CH₂C(=O)OR^{C}, -CH₂CH₂CH₂CH₂CH₂C(=O)OR^{C}, -CH₂CH₂CH₂CH₂CH₂CH₂C(=O)OR^{C}, wherein: R^{C} is undecan-5-yl, tridecan-6-yl, pentadecan-7-yl, heptadecan-8-yl, or nonadecan-9-yl.

In an embodiment, -R¹ is -L³F²R^{B}, wherein L³ is C2-C6 alkylene, F² is ester or amide functional group and R^{B} is selected from: undecan-5-yl, tridecan-6-yl, pentadecan-7-yl, heptadecan-8-yl, and nonadecan-9-yl. Examples of structures are: - CH₂CH₂C(=O)NHR^{B}, -CH²CH²CH²C(=O)NHR^{B}, -CH²CH²CH²CH²C(=O)NHR^{B}, -CH₂CH₂CH₂CH₂CH₂C(=O)NHR^{B}, -CH₂CH₂CH₂CH₂CH₂CH₂C(=O)NHR^{B}, -CH₂CH₂NHC(=O)R^{B}, -CH₂CH₂CH₂NHC(=O)R^{B}, -CH₂CH₂CH₂CH₂NHC(=O)R^{B}, -CH₂CH₂CH₂CH₂CH₂NHC(=O)R^{B}, -CH₂CH₂CH₂CH₂CH₂CH₂NHC(=O)R^{B}, -CH₂CH₂OC(=O)R^{B}, -CH₂CH₂CH₂OC(=O)R^{B}, -CH₂CH₂CH₂CH₂OC(=O)R^{B}, -CH₂CH₂CH₂CH₂CH₂OC(=O)R^{B}, -CH₂CH₂CH₂CH₂CH₂CH₂OC(=O)R^{B} -CH₂CH₂C(=O)OR^{B}, -CH₂CH₂CH₂C(=O)OR^{B}, -CH₂CH₂CH₂CH₂C(=O)OR^{B}, -CH₂CH₂CH₂CH₂CH₂C(=O)OR^{B}, -CH₂CH₂CH₂CH₂CH₂CH₂C(=O)OR^{B}, wherein: R^{B} is undecan-5-yl, tridecan-6-yl, pentadecan-7-yl, heptadecan-8-yl, or nonadecan-9-yl.

In an embodiment, -R¹ is -L¹N[L²F¹R^{A}]₂, wherein L¹ is C2-C6 alkylene, L² is C2-C6 alkylene, F¹ is ester or amide functional group and R^{A} is selected from: undecan-5-yl, tridecan-6-yl, pentadecan-7-yl, heptadecan-8-yl, and nonadecan-9-yl. Examples of -L²F¹R^{A} structures are: -CH₂CH₂C(=O)NHR^{A,} -CH₂CH₂CH₂C(=O)NHR^{A}, -CH₂CH₂CH₂CH₂C(=O)NHR^{A}, -CH₂CH₂CH₂CH₂CH₂C(=O)NHR^{A}, -CH₂CH₂CH₂CH₂CH₂CH₂C(=O)NHR^{A}, -CH₂CH₂NHC(=O)R^{A}, -CH₂CH₂CH₂NHC(=O)R^{A}, -CH₂CH₂CH₂CH₂NHC(=O)R^{A}, -CH₂CH₂CH₂CH₂CH₂NHC(=O)R^{A}, -CH₂CH₂CH₂CH₂CH₂CH₂NHC(=O)R^{A}, -CH₂CH₂OC(=O)R^{A}, -CH₂CH₂CH₂OC(=O)R^{A}, -CH₂CH₂CH₂CH₂OC(=O)R^{A}, -CH₂CH₂CH₂CH₂CH₂OC(=O)R^{A}, -CH₂CH₂CH₂CH₂CH₂CH₂OC(=O)R^{A}, -CH₂CH₂C(=O)OR^{A}, -CH₂CH₂CH₂C(=O)OR^{A}, -CH₂CH₂CH₂CH₂C(=O)OR^{A}, -CH₂CH₂CH₂CH₂CH₂C(=O)OR^{A}, -CH₂CH₂CH₂CH₂CH₂CH₂C(=O)OR^{A}, wherein: R^{A} is undecan-5-yl, tridecan-6-yl, pentadecan-7-yl, heptadecan-8-yl, or nonadecan-9-yl. Further to the examples of -L²F¹R^{A} structures, L¹ is ethylene, propylene or butylene, in particular ethylene.

In an embodiment, L¹, L², L³ and L⁴ are ethylene. In an embodiment, L¹, L², L³ and L⁴ are butylene. In an embodiment, L¹, L², L³ and L⁴ are hexene. In an embodiment, L¹ is ethylene and L², L³ and L⁴ are butylene. In an embodiment, L¹ is ethylene and L², L³ and L⁴ are hexylene.

In an embodiment, L¹ is ethylene. In a further embodiment, L¹ is a ethylene and L² and L⁴ are butylene, pentylene or hexylene, in particular L² and L⁴ are butylene.

In an embodiment, m is 1, 2, 3, 4 or5, or, m is selected from 1, 3 and 5. In a further embodiment, m is 1, 2 or 3. In yet a further embodiment, m is 1.

In a further embodiment, the ionizable lipid-like structure or pharmaceutically acceptable salt, tautomer, or stereoisomer thereof is as presented in Table 1.

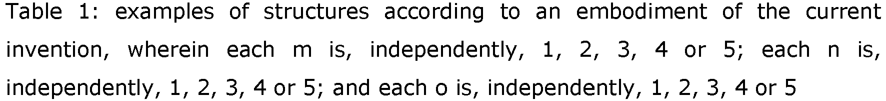

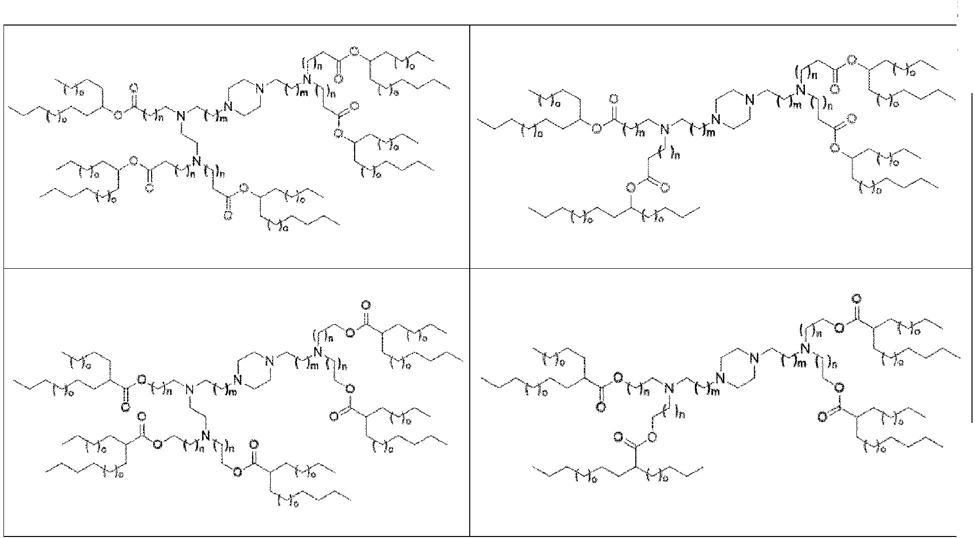

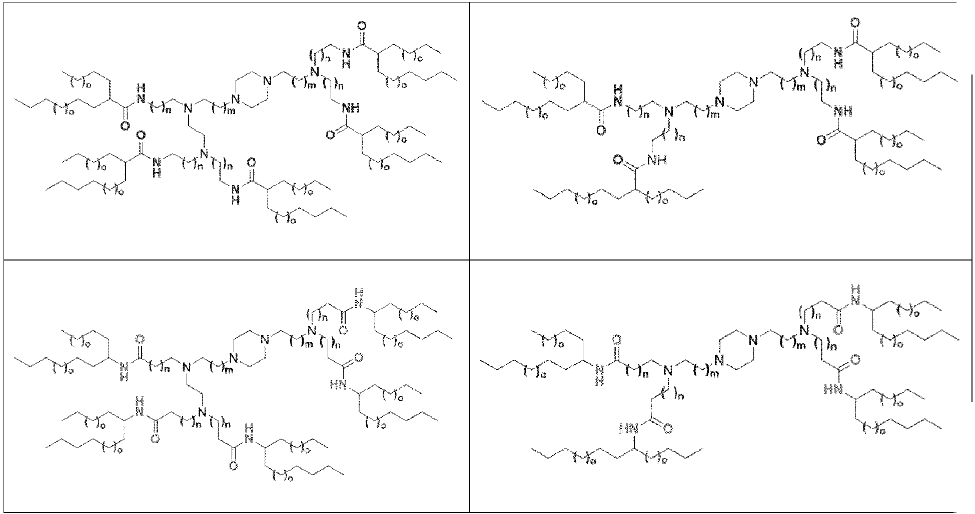

It will be understood that the current invention also extends to the compounds according to structures as disclosed in Table 1 and further including a pharmaceutically acceptable salt, tautomer, or stereoisomer thereof. For the scope of this embodiment, structures of final compounds provided in the schemes of section 1.1 of the examples are equally included. Some lipid like compounds and their corresponding LNPs may be protonated or deprotonated, as they are responsive to pH changes. At lower pH, nitrogen groups of these compounds can be protonated.

In an embodiment of the current invention, n in the structures shown in Table 1, is an integer from 1 to 5, such as 1, 3 or 5. In an embodiment, m in the structures shown in Table 1 is an integer from 1 to 5, such as 1, 3 or 5. In an embodiment, o in the structures shown in Table 1 is an integer between 1 and 5, such as 3, 4 or 5.

In an embodiment of the current invention, the ionizable lipid-like compound or pharmaceutically acceptable salt, tautomer, or stereoisomer thereof, is as presented in Table 2. Compounds described in the examples falling within the scope of compounds according to Formula I are equally considered representative embodiments.

**Table 2: examples of compounds according to an embodiment of the current invention**

| **Chemical structure** | **Chemical name** |
|---|---|
| 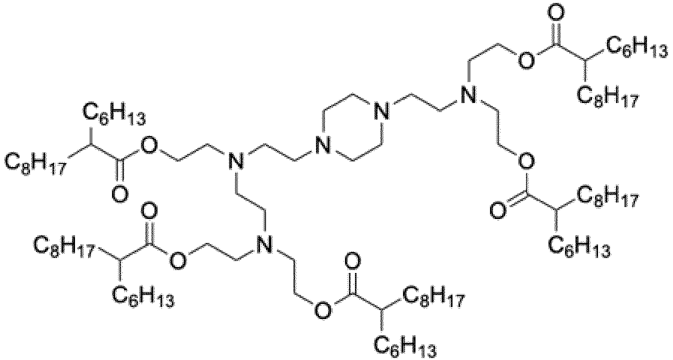 | ((2-(4-(2-((2-(bis(2-((2-hexyldecanoyl)oxy)ethyl)amino)ethyl)(2-((2-hexyldecanoyl)oxy)ethyl)amino)ethyl)piperazin -1-yl)ethyl)azanediyl)bis(ethane-2,1-diyl) bis(2-hexyldecanoate) |
| 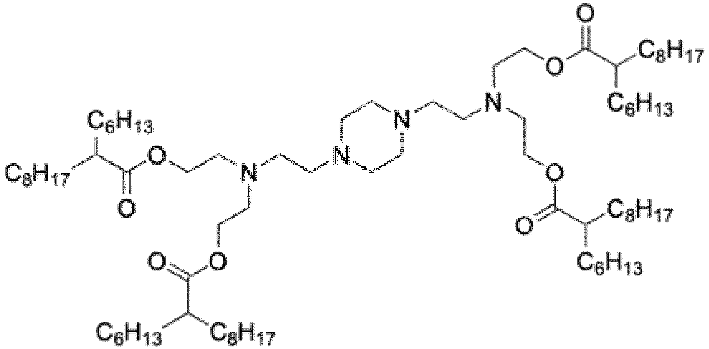 | ((piperazine-1,4-diylbis(ethane-2,1-diyl))bis(azanetriyl))tetrakis(ethane-2,1-diyl) tetrakis(2-hexyldecanoate) |
| 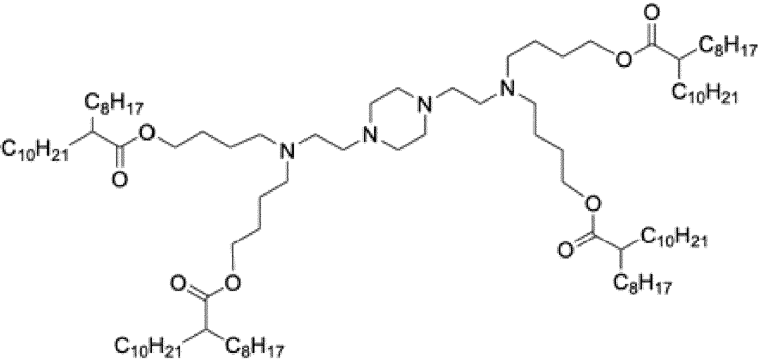 | ((piperazine-1,4-diylbis(ethane-2,1-diyl))bis(azanetriyl))tetrakis(butane-4,1-diyl) tetrakis(2-octyldodecanoate) |
| 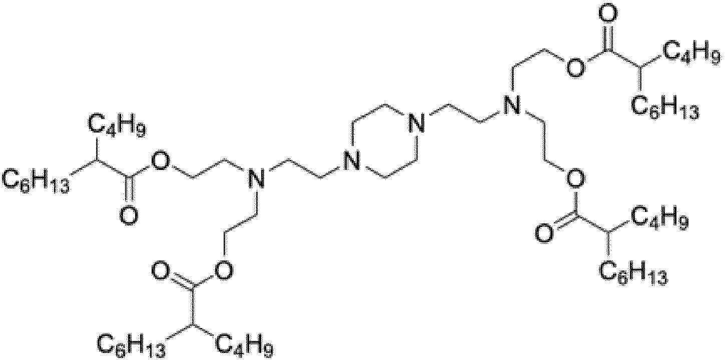 | ((piperazine-1,4-diylbis(ethane-2,1-diyl))bis(azanetriyl))tetrakis(ethane-2,1-diyl) tetrakis(2-butyloctanoate) |
| 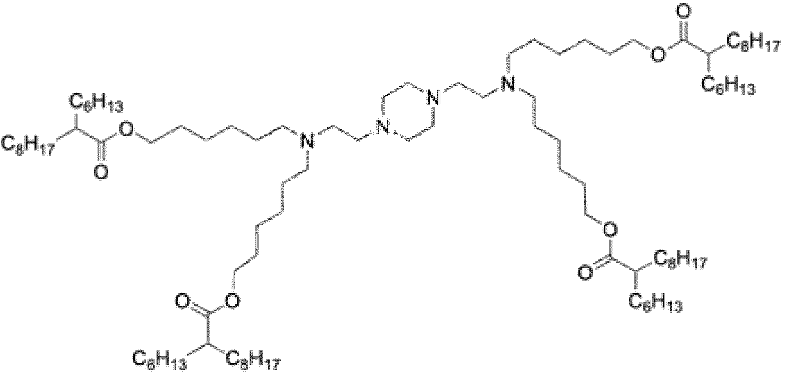 | ((piperazine-1,4-diylbis(ethane-2,1-diyl))bis(azanetriyl))tetrakis(hexane-6,1-diyl) tetrakis(2-hexyldecanoate) |
| 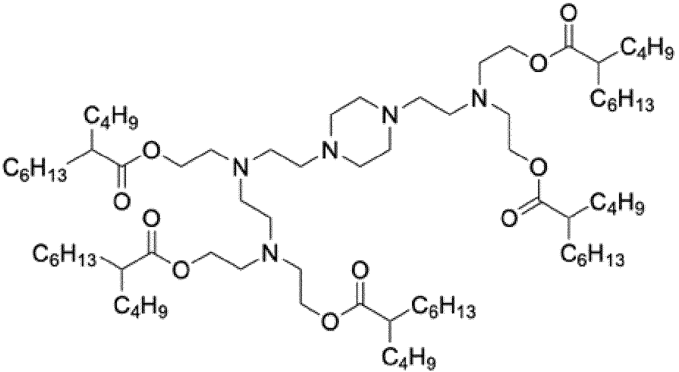 | ((2-(4-(2-((2-(bis(2-((2-butyloctanoyl)oxy)ethyl)amino)ethyl)(2-((2-butyloctanoyl)oxy)ethyl)amino)ethyl)piperazin-1-yl)ethyl)azanediyl)bis(ethane-2,1-diyl) bis(2-butyloctanoate) |
| 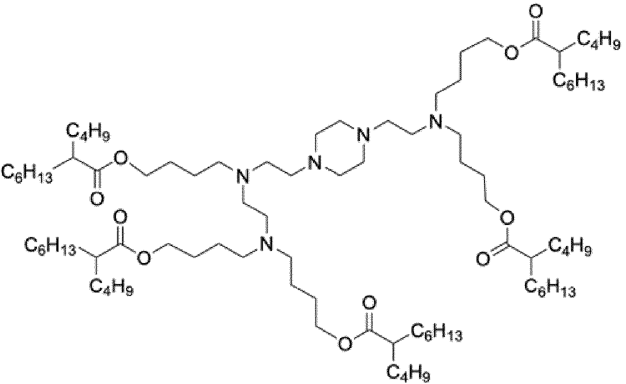 | ((2-(4-(2-((2-(bis(4-((2-butyloctanoyl)oxy)butyl)amino)ethyl)(4-((2-butyloctanoyl)oxy)butyl)amino)ethyl)piperazin-1-yl)ethyl)azanediyl)bis(butane-4,1-diyl) bis(2-butyloctanoate) |
| 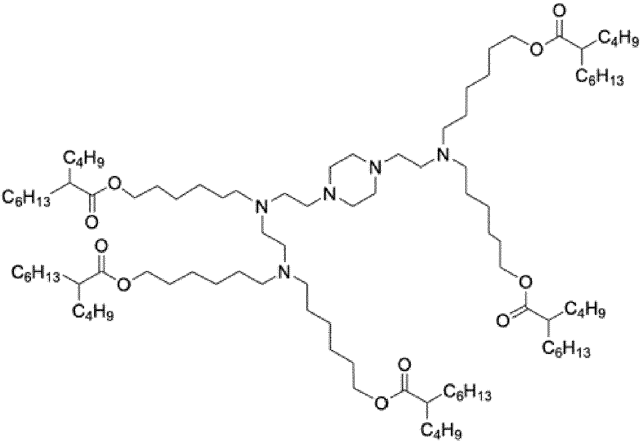 | ((2-(4-(2-((2-(bis(6-((2-butyloctanoyl)oxy)hexyl)amino)ethyl)(6-((2-butyloctanoyl)oxy)hexyl)amino)ethyl)piperazin -1-yl)ethyl)azanediyl)bis(hexane-6,1-diyl) bis(2-butyloctanoate) |
| 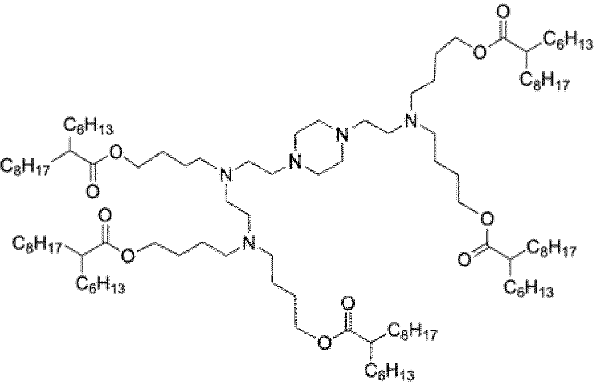 | ((2-(4-(2-((2-(bis(4-((2-hexyldecanoyl)oxy)butyl)amino)ethyl)(4-((2-hexyldecanoyl)oxy)butyl)amino)ethyl)piperazi n-1-yl)ethyl)azanediyl)bis(butane-4,1-diyl) bis(2-hexyldecanoate) |
| 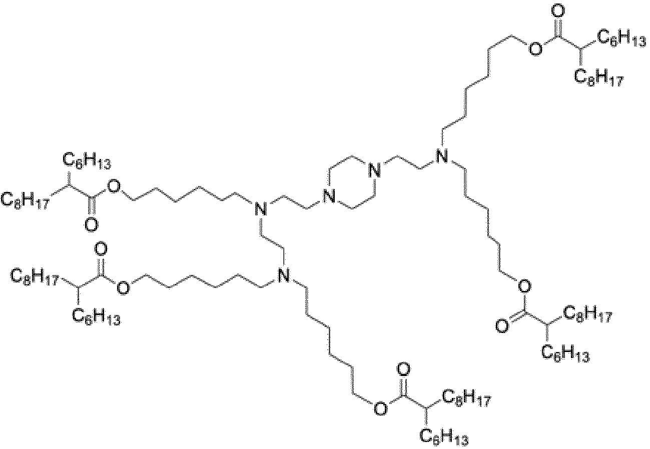 | ((2-(4-(2-((2-(bis(6-((2^{_} hexyldecanoyl)oxy)hexyl)amino)ethyl)(6-((2-hexyldecanoyl)oxy)hexyl)amino)ethyl)piperazi n-1-yl)ethyl)azanediyl)bis(hexane-6,1-diyl) bis(2-hexyldecanoate) |
| 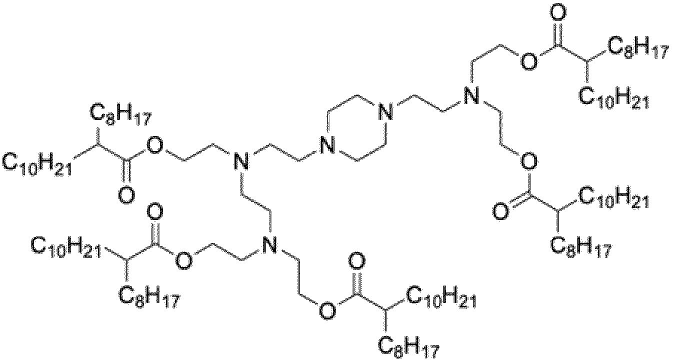 | ((2-(4-(2-((2-(bis(2-((2-octyldodecanoyl)oxy)ethyl)amino)ethyl)(2-((2-octyldodecanoyl)oxy)ethyl)amino)ethyl)piperaz in-1-yl)ethyl)azanediyl)bis(ethane-2,1-diyl) bis(2-octyldodecanoate) |
| 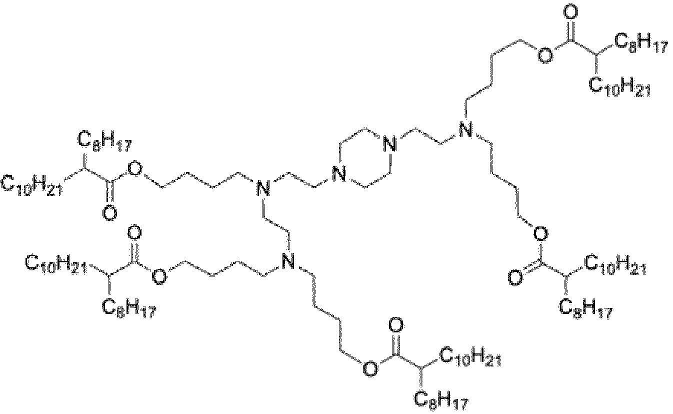 | ((2-(4-(2-((2-(bis(4-((2-octyldodecanoyl)oxy)butyl)amino)ethyl)(4-((2-octyldodecanoyl)oxy)butyl)amino)ethyl)pipera zin-1-yl)ethyl)azanediyl)bis(butane-4,1-diyl) bis(2-octyldodecanoate) |
| 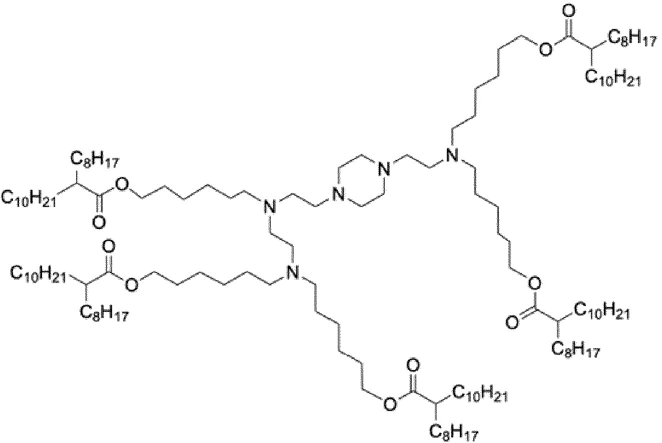 | ((2-(4-(2-((2-(bis(6-((2-octyldodecanoyl)oxy)hexyl)amino)ethyl)(6-((2-octyldodecanoyl)oxy)hexyl)amino)ethyl)pipera zin-1-yl)ethyl)azanediyl)bis(hexane-6,1-diyl) bis(2-octyldodecanoate) |
| 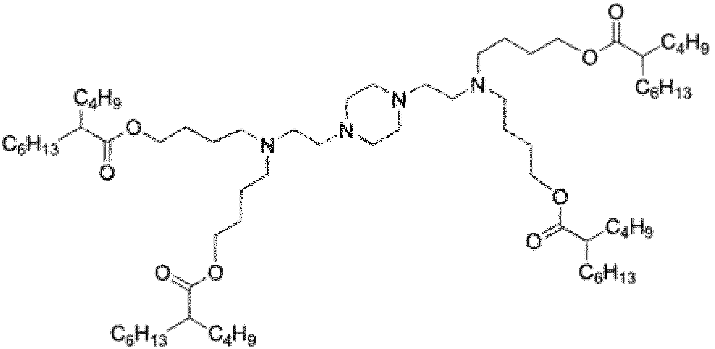 | ((piperazine-1,4-diylbis(ethane-2,1-diyl))bis(azanetriyl))tetrakis(butane-4,1-diyl) tetrakis(2-butyloctanoate) |
| 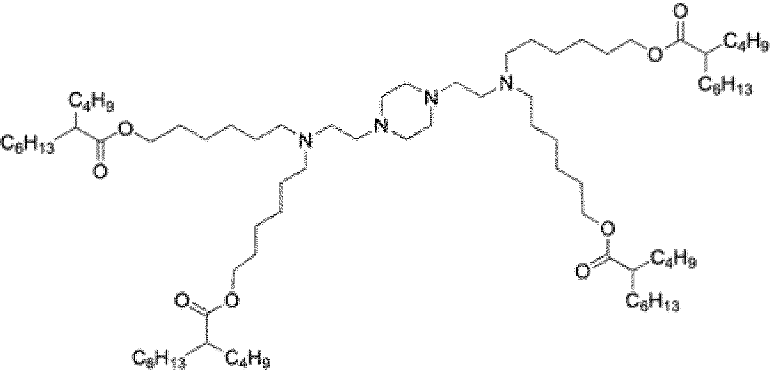 | ((piperazine-1,4-diylbis(ethane-2,1-diyl))bis(azanetriyl))tetrakis(hexane-6,1-diyl) tetrakis(2-butyloctanoate) |
| 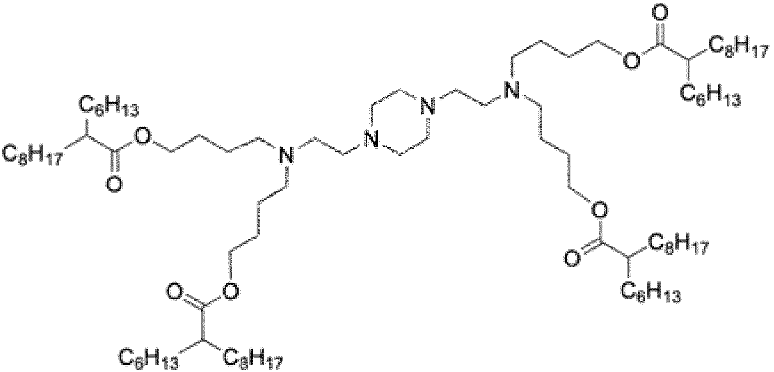 | ((piperazine-1,4-diylbis(ethane-2,1-diyl))bis(azanetriyl))tetrakis(butane-4,1-diyl) tetrakis(2-hexyldecanoate) |
| 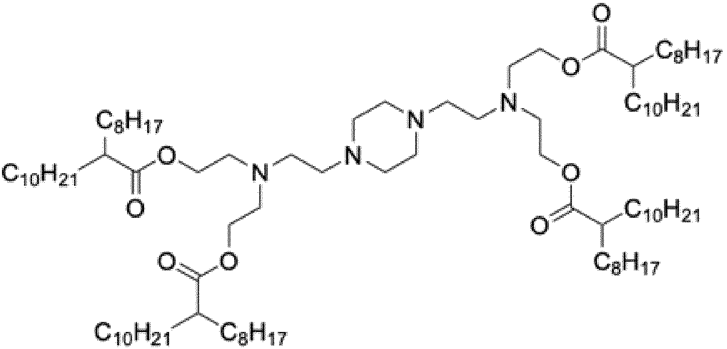 | ((piperazine-1,4-diylbis(ethane-2,1-diyl))bis(azanetriyl))tetrakis(ethane-2,1-diyl) tetrakis(2-octyldodecanoate) |
| 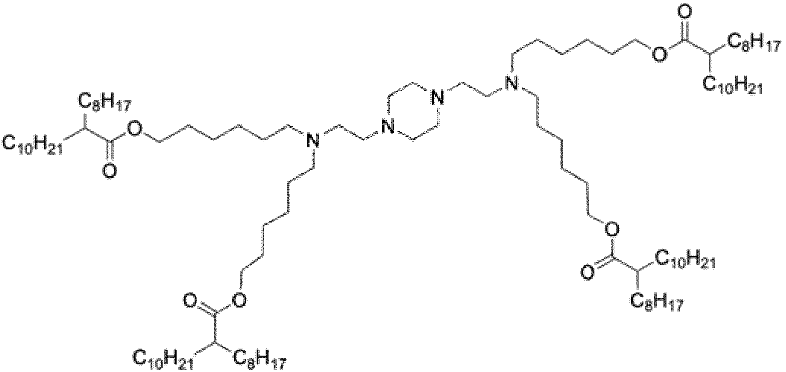 | ((piperazine-1,4-diylbis(ethane-2,1-diyl))bis(azanetriyl))tetrakis(hexane-6,1-diyl) tetrakis(2-octyldodecanoate) |
| 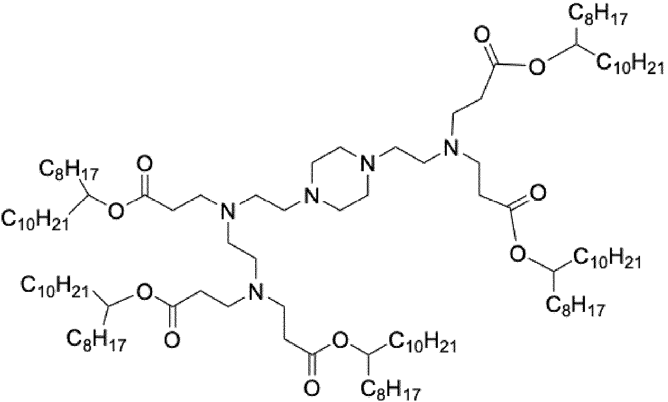 | di(nonadecan-9-yl) 3,3'-((2-(4-(2-((2-(bis(3-(nonadecan-9-yloxy)-3-oxopropyl)amino)ethyl)(3-(nonadecan-9-yloxy)-3-oxopropyl)amino)ethyl)piperazin-1-yl)ethyl)azanediyl)dipropionate |
| 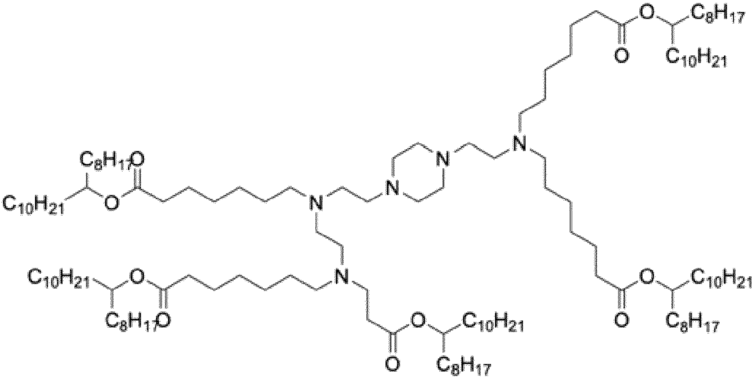 | di(nonadecan-9-yl) 7,7'-((2-(4-(2-((2-((3-(nonadecan-9-yloxy)-3-oxopropyl)(7-(nonadecan-9-yloxy)-7-oxoheptyl)amino)ethyl)(7-(nonadecan-9-yloxy)-7-oxoheptyl)amino)ethyl)piperazin-1-yl)ethyl)azanediyl)diheptanoate |

In an embodiment, for compounds according to formula I wherein R¹ is -L³F²R^{B} and R², R³ and R⁴ are each -L⁴F³R^{C}, and each L³ and L⁴ are ethylene (C2 alkylene), each each R^{A}, R^{B} and R^{C} are, independently, branched C15-C30 alkyl. Alternatively, for compounds according to formula I wherein R¹ is -L³F²R^{B} and R², R³ and R⁴ are each -L⁴F³R^{C}, and each L³ and L⁴ are ethylene (C2 alkylene), -F²R^{B} is selected from -O-C(=O)-R^{B}, -C(=O)-N-R^{B} and -NH-C(=O)-R^{B}, and, -F³R^{C} is selected from -O-C(=O)-R^{C}, -C(=O)-NH-R^{C} and -N-C(=O)-R^{C}. In a further embodiment, -F²R^{B} is -O-C(=O)-R^{B} and each -F³R^{C} is -O-C(=O)-R^{C}.

In an embodiment, for compounds according to formula I wherein R¹ is -L¹N[L²F¹R^{A}]₂ and R², R³ and R⁴ are each -L⁴F³R^{C}, each R^{A}, R^{B} and R^{C} are, independently, branched C13, C14, C15, C16, C17, C18, C19, C20, C21 or C22 alkyl.

In an embodiment, for compounds according to formula I wherein each of L¹, L², L³ and/or L⁴ are C2 alkyl, each R^{A}, R^{B} and R^{C} are, independently, branched C15, C16, C17, C18, C19, C20, C21 or C22 alkyl.

In an embodiment, R¹ is -L³F²R^{B}

In an embodiment, compounds according to Formula I are further defined for L¹ being ethylene, L², L³ and L⁴ being butylene or hexylene, and, R^{A}, R^{B} and R^{C} being C11-C15 branched alkyl. In a further embodiment, compounds according to Formula I are further defined for L¹ being ethylene, L², L³ and L⁴ being butylene, and, R^{A}, R^{B} and R^{C} being undecan-5-yl, tridecan-6-yl, or pentadecan-7-yl. In a further embodiment, F¹, F² and F³ have structure -O-C(=O)-R.

A specific embodiment relates to compounds of Formula I wherein m=1, L¹ is ethylene, L², L³ and L⁴ are butylene or hexylene and R^{A}, R^{B} and R^{C} are undecan-5-yl, tridecan-6-yl, or pentadecan-7-yl.

In a second aspect, the invention relates to (a) lipid nanoparticle(s) (LNP(s)) comprising at least one of the compounds according to formula I and as defined in embodiments herein for the formulation of oligonucleotides, in particular such LNPs for use in a method of treatment.

Accordingly, the lipid nanoparticles (LNP) comprise one or more oligonucleotides. In an embodiment, said oligonucleotide is RNA or DNA, preferably an oligonucleotide such as self- amplifying RNA (saRNA) having a length of at least 1000 bp, more preferably 2000 bp, 3000 bp, 4000 bp, 5000 bp or more. In an embodiment, one or more saRNA molecules or one or more other oligonucleotides have a minimal length of at least 1000 bp, more preferably 2000 bp, 3000 bp, 4000 bp, 5000 bp or more. The length of an oligonucleotide may be expressed in a number of base pairs (bp) or a number of nucleotides (nt). In the context of the current invention, the terms "base pairs" and "nucleotides" when being used in the context of the length of an oligonucleotide (such as DNA or RNA) are used interchangeably, wherein one base pair (bp) is considered the equivalent of one nucleotide (nt).

In an embodiment, said oligonucleotide is an RNA molecule such as mRNA. In a particular embodiment, said RNA has a minimal length of at least 1000 bp, more preferably 2000 bp, 3000 bp, 4000 bp, 5000 bp or more. In a particularly preferred embodiment, said RNA is a self-amplifying RNA (saRNA). It was found that the lipid nanoparticle provided herein are particularly suited to be used in combination with RNA of a specific size, i.e. long length RNA, preferably self-amplifying RNA (saRNA). Since saRNA is typically larger than conventional mRNA, lipid nanoparticles described in the state of the art often result in poor encapsulation, or sub-optimal *in vivo* delivery and thus expression of the gene of interest encoded by the RNA. As illustrated by the examples, it is now found that the compounds according to formula I allow for efficient encapsulation of large RNA molecules (such as saRNA constructs) whilst the resulting lipid nanoparticle allows for good functionality of the RNA (intracellular delivery and expression). In an embodiment, the lipid nanoparticle composition comprises RNA oligonucleotides, such as mRNA, in particular saRNA, having a length equivalent to 5000 bp or more. The size of the (sa)RNA can be between 500 and 50 000 bp, preferably between 1000 and 40 000 bp, more preferably between 5000 and 30 000nt, or between 8000 and 16 000 bp. More preferably the size of said RNA, such as saRNA, can be between 5000 and 20000 bp, preferably between 6000 and 19000 bp, preferably between 7000 and 18000 bp, preferably between 8000 and 17000 bp, more preferably between 8000 and 16000 bp. According to a particular embodiment, the RNA encapsulated in the LNP is at least 8000, at least 9000 or at least 10000 nt. Further to said embodiment, the RNA does not exceed 20000, 18000 or 16000 nt.

The self-replicative nature of the mRNA constructs in saRNA is based on the genomic RNA of RNA viruses, but lack the genes encoding one or more structural proteins. The self-replicating RNA molecules are capable of being translated to produce non-structural proteins of the RNA virus and heterologous proteins encoded by the self-replicating RNA.

Self-replicating RNA molecules are designed so that the self- replicating RNA molecule cannot induce production of infectious viral particles. One suitable system for achieving self-replication is to use an alphavirus-based RNA replicon. These +-stranded replicons are translated after delivery to a cell to give of a replicase (or replicase-transcriptase). The replicase is translated as a polyprotein which auto-cleaves to provide a replication complex which creates genomic --strand copies of the +-strand delivered RNA. These --strand transcripts can themselves be transcribed to give further copies of the +-stranded parent RNA and also to give a subgenomic transcript which encodes the desired gene product. Translation of the subgenomic transcript thus leads to in situ expression of the desired gene product by the cell. Suitable alphavirus replicons can use a replicase from a Sindbis virus, a Semliki Forest Virus, an eastern equine encephalitis virus, a Venezuelan Equine Encephalitis Virus, etc.

A preferred self-replicating RNA molecule encodes (i) a RNA-dependent RNA polymerase which can transcribe RNA from the self-replicating RNA molecule and (ii) protein/peptide as described herein. The polymerase can be an alphavirus replicase e.g., comprising alphavirus protein nsP4.

Whereas natural alphavirus genomes encode structural virion proteins in addition to the non-structural replicase polyprotein, it is preferred that an alphavirus based self-replicating RNA molecule of the invention does not encode alphavirus structural proteins. Thus, the self-replicating RNA can lead to the production of genomic RNA copies of itself in a cell, but not to the production of RNA-containing alphavirus virions. The inability to produce these virions means that, unlike a wild-type alphavirus, the self-replicating RNA molecule cannot perpetuate itself in infectious form. The alphavirus structural proteins which are necessary for perpetuation in wild-type viruses are absent from self-replicating RNAs of the invention and their place is taken by gene(s) encoding the desired gene product, such that the subgenomic transcript encodes the desired gene product rather than the structural alphavirus virion proteins. Therefore, in a particular embodiment, the self-replicating RNA molecule of the invention comprises a sequence encoding nonstructural alphavirus proteins and a sequence encoding a protein/peptide of interest (e.g. antigen for a vaccine). More in particular, the self-replicating RNA molecule of the invention comprises a sequence encoding the four nonstructural alphavirus proteins and a sequence encoding a protein/peptide of interest (e.g. antigen for a vaccine). Preferably, the self-replicating RNA molecule is derived from an alphavirus which has been engineered to lack the ability to produce at least one structural alphaviral protein. More preferably, the self-replicating RNA molecule is derived from an alphavirus which has been engineered to lack the ability to produce at least two, more preferably all, structural alphaviral proteins. In a particular embodiment, the self-replicating RNA molecule of the invention comprises, in 5' to 3' order (i) a 5' sequence required for nonstructural protein-mediated amplification, (ii) a nucleotide sequence encoding alphavirus, particularly Venezuelan equine encephalitis virus, nonstructural proteins nsP1, nsP2, nsP3, and nsP4, (iii) a promotor which is operably linked to a heterologous nucleic acid sequence encoding a protein/peptide of interest (e.g. antigen for a vaccine), wherein the heterologous nucleic acid sequence replaces one or all of the alphavirus structural protein genes, (iv) a 3' sequence required for nonstructural protein-mediated amplification, and (v) a polyadenylate tract.

Recently, saRNA (RNA replicon) vaccination is recognized as innovative nanotechnology-based vaccination strategy. As mentioned previously, unlike viral replicon particles (i.e. RNA encapsulated in viral capsid proteins), sa-mRNA can be produced by in vitro transcription only. Thus, the whole manufacturing process is entirely cell-free, resulting in a therapeutic whose composition is precisely defined. sa-mRNA vaccines have several attractive features, such as extending the duration (approximately 2 months) and magnitude of expression compared to their non-replicating counterparts. In addition, the intracellular replication of sa-mRNA is transient, and the double-stranded RNA (dsRNA) may induce interferon-mediated host-defense mechanisms by triggering pattern recognition receptors. This results in strong antigen-specific immune responses against the inserted target molecules. Thus, sa-mRNA vector systems are ideally suited for vaccine development because they provide high transient transgene expression and inherent adjuvant effects.

Alternatively, saRNA constructs are considered for use of the LNPs described herein which are useful in the delivery of therapeutic proteins or peptides, e.g. in protein replacement therapy, in particular because of the typically longer expression profile of saRNA compared to conventional mRNA when delivered to the cell. The repetitive nature of protein replacement therapy calls for LNPs with efficient encapsulation, good cellular delivery and high systemic tolerability (e.g. including low toxicity).

Compounds and LNPs disclosed herein show favorable characteristics to address that need.

In some embodiments, the self-amplifying RNA molecules are based on the genomic RNA of RNA viruses but lack the genes encoding one or more structural proteins. The self-amplifying RNA molecules are capable of being translated to produce non-structural proteins of the RNA virus and heterologous proteins encoded by the self-amplifying RNA.

In some embodiments, the self-amplifying RNA molecules can be designed so that the self-amplifying RNA molecule cannot induce production of infectious viral particles. This can be achieved, for example, by omitting one or more viral genes encoding structural proteins that are necessary to produce viral particles in the self-amplifying RNA. For example, when the self-amplifying RNA molecule is based on an alpha virus, such as Sindbis virus (SIN), Semliki Forest virus and Venezuelan equine encephalitis virus (VEEV), one or more genes encoding viral structural proteins, such as capsid and/or envelope glycoproteins, can be omitted. If desired, self-amplifying RNA molecules of the invention can be designed to induce production of infectious viral particles that are attenuated or virulent, or to produce viral particles that are capable of a single round of subsequent infection.

In some embodiments, the self-amplifying RNA molecules described herein, may be engineered to express multiple nucleotide sequences, from two or more open reading frames, thereby allowing co-expression of proteins, such as a two or more antigens together with cytokines or other immunomodulators, which can enhance the generation of an immune response. Such a self- replicating RNA molecule might be particularly useful, for example, in the production of various gene products (e.g., proteins) at the same time, for example, as a bivalent or multivalent vaccine, or in gene therapy applications.

In an embodiment, the molar ratio between a lipid in a lipid nanoparticle and an oligonucleotide is between 1:1 and 100:1. In some embodiments, the ratio of lipid to mRNA in liposomes may be from about 5: 1 to about 80: 1, from about 10: 1 to about 75: 1, from about 15: 1 to about 60: 1, from about 15: 1 to about 50: 1 and/or at least 40: 1. These ratios ensure optimal RNA adsorption to the lipid nanoparticle.

In some embodiments, the lipid nanoparticles have a mean diameter (also referred to as average particle size) of 30 nm to 250 nm, 40 nm to 250 nm, 50 nm to 250 nm, from 50 to 150 nm, from 50 to 130 nm, 60 nm to 230 nm, 70 nm to 210 nm, 70 nm to 200 nm, from 80 nm to 200 nm, from 90 nm to 200 nm, from 70 to 190 nm, from 80 nm to 190 nm, from 70 nm to 180 nm, from 70 nm to 150 nm, from 70 nm to 130 nm, from 70 nm to 110 nm, from 80 to 150 nm, from 80 to 130 nm, from 80 to 120 nm, from 90 nm to 110 nm, or, about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm, 160 nm, 170 nm, 180 nm, 190 nm, 200 nm, 210 nm, 220 nm, 230 nm, 240 nm, 250 nm and are substantially non- toxic.

In certain embodiments, the diameter of the particles ranges from 1 nm to 1,000 nm. In certain embodiments, the diameter of the particles ranges from 200 nm to 300 nm. In certain embodiments, the diameter of the particles ranges from 300 nm to 400 nm. In certain embodiments, the diameter of the particles ranges from 400 nm to 500 nm. In certain embodiments, the diameter of the particles ranges from 600 nm to 700 nm. In certain embodiments, the diameter of the particles ranges from 700 nm to 800 nm. In certain embodiments, the diameter of the particles ranges from 800 nm to 900 nm. In certain embodiments, the diameter of the particles ranges from 100 nm to 1,000 nm. In certain embodiments, the diameter of the particles ranges from 20 nm to 2,000 nm.

In an embodiment, the N/P ratio between the ionizable cationic lipids in said lipid nanoparticle according to the current invention and the oligonucleotide (such as saRNA) is between 5:1 and 50:1. In some embodiments, the N/P ratio in LNPs may be from about 5: 1 to about 45: 1, from about 10: 1 to about 45: 1, from about 15: 1 to about 40: 1, from about 20: 1 to about 40: 1, from about 30:1 to about 50:1. These ratios ensure optimal RNA adsorption to the lipid nanoparticle, in particular when the RNA is of certain length, such as having more than 5000 nt, more than 6000, more than 7000 nt, more than 8000 nt or more than 9000 nt.

In an embodiment, the lipid nanoparticle composition according to the current invention has a Zeta potential between range of -30 mV and +30mV. The Zeta potential is an indicator of the charge at the surface of the particle, with a negative zeta potential indicating that the surface of the particle is mostly covered by RNA, while a positive zeta potential indicates that the complexing lipids have not been saturated by RNA adsorption. The Zeta potential is higher for compositions described in the state of the art, which indicates less binding of oligonucleotides on the surface of the lipid nanoparticle. There is no significant difference in particle size between the current invention and the state of the art.

Notwithstanding the fact that the LNPs of the current invention are particularly useful to be used in the context of saRNA (or other large RNA molecules), the latter can also be used for complexing other oligonucleotides such as DNA or RNA. In some embodiments, the LNP encapsulates long-chain RNA, coding RNA, non-coding RNA, long non-coding RNA, single stranded RNA (ssRNA), double stranded RNA (dsRNA), linear RNA (linRNA), circular RNA (circRNA), messenger RNA (mRNA), Trans amplifying mRNA, RNA oligonucleotides, antisense oligonucleotides, small interfering RNA (siRNA), small hairpin RNA (shRNA), antisense RNA (asRNA), CRISPR/Cas9 guide RNAs (gRNA), riboswitches, im-munostimulating RNA (isRNA), ribozymes, aptamers, ribosomal RNA (rRNA), transfer RNA (tRNA), viral RNA (vRNA), retroviral RNA or replicon RNA, small nuclear RNA (snRNA), small nucleolar RNA (snoRNA), microRNA (miRNA), and a Piwi-interacting RNA (piRNA).

In some embodiments, the LNP encapsulates modified RNA molecules. In some embodiments, the modification of RNA molecule comprises chemical modifications comprising backbone modifications as well as sugar modifications or base modifications. In this context, a modified RNA molecule as defined herein comprises nucleotide analogues/modifications, e.g. backbone modifications, sugar modifications or base modifications. A backbone modification in connection with the present disclosure is a modification, in which phosphates of the backbone of the nucleotides contained in an RNA molecule are chemically modified. A sugar modification in connection with the present disclosure is a chemical modification of the sugar of the nucleotides of the RNA molecule. Furthermore, a base modification in connection with the present disclosure is a chemical modification of the base moiety of the nucleotides of the RNA molecule. In this context, nucleotide analogues or modifications are selected from nucleotide analogues, which are applicable for transcription and/or translation. In further embodiments, the modified RNA comprises nucleoside modifications selected from 6-aza-cytidine, 2-thio-cytidine, α-thio-cytidine, pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-chloro-purine, N6-methyl-2-amino-purine, pseudo-iso-cytidine, 6-chloro-purine, N6-methyl-adenosine, α-thio-adenosine, 8-azido-adenosine, 7-deaza-adenosine.

A lipid-like compound according to Formula (I) or pharmaceutically acceptable salt is by preference present in the LNP formulation in a concentration of about 5-60 mol%, preferably 12.5-60 mol% and more preferably 20-45 mol%.

LNPs are commonly formulated with two or more excipients: (i) a sterol, which enhances the stability of the LNP bilayer and promotes membrane fusion; (ii) optionally a phospholipid, which fortifies the LNP bilayer structure and also aids in endosomal escape; and (iii) a lipid-polyethylene glycol (PEG) conjugate, which inserts into the LNP bilayer and provides a PEG coating that reduces LNP aggregation, reduces nonspecific binding of proteins due to sterically hindrance, and reduces nonspecific endocytosis by immune cells. In a further embodiment, a LNP may further comprise one of more buffering agents.

In an embodiment, besides the lipid-like structures of Formula (I), an LNP according to the current invention further comprises:
- at least a PEG or a PEG conjugate,
- at least a sterol, and
- optionally at least a phospholipid and/or at least a second ionizable lipid.

In an embodiment, the second ionizable lipid is a compound of Formula (I). In an embodiment, the second ionizable lipid is not a compound of Formula (I).

In an embodiment the PEG or PEG conjugate is present in the LNP formulation according to the current invention in a concentration of 0.2-10 mol%, preferably 0.5-5 mol%. The PEG compound is preferably selected from PEG-ceramide, PEG-DMG, PEG-PE, poloxamer, and DSPE carboxy PEG. For instance, in certain embodiments, the PEG compound is C14 PEG2000 DMG, C15 PEG2000 DMG, C16 PEG2000 DMG, C18 PEG2000 DMG, C14 PEG 2000 ceramide, C15 PEG2000 ceramide, C16 PEG2000 ceramide, C18 PEG2000 ceramide, C14 PEG2000 PE, C15 PEG2000 PE, C16 PEG2000 PE, C18 PEG2000 PE, C14 PEG350 PE, C14 PEG5000 PE, poloxamer F-127, poloxamer F-68, poloxamer L-64, or DSPE carboxy PEG. In a particularly preferred embodiment, said PEG conjugate is DMG-PEG.

In an embodiment the sterol compound is present in the LNP formulation according to the current invention in a concentration of 30-60 mol%, preferably 30-50 mol%, more preferably 40-50 mol%. Said sterol is preferably selected from the group of ergosterol, campesterol, oxysterol, antrosterol, desmosterol, nicasterol, sitosterol, stigmasterol and cholesterol or a derivative thereof, such as 3β[N-(N',N'-dimethylaminoethane)-carbamoyl]cholesterol (DC-cholesterol). In a preferred embodiment, said sterol is cholesterol.

In an embodiment, the LNP formulation according to the current invention comprises at least a phospholipid. Possible non- limiting examples of phospholipids are distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), pal mitoyloleoyl-phosphatidylethanolamine (POPE), dioleoylphosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), DLPE (1,2-dilauroyl-sn-glycero-3-phosphoethanolamine), DPPS (1,2-dipalmitoyl-sn-glycero-3-phospho-L-serine), 16-O-monomethyl PE, 16-O-dimethyl PE, 18-1-trans PE, 1-stearoyl-2-oleoyl-phosphatidyethanolamine (SOPE). The phospholipid fortifies the LNP bilayer structure and also aids in endosomal escape. In an embodiment, the phospholipid in the nanoparticle composition is DOPE. In an embodiment, the LNP comprises a phospholipid, wherein said phospholipid is present in a concentration of 0.2-45 mol%, preferably 0.5-35 mol% in said LNP, said phospholipid is preferably DOPE.

In an embodiment, the LNP formulation according to the current invention comprises at least one second ionizable lipid other than a lipid-like structure according to Formula (I). Possible non- limiting examples for the second ionizable lipid are N,N-dioleyl-N,N- dimethylammonium chloride (DODAC); N-(2,3-dioleyloxy)propyl)-N,N,N- trimethylammonium chloride (DOTMA); N,N-distearyl-N,N-dimethylammonium bromide (DDAB); N-(2,3dioleoyloxy)propyl)-N,N,N-trimethylammonium chloride (DOTAP); N-(1-(2,3- dioleoyloxy)propyl)N-2-(sperminecarboxamido)ethyl)-N,N-dimethylammonium trifluoracetate (DOSPA), dioctadecylamidoglycyl carboxyspermine (DOGS), 1,2-dioleoyl- 3-dimethylammonium propane (DODAP), 3-(N,N-dioleylamino)-1,2-propanediol (DOAP), N,N-dimethyl-2,3-dioleoyloxy)propylamine (DODMA), and N-(1,2-dimyristyloxyprop-3-yl)-N,N-dimethyl-N-hydroxyethyl ammonium bromide (DMRIE), 1,2-dilinoleyloxy-N,N-dimethylaminopropane (DLinDMA), 1,2-dilinoleyoxy-3-(dimethylamino)acetoxypropane (DLin-DAC), 1,2-dilinoleyoxy-3morpholinopropane (DLin-MA), 1,2-dilinoleoyl-3- dimethylaminopropane (DLinDAP), 1,2-dilinoleylthio-3-dimethylaminopropane (DLin-S- DMA), 1-linoleoyl-2-linoleyloxy-3dimethylaminopropane (DLin-2-DMAP), 1,2- dilinoleyloxy-3-trimethylaminopropane chloride salt (DLin-TMA.CI), 1,2-dilinoleoyl-3-trimethylaminopropane chloride salt (DLin-TAP.CI), 1,2-dilinoleyloxy-3-(N-methylpiperazino)propane (DLin-MPZ), 1,2-dilinoleyloxo-3-(2-N,N-dimethylamino)ethoxypropane (DLin-EG-DMA), 2,2-dilinoleyl-4-dimethylaminomethyl-[1,3]-dioxolane (DLin-KC2-DMA), dilinoleyl-methyl-4-dimethylaminebutyrate (DLin-MC3-DMA), di((Z)-non-2-en-1-yl) 9-((4-(dimethylamino)butanoyl)oxy)heptadecanedioate (L319), 1,1'-((2-(4-(2-((2-(bis (2-hydroxydodecyl)amino)ethyl) (2-hydroxydodecyl)amino)ethyl) piperazin-1-yl)ethyl)azanediyl) bis(dodecan-2-ol) (C12-200), 3,6-bis({4-[bis(2-hydroxydodecyl)amino]butyl})piperazine-2,5-dione (cKK-E12). Said second ionizable lipid is present in the LNP formulation according to the current invention in a concentration of 0.5-40 mol%, preferably 0.5-30 mol%. In a more preferred embodiment, the second ionizable lipid is C12-200 or DLin-KC2-DMA or a pharmaceutically acceptable salt, tautomer, or stereoisomer thereof.

In an embodiment, the lipid nanoparticle comprises at least one second ionizable lipid, wherein the overall concentration of said ionizable lipid-like structure according to Formula (I) or pharmaceutically acceptable salt, tautomer, or stereoisomer thereof and said second ionizable lipid is present in said LNP in a concentration of 12.5-60 mol%, preferably 25-50 mol%, and more preferably 30-40 mol%.

In an embodiment, the LNP formulation according to the current invention comprises a number of commercial preparations of lipids. These include, for example, LIPOFECTIN^{®} (commercially available cationic liposomes comprising DOTMA and 1,2- dioleoyl-sn-3phosphoethanolamine (DOPE), from GIBCO/BRL, Grand Island, N.Y.); LIPOFECTAMINE^{®} (commercially available cationic liposomes comprising N-(1- (2,3dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.).

In an embodiment, the LNP formulation according to the current invention comprises at least two lipids or lipid-like compounds according to Formula (I) or selected from Table 1. It was found that the presence of at least two ionizable lipids or lipid like compounds positively influences the encapsulation of the oligonucleotides such as the saRNA, as well as the *in vivo* delivery of the LNPs.

In an embodiment, the lipid nanoparticle comprises at least one ionizable lipid-like structure according to Formula (I) or pharmaceutically acceptable salt, tautomer, or stereoisomer thereof in a concentration of 12.5-60 mol%; DOPE in a concentration of 0.5-35 mol%; cholesterol in a concentration of 30-50 mol%; and DMG-PEG in a concentration of 0.5-5 mol%, wherein the sum of the concentrations does not exceed 100%.

In an embodiment, the lipid nanoparticle comprises at least two compounds: one ionizable lipid-like structure according to Formula (I) or pharmaceutically acceptable salt, tautomer, or stereoisomer thereof and a second ionizable lipid like compound. In a further embodiment, the first compound being the ionizable lipid-like structure according to Formula (I) and the second compound being another ionizable lipid like compound are present in a 2:1 to 1:1 ratio. In an embodiment, a first ionizable lipid-like structure according to Formula (I) or pharmaceutically acceptable salt, tautomer, or stereoisomer thereof, and a second ionizable lipid or lipid like compound, are present in the LNP composition at equimolar ratio. Accordingly, the first and the second lipid or lipid like compound are present in the composition at a 1:1 ratio. In an embodiment, the overall contribution of said components does not exceed 50 mol% in the overall lipid nanoparticle composition, preferably the overall contribution of said components is at least 25 mol%, more preferably the overall contribution of said components is about 35 mol%. In a particular embodiment, said two compounds are present in the LNP at equimolar ratio, whereby said first ionizable lipid-like compound is present between 10-30 mol% and said second ionizable lipid like compound is present between 10-30 mol%. In an alternative particular embodiment, the ratio of the first over the second ionizable lipid is higher than 1:1 and both lipids are present at 10-30 mol%.

The LNPs may be prepared using any method known in this art. These include, but are not limited to, spray drying, single and double emulsion solvent evaporation, solvent extraction, phase separation, simple and complex coacervation, and other methods well known to those of ordinary skill in the art. In certain embodiments, methods of preparing the particles are the double emulsion process and spray drying. The conditions used in preparing the particles may be altered to yield particles of a desired size or property (e.g., hydrophobicity, hydrophilicity, external morphology, "stickiness", shape, etc.). The method of preparing the particle and the conditions (e.g., solvent, temperature, concentration, air flow rate, etc.) used may also depend on the agent being encapsulated. Methods developed for making particles for delivery of encapsulated agents are described in the literature.

The lipid nanoparticles of the present invention may be prepared. More generally, the LNP's may be prepared using a method comprising:
- preparing a first alcoholic composition comprising one or more ionizable lipids according to Formula (I), a phospholipid different from Formula (I), a sterol, a PEG lipid, and a suitable alcoholic solvent;
- preparing a second aqueous composition comprising one or more nucleic acids and an aqueous solvent;
- mixing said first and second composition in a microfluidic mixing device.

In further step, the lipid components are combined in suitable concentrations in an alcoholic vehicle such as ethanol. Thereto, an aqueous composition comprising the nucleic acid is added, and subsequently loaded in a microfluidic mixing device.

The aim of microfluidic mixing is to achieve thorough and rapid mixing of multiple samples (i.e. lipid phase and nucleic acid phase) in a low pressure accurate mixing device. Such sample mixing is typically achieved by enhancing the diffusion effect between the different species flows. Thereto several low pressure accurate mixing devices can be used.

Other technologies suitable for preparing the LNP's of the present invention include dispersing the components in a suitable dispersing medium, for example, aqueous solvent and alcoholic solvent, and applying one or more of the following methods: ethanol dilution method, a simple hydration method, sonication, heating, vortex, an ether injecting method, a French press method, a cholic acid method, a Ca²⁺ fusion method, a freeze-thaw method, a reversed-phase evaporation method, T-junction mixing, Microfluidic Hydrodynamic Focusing, Staggered Herringbone Mixing, and the like.

If the particles prepared by any of the above methods have a size range outside of the desired range, the particles can be sized, for example, using a sieve. The particle may also be coated. In certain embodiments, the particles are coated with a targeting agent. In other embodiments, the particles are coated to achieve desirable surface properties (e.g., a particular charge).

In another aspect of the invention, a pharmaceutical composition or (RNA) vaccine comprising one or more lipid nanoparticles as defined above is disclosed. Said compositions or vaccines are particularly useful for veterinary and human use.

The pharmaceutical composition or vaccine may be formulated in an aqueous liquid, comprising one of more buffering agents. Examples of buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, d-gluconic acid, calcium glycerophosphate, calcium lactate, calcium lactobionate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, amino-sulfonate buffers (e.g. HEPES), magnesium hydroxide, aluminum hydroxide, alginic acid, pyrogen-free water, isotonic saline, Ringer's solution, ethyl alcohol, and/or combinations thereof.

Suitable routes of administration include parenteral administration. Formulations suitable for parenteral administration, such as - but not limited to - intraarticular, intravenous, intraperitoneal, intramuscular, intradermal or subcutaneous injection, include aqueous and non-aqueous, isotonic sterile injection solutions or suspensions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions are preferably administered, for example, by intravenous infusion, orally, topically, intraperitoneally, intravesically, or intrathecally.

The composition of (self-replicating) RNA molecules can be presented in unit-dose or multi-dose sealed containers, such as ampoules and vials. Injection solutions and suspensions can be prepared from sterile powders, granules, and tablets. Cells transfected by the (self-replicating) RNA molecules can also be administered intravenously or parenterally.

Said compositions or vaccines can be administered as a single dose or as a multi-dose, requiring a series of two or more doses, administered within a pre-defined timespan. Such timespan may be a week, two weeks, three weeks, four weeks, five weeks, six weeks, seven weeks, 8 weeks, 9 weeks, 10 weeks, 11 weeks up until one year.

In an embodiment, the compositions or vaccines are administered periodically, such as annually or bi-annually. A suited dose may be between 0.05 and 1 ml, more preferably between 0.25 and 0.75 ml, such as 0.5 ml.

The pharmaceutical composition or the vaccine are preferably sterile and may be sterilized by conventional sterilization techniques. The vaccines or compositions may contain pharmaceutically acceptable auxiliary substances, to approximate physiological conditions, such as pH adjusting and/or buffering agents and tonicity adjusting agents, for example sodium acetate, sodium chloride, potassium chloride, calcium chloride, sodium lactate and the like.

The tonicity of the compositions or vaccines may have to be adjusted with sodium salts, for example, sodium chloride. The tonicity of a pharmaceutical composition for parenteral administration is typically 0,9% or 9mg/ml NaCl.

The vaccines of the current invention may have an osmolality of between 200 mOsm/kg and 400 mOsm/kg, e.g. between 240-360 mOsm/kg or between 290-310 mOsm/kg.

Preservative-free vaccines are preferred. However, if desired, the vaccine of the invention may include one or more preservatives, such as phenol and 2-phenoxyethanol. Thiomersal, a mercury containing preservative, should be avoided as mercury-free compositions are preferred.

The vaccines of the invention is preferably non-pyrogenic e.g. containing <1 EU (endotoxin unit, a standard measure) per dose and preferably <0.1 EU per dose. The vaccine is preferably gluten free.

In a preferred embodiment, the compositions or RNA vaccines comprise saRNA molecules, said each saRNA molecule comprising a sequence encoding nonstructural alphavirus proteins and one or multiple sequences encoding an antigen.

The self-replicating RNA content of the compositions or RNA vaccines of the invention will generally be expressed in terms of the amount of RNA per dose. A preferred dose has between 0.1 to 100 µg self-replicating RNA, preferably between 0.5 to 90 µg self-replicating RNA, preferably between 0.1 to 75 µg self-replicating RNA, preferably between 0.1 to 50 µg self-replicating RNA, preferably between 0.5 to 50 µg self-replicating RNA, preferably between 0.5 to 25 µg self-replicating RNA, more preferably between 0.5 to 10 µg self-replicating RNA, more preferably between 1 and 10 µg, even more preferably between 1 and 5 µg self-replicating RNA and expression can be seen at much lower levels (e.g. 0.05 µg self-replicating RNA/dose during *in vitro* use).

Preferably, said alphavirus is a Venezuelan Equine Encephalitis Virus (VEEV). In a more particular embodiment, the alphavirus is a live attenuated Venezuelan Equine Encephalitis Virus (VEEV), such as strain TC-83 or a strain having at least 90% sequence identity, preferably at least 95%, more preferably at least 97%, even more preferably at least 99%. Strain TC-83 is publicly available and its genome is present in Genbank under accession number L01443.1.

Various genetically modified variants of alphaviruses have been generated that improve their use for self-replicating RNA molecule generation and vaccination, such as e.g. disclosed in US2015299728, WO1999018226 and US7332322, all of which are incorporated herein by reference. In particular, it has been found to be beneficial to have a guanine as the third nucleotide in the 5' UTR of the replicon and/or to have a Q739L mutation in Nonstructural Protein 2 (nsP2). Therefore, in a particular embodiment of the invention, the self-replicating RNA molecule comprises an A3G mutation in the 5' UTR region. In another particular embodiment, the self-replicating RNA molecule comprises a Q739L mutation in Nonstructural Protein 2 (nsP2). In a preferred embodiment, the self-replicating RNA molecule comprises a sequence encoding the nonstructural proteins of an alphavirus, particularly VEEV, more particularly VEEV TC-83, wherein the self-replicating RNA molecule comprises an A3G mutation in the 5' UTR and a Q739L mutation in nsP2. In an even more preferred embodiment, the self-replicating RNA molecule encodes the nonstructural proteins nsP1, nsP2, nsP3 and nsP4 of VEEV TC-83, wherein preferably the Q739L mutation is present in nsP2.

In another aspect, the invention relates to a pharmaceutical composition or vaccine for use in the treatment and/or prophylaxis of a disease, both for human and/or veterinary disorders. The invention provides a vaccine comprising one or more LNP's according to the present invention. The vaccine of the invention may be used for inducing an immune response, in particular an immune response against a disease-associated antigen or cells expressing a disease- associated antigen, such as an immune response against cancer. Accordingly, the vaccine may be used for prophylactic and/or therapeutic treatment of a disease involving a disease-associated antigen or cells expressing a disease- associated antigen, such as cancer. Preferably said immune response is a T cell response. In one embodiment, the disease- associated antigen is a tumor antigen or an antigen linked to an infectious disease. The antigen encoded by the RNA comprised in the LNPs described herein preferably is a disease-associated antigen or elicits an immune response against a disease-associated antigen or cells expressing a disease-associated antigen.

In another embodiment, the LNPs as described herein may be used in the framework of protein replacement therapies, in subjects in whom a particular protein is deficient or absent. In a further embodiment, the LNPs and formulations as described herein may be used in protein replacement therapies for rare (genetic) diseases.

The vaccine or pharmaceutical composition according to the current invention may be used in the prevention and/or treatment of infectious diseases caused by viruses, bacteria, fungi and/or parasites.

Without wanting to be limitative, said virus may be viruses belonging to Coronaviruses, Orthomyxoviruses, Paramyxoviridae viruses, Pneumoviruses, Rubulaviruses, Paramyxoviruses, Metapneumoviruses and Morbilliviruses, Poxviridae, Orthopoxvirus such as Variola vera, Picornaviruses, Enteroviruses, Rhinoviruses, Heparnaviruses, Cardioviruses, Aphthoviruses, Bunyavirus, Heparnaviruses, Filoviruses, Togaviruses, Flaviviruses, Pestiviruses, Hepadnaviruses, Other hepatitis viruses, Rhabdoviruses, Caliciviridae, Retroviruses, Reoviruses, Parvoviruses, Herpesviruses, Papovaviruses, Adenoviruses.

Without wanting to be limitative, said fungus may be a chosen from Dermatophytres, including: Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum var. album, var. discoides, var. ochraceum, Trichophyton violaceum, and/or Trichophyton faviforme; or from Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Microsporidia, Encephalitozoon spp., Septata intestinalis and Enterocytozoon bieneusi; the less common are Brachiola spp, Microsporidium spp., Nosema spp., Pleistophora spp., Trachipleistophora spp., Vittaforma spp Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Without wanting to be limitative, said parasite may be chosen from Plasmodium genus, such as P.falciparum, P.vivax, P.malariae or P.ovale. Thus the invention may be used for immunizing against malaria. In some embodiments the immunogen elicits an immune response against a parasite from the Caligidae family, particularly those from the Lepeophtheirus and Caligus genera e.g. sea lice such as Lepeophtheirus salmonis or Caligus rogercresseyi.

Without wanting to be limitative, said bacteria may be chosen from Neisseria meningitidis, Streptococcus pneumoniae, Streptococcus pyogenes, Moraxella catarrhalis, Bordetella pertussis, Staphylococcus aureus, Clostridium tetani, Cornynebacterium diphtheriae, Haemophilus influenzae, Pseudomonas aeruginosa, Streptococcus agalactiae, Chlamydia trachomatis, Chlamydia pneumoniae, Helicobacter pylori, Escherichia coli, Bacillus anthracis, Yersinia pestis, Staphylococcus epidermis, Clostridium perfringens or Clostridium botulinums, Legionella pneumophila, Coxiella burnetiid, Brucella, Francisella, Neisseria gonorrhoeae, Treponema pallidum, Haemophilus ducreyi, Enterococcus faecalis or Enterococcus faecium, Staphylococcus saprophyticus, Yersinia enterocolitica, Mycobacterium tuberculosis, Rickettsia, Listeria monocytogenes, Vibrio cholerae, Salmonella typhi, Borrelia burgdorferi, Porphyromonas gingivalis, Klebsiella.

In an embodiment, the treatment of cancer comprises administering to a subject in need thereof an effective amount of a vaccine or pharmaceutical composition according to an embodiment of the current invention. In certain embodiments, the method further comprises administering an anti-cancer agent.

Without wanting to be limitative, said tumor-antigens may be chosen from Cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors; (b) mutated antigens, for example, p53 (associated with various solid tumors, e.g., colorectal, lung, head and neck cancer), p21/Ras (associated with, e.g., melanoma, pancreatic cancer and colorectal cancer), CDK4 (associated with, e.g., melanoma), MUM1 (associated with, e.g., melanoma), caspase-8 (associated with, e.g., head and neck cancer), CIA 0205 (associated with, e.g., bladder cancer), HLA-A2-R1701, beta catenin (associated with, e.g., melanoma), TCR (associated with, e.g., T-cell non-Hodgkins lymphoma), BCR-ab1 (associated with, e.g., chronic myelogenous leukemia), triosephosphate isomerase, KIA 0205, CDC-27, and LDLR-FUT; (c) over-expressed antigens, for example, Galectin 4 (associated with, e.g., colorectal cancer), Galectin 9 (associated with, e.g., Hodgkin's disease), proteinase 3 (associated with, e.g., chronic myelogenous leukemia), WT 1 (associated with, e.g., various leukemias), carbonic anhydrase (associated with, e.g., renal cancer), aldolase A (associated with, e.g., lung cancer), PRAME (associated with, e.g., melanoma), HER-2/neu (associated with, e.g., breast, colon, lung and ovarian cancer), mammaglobin, alpha-fetoprotein (associated with, e.g., hepatoma), KSA (associated with, e.g., colorectal cancer), gastrin (associated with, e.g., pancreatic and gastric cancer), telomerase catalytic protein, MUC-1 (associated with, e.g., breast and ovarian cancer), G-250 (associated with, e.g., renal cell carcinoma), p53 (associated with, e.g., breast, colon cancer), and carcinoembryonic antigen (associated with, e.g., breast cancer, lung cancer, and cancers of the gastrointestinal tract such as colorectal cancer); (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, e.g., melanoma); (e) prostate associated antigens such as PAP, PSA, PSMA, PSH-P1, PSM-P1, PSM-P2, associated with e.g., prostate cancer; (f) immunoglobulin idiotypes (associated with myeloma and B cell lymphomas, for example). In certain embodiments, tumor immunogens include, but are not limited to, p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, TPS, and the like.

The invention is further described by the following non-limiting examples which further illustrate the invention, and are not intended to, nor should they be interpreted to, limit the scope of the invention.

### EXAMPLES

### 1. SYNTHESIS OF COMPOUNDS ACCORDING TO FORMULA I

### 1.1. General schemes

### 1.1.1. Synthetic route towards compound 9-4

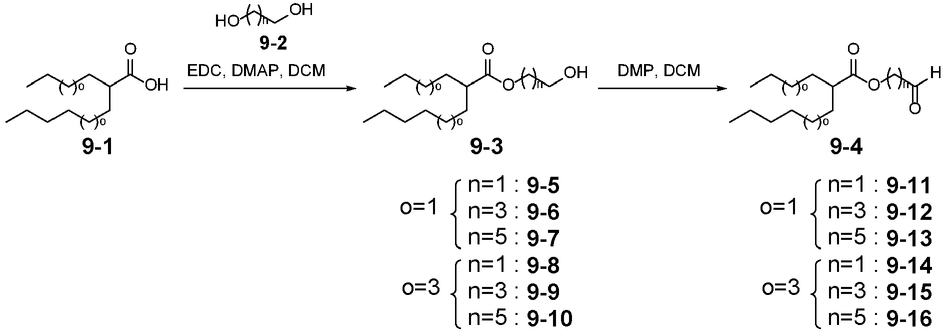

### 1.1.2. Synthetic route towards compound 9-19

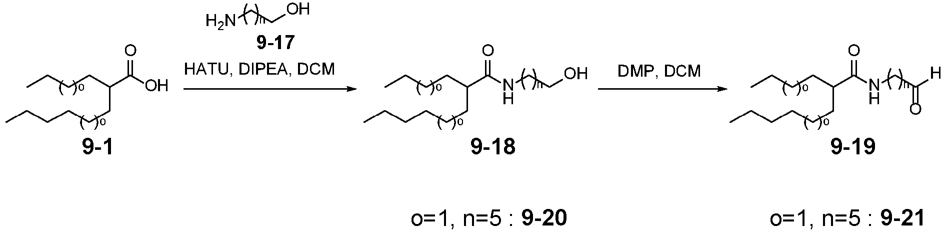

### 1.1.3. Synthetic route towards compounds 9-23 and 9-25

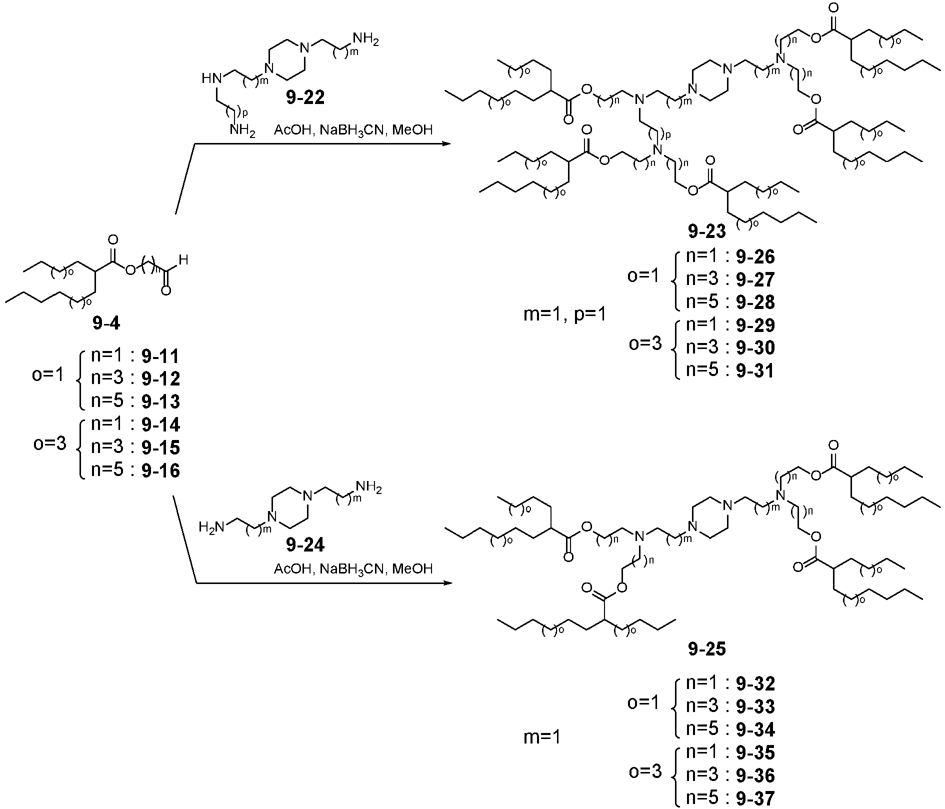

### 1.1.4. Synthetic route towards compounds 9-38 and 9-39

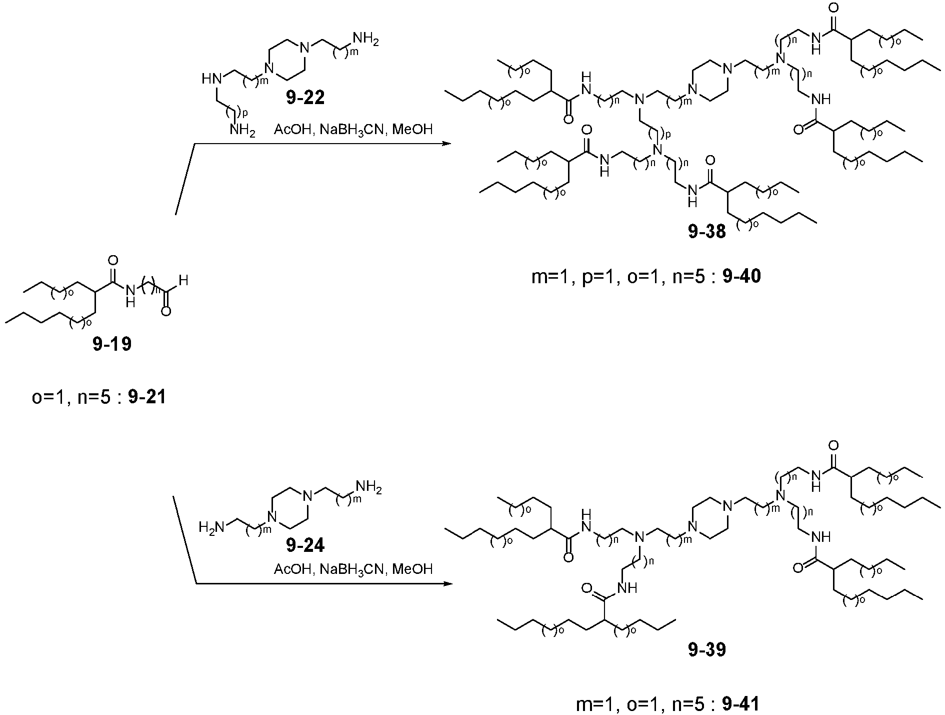

### 1.1.5. Synthetic route towards compound 9-46

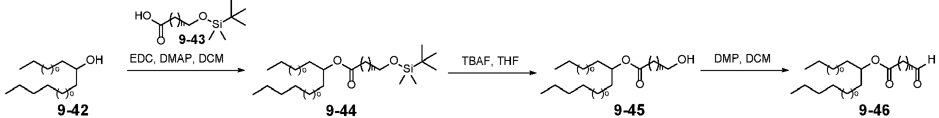

### 1.1.6. Synthetic route towards compound 9-50

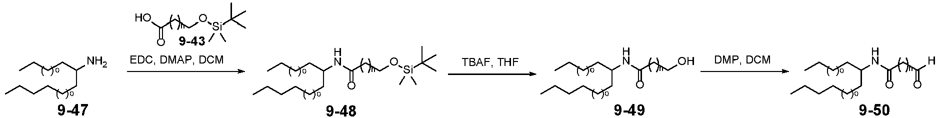

### 1.1.7. Synthetic route towards compounds 9-51 and 9-52

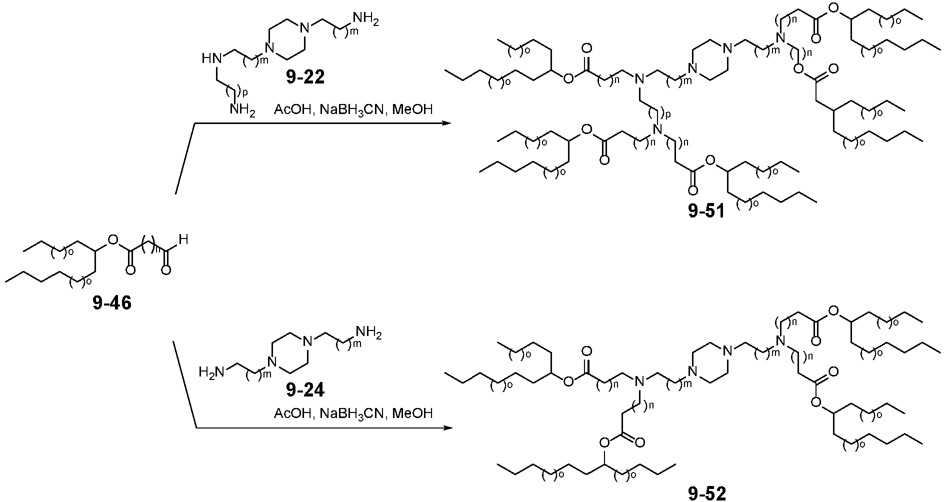

### 1.1.8. Synthetic route towards compounds 9-53 and 9-54

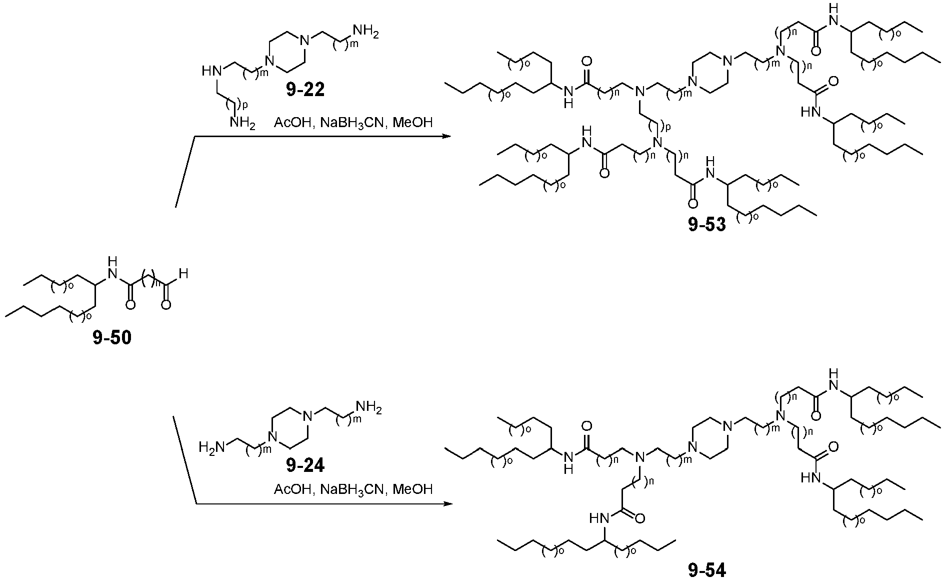

### 1.2. General experimental protocols for ZIPH009

### Abbreviations used

- AcOH: Acetic acid
- DCM: Dichloromethane
- DIPEA: Di-isopropylethylamine
- DMAP: 4-Dimethylaminopyridine
- DMP: Dess-Martin periodinane
- EDC: 1-(3-Dimethylaminopropyl)-3-ethylcarbodiimide Hydrochloride
- ESI: Electrospray ionization
- EtOAc: Ethyl acetate
- HATU: Hexafluorophosphate Azabenzotriazole Tetramethyl Uronium
- MeOH: Methanol
- MS: Mass spectrometry
- NMR: Nuclear magnetic resonance
- TBAF: Tetra-n-butylammonium fluoride
- THF: Tetrahydrofuran

### General Considerations.

All chemicals and solvents were used as obtained by their respective suppliers, unless otherwise noted. Acetone (99+%), dichloromethane (99.8+%), ethyl acetate (99.5+%), ethanol (99.8%), toluene (99.85%), 1,2-dichloroethane (99.8+%), methanol (99.8+%), ammonia (7N in methanol) and heptane (99+%) were purchased from a commercial source. The millipore water (MILLI-Q IQ 7005 PURIFICATION SYSTEM) was used.

Chromatographic purification was performed using an automated flash chromatography NextGen300+ system having ELSD and UV detectors utilizing commercially available normal phase Silica Flash Cartridges (12, 40, or 80 g) at a flow rate of 20-30 mL/min. Thin-layer chromatography (TLC) analysis was performed using precoated TLC aluminium sheets (DC Kleselgel 60 F254) /UV254 (layer: 0.20 mm silica gel with fluorescent indicator UV254). The spots were detected with UV light at 254 nm.

Nuclear magnetic resonance (NMR) spectra were recorded on a Bruker Model Avance II 400 (Deimos) and Bruker Model Avance II 700 (Hera) Fourier transform NMR spectrometer in CDCl₃ at 303 K (unless stated otherwise). Samples were prepared using ca. 10-30 mg of compound dissolved in 0.6-1.0 mL of deuterated solvents (CDCl₃). All spectra were referenced either to the TMS reference peak or solvent residual peak (e.g. δ = 0.00 ppm for ¹H and δ = 77.16 ppm for ¹³C in CDCl₃).

Chemical shifts (δ) are reported in ppm; coupling constants (J) are reported in Hz; splitting patterns are assigned as singlet (s), broad singlet (br s), doublet (d), triplet (t), quartet (q), quintet (quint), sextet (sext), septet (sept) multiplet (m) or combinations thereof.

Liquid chromatography-mass spectrometry (LC-MS)(Agilent LC/MSD and 1260 Infinity II LC System with Open LAB CDS ChemStation Edition and Thermo Scientific Charged Aerosol Detectors) samples were prepared by dissolving 0.1-1 mg of the compound in Acetonitrile: Isopropanol (1:1) mixture (~0.5 mL). Reverse phase column (CSH Phenyl-Hexyl Column, 130Å, 2.5 µm, 2.1 mm X 50 mm) was used. Two types of methods were used. In method1: The eluent used was 50% A (0.1% formic acid in H₂O), 12.5% B (0.1% formic acid in CH₃CN) and 37.5.5% c (0.1% formic acid in Isopropanol) at a flow rate of 0.350 mL/min over 33 min. In method2: The eluent used was 100% A (0.1% formic acid in H₂O) to 100% B (0.1% formic acid in CH₃CN) at a flow rate of 0.400 mL/min over 13.5 min.

### 1.2.1. Preparation of Compounds 9-11, 9-12, 9-13, 9-14, 9-15 and 9-16

### 1.2.1.1. Step 1:

Under Ar atmosphere, the diol **9-2** (3 equiv) was dissolved in DCM. To this solution were sequentially added the carboxylic acid **9-1** (1 equiv), EDC.HCl (1.1 equiv) and DMAP (0.8-1 equiv). The solution was stirred overnight at room temperature. The reaction mixture was then washed twice with (0.2M-0.5M) HCl, dried over anhydrous magnesium sulphate, filtered and the solvent was evaporated in vacuo. The residue was then purified by normal phase on a NextGen 300+ flash chromatography system (gradient from 100% Heptane to 20%/80% Heptane/EtOAc), yielding the ester intermediates **9-5, 9-6, 9-7, 9-8, 9-9** and **9-10.**

### 1.2.1.2. Step 2:

Under Ar atmosphere, the alcohol **9-3** (1 equiv) was dissolved in DCM. This solution was treated with Dess-Martin periodinane reagent (1.2-1.3 equiv), portion wise, at 0-10 °C. The reaction mixture was allowed to warm to room temperature and stirred for 4 h. The reaction mixture was then treated with a saturated Na₂S₂O₃ solution. The aqueous phase was discarded, and the organic phase was washed twice with a saturated NaHCO₃ solution. The combined organic phases were dried over anhydrous magnesium sulphate and evaporated in vacuo. Next, the crude residue was suspended in heptane and the white solid was removed by filtration. The solvents were evaporated in vacuo and the residue was then purified by normal phase on a NextGen 300+ flash chromatography system (gradient from 100% Heptane to 60%/40% Heptane/EtOAc), yielding the aldehyde intermediates **9-11, 9-12, 9-13, 9-14, 9-15** and **9-16.**

### 1.2.2. Preparation of Compound 9-21

### 1.2.2.1. Step 1:

Under Ar atmosphere, the amino alcohol **9-17** (1 equiv) was dissolved in DCM. To this solution were sequentially added the carboxylic acid **9-1** (1 equiv), HATU (1.1 equiv) and DIPEA (5 equiv). The solution was stirred overnight at room temperature. The reaction mixture was then washed twice with brine solution, dried over anhydrous magnesium sulphate, filtered and the solvent was evaporated in vacuo. The residue was then purified by normal phase on a NextGen 300+ flash chromatography system (gradient from 100% Heptane to 35%/75% Heptane/EtOAc), yielding the amide intermediate **9-20.**

### 1.2.2.2. Step 2:

Under Ar atmosphere, the alcohol **9-18** (1 equiv) was dissolved in DCM. This solution was treated with Dess-Martin periodinane reagent (1.55 equiv), portion wise, at 0-10 °C. The reaction mixture was allowed to warm to room temperature and stirred for 4 h. The reaction mixture was then treated with a saturated Na₂S₂O₃ solution. The aqueous phase was discarded, and the organic phase was washed twice with a saturated NaHCO₃ solution. The combined organic phases were dried over anhydrous magnesium sulphate, and evaporated in vacuo. Next, the crude residue was suspended in heptane and the white solid was removed by filtration. The solvents were evaporated in vacuo and the residue was then purified by normal phase on a NextGen 300+ flash chromatography system (gradient from 100% Heptane to 60%/40% Heptane/EtOAc), yielding the aldehyde intermediate **9-21.**

### 1.2.3. Preparation of Compounds 9-26, 9-27, 9-28, 9-29, 9-30 and 9-31

### 1.2.3.1. Step 1:

Under Ar atmosphere, the aldehyde intermediate **9-4** (5.5-7.5 equiv) was dissolved in MeOH and the solution was heated to 30°C. AcOH (10-30 equiv, 0.17-0.68 vol%) was then added to the solution, followed by N1-(2-(4-(2-aminoethyl)piperazin-1-yl)ethyl)ethane-1,2-diamine (1 equiv). After one minute, sodium cyanoborohydride (6.5-10 equiv) was added and was then allowed to stir at room temperature overnight. The solvents were evaporated in vacuo. The residue was then purified by normal phase on a NextGen 300+ flash chromatography system (gradient from 100% DCM to 95%/3.8%/1.2% DCM/MeOH/NH₃ 7N in MeOH), yielding compounds **9-26, 9-27, 9-28, 9-29, 9-30** and **9-31.**

### 1.2.4. Preparation of Compounds 9-32, 9-33, 9-34, 9-35, 9-36 and 9-37

### 1.2.4.1. Step 1:

Under Ar atmosphere, the aldehyde intermediate **9-4** (4.6-7 equiv) was dissolved in MeOH and the solution was heated to 30°C. AcOH (10-30 equiv, 0.17-0.68 vol%) was then added to the solution, followed by 2,2'-(Piperazine-1,4-diyl)diethanamine (1 equiv). After one minute, sodium cyanoborohydride (6.5-9 equiv) was added and was then allowed to stir at room temperature overnight. The solvents were evaporated in vacuo. The residue was then purified by normal phase on a NextGen 300+ flash chromatography system (gradient from 100% DCM to 95%/3.8%/1.2% DCM/MeOH/NH₃ 7N in MeOH), yielding compounds **9-32, 9-33, 9-34, 9-35, 9-36** and **9-37.**

### 1.2.5. Preparation of Compound 9-40

### 1.2.5.1. Step 1:

Under Ar atmosphere, the aldehyde intermediate **9-19** (5.6 equiv) was dissolved in MeOH and the solution was heated to 30°C. AcOH (30 equiv, 0.17 vol%) was then added to the solution, followed by N1-(2-(4-(2-aminoethyl)piperazin-1-yl)ethyl)ethane-1,2-diamine (1 equiv). After one minute, sodium cyanoborohydride (10 equiv) was added and was then allowed to stir at room temperature overnight. The solvents were evaporated in vacuo. The residue was then purified by normal phase on a NextGen 300+ flash chromatography system (gradient from 100% DCM to 95%/3.5%/1.5% DCM/MeOH/NH₃ 7N in MeOH), yielding compound **9-40.**

### 1.2.6. Preparation of Compounds 9-41

### 1.2.6.1. Step 1:

Under Ar atmosphere, the aldehyde intermediate **9-19** (5 equiv) was dissolved in MeOH and the solution was heated to 30°C. AcOH (25 equiv, 0.12 vol%) was then added to the solution, followed by 2,2'-(Piperazine-1,4-diyl)diethanamine (1 equiv). After one minute, sodium cyanoborohydride (9 equiv) was added and was then allowed to stir at room temperature overnight. The solvents were evaporated in vacuo. The residue was then purified by normal phase on a NextGen 300+ flash chromatography system (gradient from 100% DCM to 95%/3.5%/1.5% DCM/MeOH/NH₃ 7N in MeOH), yielding compounds **9-41.**

### 1.3. Detailed experimental protocols

### 1.3.1. Preparation of Compounds 9-11, 9-12, 9-13, 9-14, 9-15 and 9-16

### 1.3.1.1. Step 1:

| **Sample code** | **Experimental procedure** |
|---|---|
| 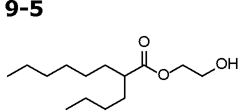 | Step 1 (1.2.1.1.) was followed using 2-butyloctanoic acid (6.00 g, 30.0 mmol, 1 equiv), ethylene glycol (5.58 g, 89.9 mmol, 3 equiv), EDC.HCl (6.32 g, 33.0 mmol, 1.1 equiv), and DMAP (2.93 g, 24.0 mmol, 0.8 equiv), in DCM (100 mL). The product was purified by normal phase flash chromatography (Heptane/EtOAc) to afford 2-hydroxyethyl 2-butyloctanoate (5.31 g, 21.7 mmol, yield 73%) as a transparent oil. |
| | MS (ESI) m/z 267.2 [M+Na]⁺ |
| | **¹H NMR (400MHz, CDCl₃):** δ 0.88 (3H, t, *J* = 6.85 Hz), 0.89 (3H, t, *J* = 7.06 Hz), 1.15 - 1.40 (12H, m), 1.40 - 1.54 (2H, m), 1.54 - 1.68 (2H, m), 1.90 (1H, t, *J* = 6.06 Hz), 2.36 (1H, tt, *J* = 13.29, 5.40 Hz), 3.80 - 3.86 (2H, m), 4.20 - 4.25 (2H, m). |
| 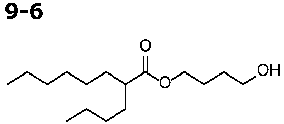 | Step 1 (1.2.1.1.) was followed using 2-butyloctanoic acid (6.00 g, 30.0 mmol, 1 equiv), butan-1,4-diol (8.10 g, 89.9 mmol, 3 equiv), EDC.HCl (6.32 g, 33.0 mmol, 1.1 equiv), and DMAP (2.93 g, 24.0 mmol, 0.8 equiv), in DCM (100 mL). The product was purified by normal phase flash chromatography (Heptane/EtOAc) to afford 4-hydroxybutyl 2-butyloctanoate (4.81 g, 17.7 mmol, yield 59%) as a transparent oil. |
| | MS (ESI) m/z 295.2 [M+Na]⁺ |
| | **¹H NMR (400MHz, CDCl₃):** δ 0.87 (3H, t, *J* = 6.94 Hz), 0.88 (3H, t, *J* = 7.08 Hz), 1.18 - 1.34 (12H, m), 1.37 (1H, t, *J* = 5.34 Hz) 1.39 - 1.50 (2H, m), 1.53 - 1.81 (6H, m), 2.30 (1H, tt, *J* = 13.44, 5.40 Hz), 3.69 (2H, q, *J* = 5.94 Hz), 4.11 (2H, t, *J* = 6.42 Hz). |
| 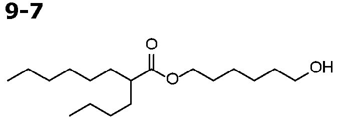 | Step 1 (1.2.1.1.) was followed using 2-butyloctanoic acid (4.64 g, 23.2 mmol, 1 equiv), 1,6-hexane-diol (8.22 g, 69.6 mmol, 3 equiv), EDC.HCl (4.89 g, 43 mmol, 1.1 equiv), and DMAP (2.27 g, 18.5 mmol, 0.8 equiv), in DCM (100 mL). The product was purified by normal phase flash chromatography (Heptane/EtOAc) to afford 6-hydroxyhexyl 2-butyloctanoate (4.773 g, 15.9 mmol, yield 69%) as a transparent oil. |
| | MS (ESI) m/z 323.2 [M+Na]⁺ |
| | **¹H NMR (400MHz, CDCl₃):** δ 0.87 (3H, t, *J* = 6.94 Hz), 0.88 (3H, t, *J* = 7.08 Hz), 1.17 - 1.31 (13H, m), 1.35 - 1.50 (6H, m), 1.52 - 1.69 (6H, m), 2.29 (1H, tt, *J* = 13.45, 5.31 Hz), 3.65 (2H, q, *J* = 6.15 Hz), 4.08 (2H, t, *J* = 6.62 Hz). |
| 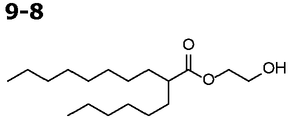 | Step 1 (1.2.1.1.) was followed using 2-hexyldecanoic acid (5.00 g, 19.5 mmol, 1 equiv), ethylene glycol (3.63 g, 58.5 mmol, 3 equiv), EDC.HCl (4.11 g, 21.5 mmol, 1.1 equiv), and DMAP (2.38 g, 19.5 mmol, 1 equiv), in DCM (60 mL). The product was purified by normal phase flash chromatography (Heptane/EtOAc) to afford 2-hydroxyethyl 2-hexyldecanoate (3.68 g, 12.2 mmol, yield 63%) as a transparent oil. |
| | MS (ESI) m/z 301.3 [M+H]⁺ |
| | **¹H NMR (400MHz, CDCl₃):** δ 0.87 (6H, distorted t, *J* = 6.66 Hz), 1.17 - 1.36 (20H, m), 1.38 - 1.52 (2H, m), 1.52 - 1.70 (2H, m), 1.90 (1H, t, *J* = 6.03 Hz), 2.36 (1H, tt, *J* = 13.17, 5.42 Hz), 3.77 - 3.86 (2H, m), 4.19 - 4.26 (2H, m). |
| 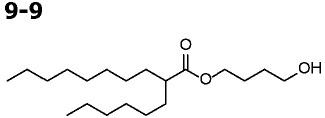 | Step 1 (1.2.1.1.) was followed using 2-hexyldecanoic acid (3.00 g, 11.7 mmol, 1 equiv), butan-1,4-diol (3.16 g, 35.1 mmol, 3 equiv), EDC.HCl (2.47 g, 12.9 mmol, 1.1 equiv), and DMAP (1.43 g, 11.7 mmol, 1 equiv), in DCM (50 mL). The product was purified by normal phase flash chromatography (Heptane/EtOAc) to afford 4-hydroxybutyl 2-hexyldecanoate (3.37 g, 10.3 mmol, yield 88%) as a transparent oil. |
| | MS (ESI) m/z 351.3 [M+Na]⁺ |
| | ¹H **NMR (400MHz, CDCl₃):** δ 0.87 (6H, distorted t, *J* = 6.60 Hz), 1.16 - 1.34 (20H, m), 1.33 (1H, t, *J* = 5.36 Hz), 1.36 - 1.50 (2H, m), 1.50 - 1.80 (6H, m), 2.30 (1H, tt, *J* = 13.28, 5.34 Hz), 3.69 (2H, q, *J* = 5.90 Hz), 4.11 (2H, t, *J* = 6.35 Hz). |
| 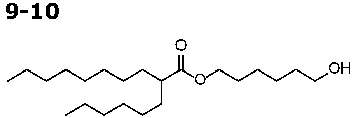 | Step 1 (1.2.1.1.) was followed using 2-hexyldecanoic acid (10.0 g, 39.0 mmol, 1 equiv), 1,6-hexane-diol (13.8 g, 117 mmol, 3 equiv), EDC.HCl (8.22 g, 42.9 mmol, 1.1 equiv), and DMAP (3.81 g, 31.2 mmol, 0.8 equiv), in DCM (70 mL). The product was purified by normal phase flash chromatography (Heptane/EtOAc) to afford 6-hydroxyhexyl 2-hexyldecanoate (7.89 g, 22.1 mmol, yield 57%) as a transparent oil. |
| | MS (ESI) m/z 357.3 [M+H]⁺ |
| | **¹H NMR (400MHz, CDCl₃):** δ 0.87 (6H, distorted t, *J* = 6.66 Hz), 1.17 - 1.36 (21H, m), 1.36 - 1.48 (6H, m), 1.52 - 1.69 (6H, m), 2.29 (1H, tt, *J* = 13.48, 5.28 Hz), 3.65 (2H, q, *J* = 5.63 Hz), 4.07 (2H, t, *J* = 6.62 Hz). |
| | **¹³C NMR (101MHz, CDCl₃):** δ 14.20, 14.23, 22.7, 22.8, 25.5, 25.9, 27.57, 27.61, 28.8, 29.36, 29.38, 29.6, 29.7, 31.8, 32.0, 32.7, 32.8, 46.0, 63.0, 64.1, 176.9. |

### 1.3.1.2. Step 2:

| **Sample code** | **Experimental procedure** |
|---|---|
| 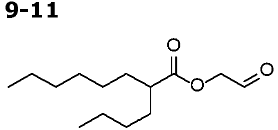 | Step 2 (1.2.1.2.) was followed using **9-5** (5.31 g, 21.7 mmol, 1 equiv) as starting material, and DMP (11.1 g, 26.1 mmol, 1.2 equiv), in DCM (100 mL). The product was purified by normal phase flash chromatography (Heptane/EtOAc) to afford 2-oxoethyl 2-butyloctanoate (5.71 g, 23.5 mmol, yield 54%) as a transparent oil. |
| | MS (ESI) m/z 243.2 [M+Na]⁺ |
| | ¹H **NMR (400MHz, CDCl₃):** δ 0.88 (3H, t, *J* = 5.93 Hz), 0.89 (3H, t, *J* = 7.98 Hz), 1.16 - 1.38 (12H, m), 1.42 - 1.54 (2H, m), 1.57 - 1.84 (2H, m), 2.46 (1H, tt, *J* = 13.18, 5.41 Hz), 4.65 (2H, d, *J* = 0.6 Hz), 9.61 (1H, t, *J* = 0.65 Hz). |
| 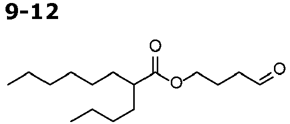 | Step 2 (1.2.1.2.) was followed using **9-6** (4.81 g, 17.7 mmol, 1 equiv) as starting material, and DMP (8.99 g, 21.2 mmol, 1.2 equiv), in DCM (100 mL). After 4 hours an additional 0.1 equiv of DMP (749 mg, 1.77 mmol, 0.1 equiv) was added and the reaction mixture was stirred for 1 hour. The product was purified by normal phase flash chromatography (Heptane/EtOAc) to afford 4-oxobutyl 2-butyloctanoate (3.82 g, 14.1 mmol, yield 80%) as a transparent oil. |
| | MS (ESI) m/z 293.2 [M+Na]⁺ |
| | **¹H NMR (400MHz, CDCl₃):** δ 0.87 (3H, t, *J* = 6.90 Hz), 0.88 (3H, t, *J* = 7.10 Hz), 1.16 - 1.38 (12H, m), 1.38 - 1.51 (2H, m), 1.52 - 1.67 (2H, m), 1.93 - 2.03 (2H, m), 2.30 (1H, tt, *J* = 13.34, 5.38 Hz), 2.54 (2H, td, *J* = 7.23, 1.28 Hz), 4.11 (2H, t, *J* = 6.37 Hz), 9.80 (1H, t, *J* = 1.30 Hz). |
| 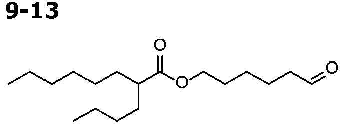 | Step 2 (1.2.1.2.) was followed using **9-7** (4.77 g, 15.9 mmol, 1 equiv) as starting material, and DMP (8.09 g, 19.1 mmol, 1.2 equiv), in DCM (100 mL). After 4 hours an additional 0.1 equiv of DMP (674 mg, 1.59 mmol, 0.1 equiv) was added and the reaction mixture was stirred for 1 hour. The product was purified by normal phase flash chromatography (Heptane/EtOAc) to afford 2-oxohexyl 2-butyloctanoate (3.29 g, 11.0 mmol, yield 69%) as a transparent oil. |
| | MS (ESI) m/z 321.2 [M+Na]⁺ |
| | **¹H NMR (400MHz, CDCl₃):** δ 0.87 (3H, t, *J* = 6.94 Hz), 0.88 (3H, t, *J* = 7.08 Hz), 1.16 - 1.36 (12H, m), 1.36 - 1.51 (4H, m), 1.51 - 1.60 (2H, m), 1.61 - 1.75 (4H, m), 2.31 (1H, tt, *J* = 13.44, 5.32 Hz), 2.45 (2H, td, *J* = 7.33, 1.67 Hz), 4.08 (2H, t, *J* = 6.56 Hz), 9.77 (1H, t, *J* = 1.69 Hz). |
| 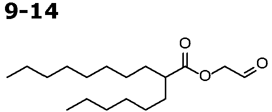 | Step 2 (1.2.1.2.) was followed using **9-8** (5.17 g, 17.2 mmol, 1 equiv) as starting material, and DMP (8.76 g, 20.7 mmol, 1.2 equiv), in DCM (100 mL). The product was purified by normal phase flash chromatography (Heptane/EtOAc) to afford 2-oxoethyl 2-hexyldecanoate (4.57 g, 15.3 mmol, yield 89%) as a transparent oil. |
| | MS (ESI) m/z 299.2 [M+H]⁺ |
| | **¹H NMR (300MHz, CDCl₃):** δ 0.88 (6H, distortet t, *J* = 6.62 Hz), 1.16 - 1.37 (20H, m), 1.38 - 1.57 (2H, m), 1.58 - 1.75 (2H, m), 2.45 (1H, tt, *J* = 13.07, 5.41 Hz), 4.65 (2H, s), 9.60 (1H, s). |
| 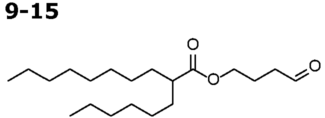 | Step 2 (1.2.1.2.) was followed using **9-9** (3.37 g, 10.3 mmol, 1 equiv) as starting material, and DMP (5.22 g, 12.3 mmol, 1.2 equiv), in DCM (50 mL). The product was purified by normal phase flash chromatography (Heptane/EtOAc) to afford 4-oxobutyl 2-hexyldecanoate (2.91 g, 8.91 mmol, yield 87%) as a transparent oil. |
| | MS (ESI) m/z 349.3 [M+Na]⁺ |
| | **¹H NMR (400MHz, CDCl₃):** δ 0.87 (6H, distortet t, *J* = 6.68 Hz), 1.12 - 1.36 (20H, m), 1.36 - 1.50 (2H, m), 1.50 - 1.67 (2H, m), 1.98 (2H, p, *J* = 6.80 Hz), 2.30 (1H, tt, *J* = 13.24, 5.39 Hz), 2.53 (2H, td, *J* = 10.85, 1.19 Hz), 4.11 (2H, t, *J* = 6.36 Hz), 9.80 (1H, t, *J* = 1.22 Hz). |
| 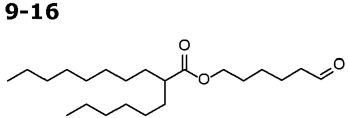 | Step 2 (1.2.1.2.) was followed using **9-10** (7.80 g, 21.9 mmol, 1 equiv) as starting material, and DMP (11.1 g, 26.3 mmol, 1.2 equiv), in DCM (150 mL). The product was purified by normal phase flash chromatography (Heptane/EtOAc) to afford 6-oxohexyl 2-hexyldecanoate (6.93 g, 19.5 mmol, yield 89%) as a transparent oil. |
| | MS (ESI) m/z 377.3 [M+Na]⁺ |
| | **¹H NMR (400MHz, CDCl₃):** δ 0.88 (6H, distorted t, *J* = 6.64 Hz), 1.18 - 1.34 (20H, m), 1.35 - 1.49 (4H, m), 1.52 - 1.63 (2H, m), 1.67 (4H, sext, *J* = 7.23 Hz), 2.25 - 2.39 (1H, tt, *J* = 13.44, 5.31 Hz), 2.44 (2H, td, *J* = 10.99, 1.65 Hz), 4.07 (2H, t, *J* = 6.56 Hz), 9.77 (1H, t, *J* = 1.68 Hz). |
| | **¹³C NMR (101MHz, CDCl₃):** δ 14.20, 14.24, 21.8, 22.7, 22.8, 25.73, 27.57, 27.62, 28.6, 29.35, 29.39, 29.6, 29.7, 31.8, 32.0, 32.7, 43.9, 46.0, 63.8, 176.8, 202.4. |

### 1.3.2. Preparation of Compound 9-20

### 1.3.2.1. Step 1:

| **Sample code** | **Experimental procedure** |
|---|---|
| 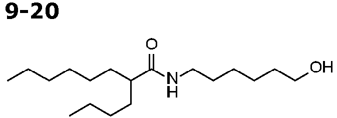 | Step 1 (1.2.2.1.) was followed using 2-butyloctanoic acid (1.00 g, 4.99 mmol, 1 equiv), 6-aminohexan-1-ol (585 mg, 4.99 mmol, 1 equiv), HATU (2.09 g, 5.49 mmol, 1.1 equiv), and N-ethyl-N-isopropylpropan-2-amine (4.43 mL, 25.0 mmol, 5 equiv), in DCM (15 mL). The product was purified by normal phase flash chromatography (Heptane/EtOAc) to afford 2-butyl-N-(6-hydroxyhexyl)octanamide (1.40 g, 4.67 mmol, yield 94%) as a transparent oil. |
| | MS (ESI) m/z 300.3 [M+H]⁺ |
| | **¹H NMR (400MHz, CDCl₃):** δ 0.87 (3H, t, *J* = 6.87 Hz), 0.88 (3H, t, *J* = 7.02 Hz), 1.17- 1.32 (12H, m), 1.32-1.46 (6H, m), 1.48-1.63 (6H, m), 1.94 (1H, sept, *J* = 4.7 Hz), 3.26 (2H, q, *J* = 6.65 Hz,), 3.63 (2H, t, *J* = 6.23 Hz,), 5.47 (1H, t, *J* = 5.33 Hz). |

### 1.3.2.2. Step 2:

| **Sample code** | **Experimental procedure** |
|---|---|
| 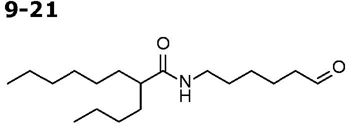 | Step 2 (1.2.2.2.) was followed using **9-20** (1.20 g, 4.01 mmol, 1 equiv) as starting material, and DMP (2.6 g, 6.2 mmol, 1.55 equiv), in DCM (60 mL). The product was purified by normal phase flash chromatography (Heptane/EtOAc) to afford 2-butyl-N-(6-oxohexyl)octanamide (770 mg, 2.59 mmol, yield 65%) as a white solid. |
| | MS (ESI) m/z 298.3 [M+H]⁺ |
| | **¹H NMR (300MHz, CDCl₃):** δ 0.87 (6H, distorted t, *J* = 6.93 Hz), 1.21-1.30 (12H, m), 1.33-1.40 (m, 4H), 1.48-1.71 (6H, m), 1.95 (1H, sept, *J* = 4.69 Hz), 2.44 (2H, td, *J* = 7.21, 1.52 Hz), 3.27 (2H, q, *J* = 6.64 Hz), 5.50 (1H, br s), 9.76 (1H, t, *J* = 1.61 Hz). |

### 1.3.3. Preparation of Compounds 9-26, 9-27, 9-28, 9-29, 9-30 and 9-31

### 1.3.3.1. Step 1:

| **Sample code** | **Experimental procedure** |
|---|---|
| **9-26** 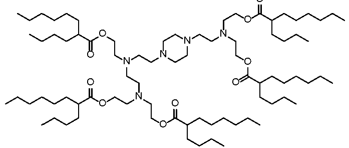 | Step 1 (1.2.3.1.) was followed using **9-11** (500 mg, 2.06 mmol, 5.6 equiv), N1-(2-(4-(2-aminoethyl)piperazin-1-yl)ethyl)ethane-1,2-diamine (79.3 mg, 368 µmol, 1 equiv) as starting materials, and NaBH₃CN (231 mg, 3.68 mmol, 10 equiv) as reducing agent, with 0.17 vol% AcOH (632 µL, 11.0 mmol, 30 equiv) in MeOH (368 mL). The product was purified by normal phase flash chromatography to afford ((2-(4-(2-((2-(bis(2-((2-butyloctanoyl)oxy)ethyl)amino)ethyl)(2-((2-butyloctanoyl)oxy)ethyl)amino)ethyl)piperazi n-1-yl)ethyl)azanediyl)bis(ethane-2,1-diyl) bis(2-butyloctanoate) (247 mg, 184 µmol, yield 50%) as a transparent oil. |
| | MS (ESI) m/z 542.9 [M+3H]³⁺ |
| | **¹H NMR (400MHz, CDCl₃):** δ 0.87 (15H, t, *J* = 6.88 Hz), 0.88 (15H, t, *J* = 7.03 Hz), 1.17-.36 (60H, m), 1.37-1.50 (10H, m), 1.51-1.65 (10H, m), 2.31 (5H, tt, *J* = 8.9, 5.4 Hz), 2.36-2.85 (30H, m), 4.12 (10H, td, *J* = 6.3, 3.2 Hz). |
| | **¹³C NMR (176MHz, CDCl₃):** δ 13.9, 14.0, 22.57, 22.60, 27.4, 29.21, 29.22, 29.6, 31.7, 32.09, 32.10, 32.40, 32.42, 45.68, 45.70, 52.4, 53.29, 53.32, 53.34, 53.5, 53.7, 56.8, 62.0, 62.1, 176.39, 176.44. |
| | Remark: Not all of the carbons of the N1-(2-(4-(2-aminoethyl)piperazin-1-yl)ethyl)ethane-1,2-diamine core are detected. |
| **9-27** 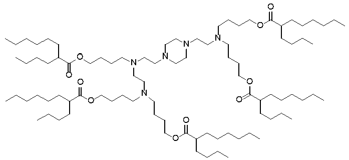 | Step 1 (1.2.3.1.) was followed using **9-12** |
| | (500 mg, 1.85 mmol, 5.6 equiv), N1-(2-(4-(2-aminoethyl)piperazin-1-yl)ethyl)ethane-1,2-diamine (71.1 mg, 330 µmol, 1 equiv) as starting materials, and NaBH₃CN (156 mg, 2.48 mmol, 7.5 equiv) as reducing agent, with 0.17 vol% AcOH (567 µL, 9.91 mmol, 30 equiv) in MeOH (330 mL). The product was purified by normal phase flash chromatography to afford ((2-(4-(2-((2-(bis(4-((2-butyloctanoyl)oxy)butyl)amino)ethyl)(4-((2-butyloctanoyl)oxy)butyl)amino)ethyl)piperaz in-1-yl)ethyl)azanediyl)bis(butane-4,1-diyl) bis(2-butyloctanoate) (186 mg, 125 µmol, yield 38%) as a transparent oil. |
| | MS (ESI) m/z 496.6 [M+3H]³⁺ |
| | **¹H NMR (400MHz, CDCl₃):** δ 0.87 (15H, t, J = 6.87 Hz), 0.88 (15H, t, J = 7.07 Hz), 1.17-1.37 (60H, m), 1.37-1.50 (10H, m), 1.51-1.75 (30H, br m), 2.32 (5H, tt, J = 8.9, 5.5 Hz), 2.53-3.19 (30H, m), 4.09 (10H, t, J = 5.74 Hz). |
| | **¹³C NMR (176MHz, CDCl₃):** δ 13.95, 14.04, 22.57, 22.58, 26.4, 26.5, 27.4, 29.2, 29.6, 31.7, 32.08, 32.09, 32.40, 32.41, 45.65, 45.67, 53.2, 63.2, 176.61, 176.63, 176.66. |
| | Remark: Not all of the carbons of the N1-(2-(4-(2-aminoethyl)piperazin-1-yl)ethyl)ethane-1,2-diamine core are detected. |
| **9-28** 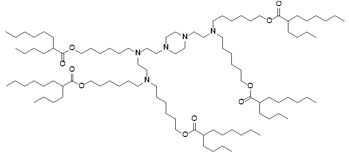 | Step 1 (1.2.3.1.) was followed using **9-13** (500 mg, 1.68 mmol, 5.5 equiv), N1-(2-(4-(2-aminoethyl)piperazin-1-yl)ethyl)ethane-1,2-diamine (65.6 mg, 305 µmol, 1 equiv) as starting materials, and NaBH₃CN (144 mg, 2.28 mmol, 7.5 equiv) as reducing agent, with 0.17 vol% AcOH (523 µL, 9.14 mmol, 30 equiv) in MeOH (305 mL). The product was purified by normal phase flash chromatography to afford ((2-(4-(2-((2-(bis(6-((2-butyloctanoyl)oxy)hexyl)amino)ethyl)(6-((2-butyloctanoyl)oxy)hexyl)amino)ethyl)piperaz in-1-yl)ethyl)azanediyl)bis(hexane-6,1-diyl) bis(2-butyloctanoate) (249 mg, 153 µmol, yield 50%) as a transparent oil. |
| | MS (ESI) m/z 543.4 [M+3H]³⁺ |
| | **¹H NMR (400MHz, CDCl₃):** δ 0.87 (15H, t, *J* = 6.92 Hz), 0.88 (15H, t, *J* = 7.09 Hz), 1.16-1.31 (60H, m), 1.32-1.50 (30H, m), 1.50-1.71 (30H, m), 2.30 (5H, tt, *J* = 13.45, 5.32 Hz), 2.38-3.19 (30H, br), 4.07 (10H, t, *J* = 6.6 Hz). |
| | **¹³C NMR (176MHz, CDCl₃):** δ 13.9, 14.0, 22.55, 22.58, 25.7, 25.8, 27.0, 27.4, 28.58, 28.63, 29.2, 29.6, 31.7, 32.2, 32.5, 45.7, 63.76, 63.81, 176.65. |
| | Remark: Not all of the carbons of the N1-(2-(4-(2-aminoethyl)piperazin-1-yl)ethyl)ethane-1,2-diamine core are detected. |
| **9-29** 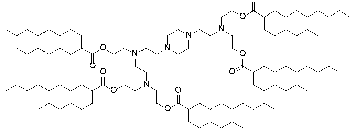 | Step 1 (1.2.3.1.) was followed using **9-14** (438 mg, 1.34 mmol, 7.5 equiv), N1-(2-(4-(2-aminoethyl)piperazin-1-yl)ethyl)ethane-1,2-diamine (38.5 g, 179 µmol, 1 equiv) as starting materials, and NaBH₃CN (73.0 mg, 1.16 mmol, 6.5 equiv) as reducing agent, with 0.68 vol% AcOH (102 µL, 1.79 mmol, 10 equiv) in MeOH (15 mL). The product was purified by normal phase flash chromatography to afford ((2-(4-(2-((2-(bis(2-((2-hexyldecanoyl)oxy)ethyl)amino)ethyl)(2-((2-hexyldecanoyl)oxy)ethyl)amino)ethyl)pipera zin-1-yl)ethyl)azanediyl)bis(ethane-2,1-diyl) bis(2-hexyldecanoate) (188 mg, 115 µmol, yield 64%) as a transparent oil. |
| | MS (ESI) m/z 542.9 [M+3H]³⁺ |
| | **¹H NMR (400MHz, CDCl₃):** δ 0.87 (30H, distortet t, *J* = 6.84 Hz), 1.17-1.33 (100H, m), 1.37-1.49 (10H, m), 1.50-1.64 (10H, m), 2.24-2.35 (5H, m), 2.41-3.28 (20H, br m), 2.71-2.85 (10H, m), 4.12 (10H, q, *J* = 5.16 Hz). |
| | **¹³C NMR (176MHz, CDCl₃):** δ 14.2, 14.3, 22.75, 22.81, 27.57, 27.62, 29.39, 29.40, 29.44, 29.5, 29.6, 29.7, 29.75, 29.83, 31.8, 32.0, 32.5, 45.86, 45.88, 53.4, 53.6, 61.9, 176.61, 176.64. |
| | Remark: Not all of the carbons of the N1-(2-(4-(2-aminoethyl)piperazin-1-yl)ethyl)ethane-1,2-diamine core are detected. |
| **9-30** 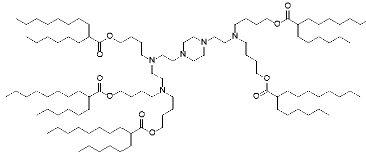 | Step 1 (1.2.3.1.) was followed using **9.15** (400 mg, 1.23 mmol, 5.7 equiv), N1-(2-(4-(2-aminoethyl)piperazin-1-yl)ethyl)ethane-1,2-diamine (46.3 g, 215 µmol, 1 equiv) as starting materials, and NaBH₃CN (135 mg, 2.15 mmol, 10 equiv) as reducing agent, with 0.17 vol% AcOH (369 µL, 6.45 mmol, 30 equiv) in MeOH (215 mL). The product was purified by normal phase flash chromatography to afford ((2-(4-(2-((2-(bis(4-((2-hexyldecanoyl)oxy)butyl)amino)ethyl)(4-((2-hexyldecanoyl)oxy)butyl)amino)ethyl)pipera zin-1-yl)ethyl)azanediyl)bis(butane-4,1-diyl)bis(2-hexyldecanoate) (286 mg, 162 µmol, yield 75%) as a transparent oil. |
| | MS (ESI) m/z 590.1 [M+3H]³⁺ |
| | **¹H NMR (700MHz, CDCl₃):** δ 0.86 (15H, t, *J* = 10.56 Hz), 0.87 (15H, t, *J* = 10.57 Hz), 1.19-1.32 (100H, m), 1.38-1.52 (20H, m), 1.53-1.65 (20H, m), 2.30 (5H, sept, *J* = 4.71 Hz), 2.36-2.65 (30H, m), 4.06 (10H, t, *J* = 6.71 Hz). |
| | **¹³C NMR (176MHz, CDCl₃):** δ 13.23, 13.26, 21.75, 21.82, 22.85, 22.90, 25.87, 25.89, 26.57, 26.62, 28.38, 28.41, 28.60, 28.73, 30.85, 31.0, 31.63, 31.65, 44.95, 44.96, 50.7, 51.2, 51.7, 52.273, 52.9, 53.25, 53.3, 53.9, 55.9, 56.0, 63.1, 175.76, 175.8. |
| **9-31** 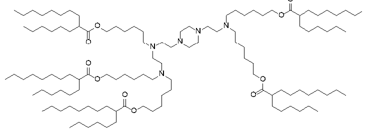 | Step 1 (1.2.3.1.) was followed using **9-16** (501 mg, 1.41 mmol, 5.6 equiv), N1-(2-(4-(2-aminoethyl)piperazin-1-yl)ethyl)ethane-1,2-diamine (54.3 mg, 252 µmol, 1 equiv) as starting materials, and NaBH₃CN (158 mg, 2.52 mmol, 10 equiv) as reducing agent, with 0.17 vol% AcOH (433 µL, 30 equiv) in MeOH (252 mL). The product was purified by normal phase flash chromatography to afford ((2-(4-(2-((2-(bis(6-((2-hexyldecanoyl)oxy)hexyl)amino)ethyl)(6-((2-hexyldecanoyl)oxy)hexyl)amino)ethyl)pipera zin-1-yl)ethyl)azanediyl)bis(hexane-6,1-diyl) bis(2-hexyldecanoate) (152 mg, 79.5 µmol, yield 32%) as a transparent oil. |
| | MS (ESI) m/z 636.8 [M+3H]³⁺ |
| | **¹H NMR (700MHz, CDCl₃):** δ 0.87 (30H, distorted t, *J* = 6.88 Hz), 1.16-1.33 (100H, m), 1.33-1.49 (30H, m), 1.50-1.73 (30H, m), 2.28 (5H, tt, *J* = 13.66, 5.13 Hz), 2.32-2.62 (30H, m), 4.04 (10H, t, *J* = 6.88 Hz). |
| | **¹³C NMR (176MHz, CDCl₃):** δ 14.0, 14.1, 22.56, 22.63, 25.9, 27.06, 27.10, 27.15, 27.18, 27.22, 27.25, 27.39, 27.43, 28.7, 29.19, 29.22, 29.4, 29.5, 29.7, 31.7, 31.8, 32.5, 45.8, 51.5, 52.0, 52.5, 53.0, 53.66, 53.68, 54.6, 54.7, 55.2, 56.69, 56.74, 64.0, 176.6, 176.7. |

### 1.3.4. Preparation of Compounds 9-32, 9-33, 9-34, 9-35, 9-36 and 9-37

### 1.3.4.1. Step 1:

| **Sample code** | **Experimental procedure** |
|---|---|
| 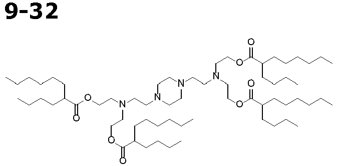 | Step 1 (1.2.4.1.) was followed using **9-11** (400 mg, 1.65 mmol, 4.6 equiv), 2,2-(Piperazine-1,4-diyl)diethanamine (61.8 mg, 359 µmol, 1 equiv) as starting materials, and NaBH₃CN (203 mg, 3.23 mmol, 9 equiv) as reducing agent, with 0.17 vol% AcOH (616 µL, 10.8 mmol, 30 equiv) in MeOH (359 mL). The product was purified by normal phase flash chromatography to afford ((piperazine-1,4-diylbis(ethane-2,1-diyl))bis(azanetriyl))tetrakis(ethane-2,1-diyl) tetrakis(2-butyloctanoate) (241 mg, 224 µmol, yield 62%) as a transparent oil. MS (ESI) m/z 449.9 [M+3H]³⁺, 674.7 [M+2H]²⁺ |
| | **¹H NMR (400MHz, CDCl₃):** δ 0.87 (12H, t, *J* = 6.90 Hz), 0.88 (12H, t, *J* = 7.08 Hz), 1.18-1.35 (48H, m), 1.37-1.51 (8H, m), 1.59 (8H, m), 2.30 (4H, tt, *J* = 13.23, 5.39 Hz), 2.34-3.09 (24H, br), 4.12 (8H, t, *J* = 6.3 Hz). |
| | **¹³C NMR (101MHz, CDCl₃):** δ 13.98, 14.08, 22.62, 22.64, 27.45, 29.3, 29.7, 31.771, 32.1, 32.5, 45.774, 53.26, 53.35, 56.8, 62.2, 176.5. |
| 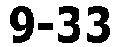 | Step 1 (1.2.4.1.) was followed using **9-12** (400 mg, 1.48 mmol, 4.6 equiv), 2,2- |
| 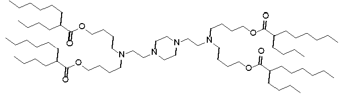 | (Piperazine-1,4-diyl)diethanamine (55.4 mg, 322 µmol, 1 equiv) as starting materials, and NaBH₃CN (182 mg, 2.89 mmol, 9 equiv) as reducing agent, with 0.17 vol% AcOH (552 µL, 9.65 mmol, 30 equiv) in MeOH (322 mL). The product was purified by normal phase flash chromatography to afford ((piperazine-1,4-diylbis(ethane-2,1-diyl))bis(azanetriyl))tetrakis(butane-4,1-diyl) tetrakis(2-butyloctanoate) (287 mg, 241 µmol, yield 75%) as a transparent oil. MS (ESI) m/z 595.7 [M+2H]²⁺ |
| | **¹H NMR (400MHz, CDCl₃):** δ 0.87 (12H, t, *J* = 6.92 Hz), 0.88 (12H, t, *J* = 7.08 Hz), 1.16-1.36 (48H, m), 1.36-1.53 (16H, m), 1.54-1.69 (16H, m), 2.31 (4H, tt, *J* = 13.37, 5.34 Hz), 2.37-2.78 (24H, br), 4.07 (8H, t, *J* = 6.6 Hz). |
| | **¹³C NMR (101MHz, CDCl₃):** δ 13.98, 14.09, 22.61, 22.64, 23.7, 26.7, 27.4, 29.2, 29.7, 31.7, 32.2, 32.5, 45.8, 51.5, 53.7, 54.1, 56.7, 64.0, 176.7. |
| 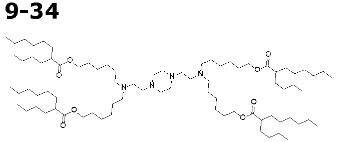 | Step 1 (1.2.4.1.) was followed using **9-13** (500 mg, 1.67 mmol, 4.6 equiv), 2,2-(Piperazine-1,4-diyl)diethanamine (62.7 mg, 364 µmol, 1 equiv) as starting materials, and NaBH₃CN (206 mg, 3.28 mmol, 9 equiv) as reducing agent, with 0.17 vol% AcOH (625 µL, 10.9 mmol, 30 equiv) in MeOH (364 mL). The product was purified by normal phase flash chromatography to afford ((piperazine-1,4-diylbis(ethane-2,1-diyl))bis(azanetriyl))tetrakis(hexane-6,1-diyl) tetrakis(2-butyloctanoate) (97.3 mg, 74.7 µmol, yield 21%) as a transparent oil. MS (ESI) m/z 651,8 [M+2H]²⁺ |
| | **¹H NMR (400MHz, CDCl₃):** δ 0.87 (12H, t, *J* = 6.92 Hz), 0.88 (12H, t, *J* = 7.07 Hz), 1.18-1.38 (64H, m), 1.38-1.51 (16H, m), 1.53-1.70 (16H, m), 2.31 (4H, tt, *J* = 13.46, 5.29 Hz), 2.36-2.65 (24H, br), 4.06 (8H, t, *J* = 6.6 Hz). |
| | **¹³C NMR (101MHz, CDCl₃):** δ 13.98, 14.01, 22.60, 22.64, 26.01, 27.1, 27.2, 27.4, 28.6, 29.2, 29.7, 31.7, 32.2, 32.6, 45.8, 51.6, 53.7, 54.7, 56.8, 64.084, 176.7. |
| 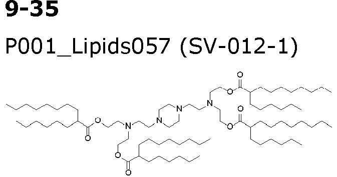 | Step 1 (1.2.4.1.) was followed using **9-14** (350 mg, 1.17 mmol, 7 equiv), 2,2-(Piperazine-1,4-diyl)diethanamine (28.9 mg, 168 µmol, 1 equiv) as starting materials, and NaBH₃CN (68.5 mg, 1.09 mmol, 6.5 equiv) as reducing agent, with 0.24 vol% AcOH (95.9 µL, 10 equiv) in MeOH (40 mL). The product was purified by normal phase flash chromatography to afford ((piperazine-1,4-diylbis(ethane-2,1-diyl))bis(azanetriyl))tetrakis(ethane-2,1-diyl) tetrakis(2-hexyldecanoate) (194 mg, 149 µmol, yield 89%) as a transparent oil. MS (ESI) m/z 651.8 [M+2H]²⁺ |
| | **¹H NMR (400MHz, CDCl₃):** δ 0.88 (24H, distortet t, J = 6.86 Hz), 1.18-1.35 (80H, m), 1.37-1.51 (8H, m), 1.51-1.66 (8H, m), 2.30 (4H, tt, J = 12.95, 5.47 Hz), 2.83 (8H, br s), 2.43-3.48 (16H, m), 4.15 (8H, t, J = 5.59 Hz). |
| | **¹³C NMR (176MHz, CDCl₃):** δ 14.0, 14.1, 22.57, 22.63, 27.4, 27.5, 29.2, 29.3, 29.4, 29.6, 31.7, 31.8, 32.3, 45.7, 53.2, 61.5, 176.5 |
| | Remark: Not all of the carbons of the 2,2-(Piperazine-1,4-diyl)diethanamino core are detected. |
| 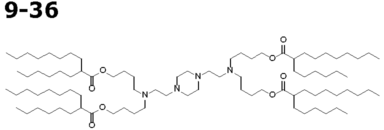 | Step 1 (1.2.4.1.) was followed using **9.15** (497 mg, 1.52 mmol, 4.6 equiv), 2,2-(Piperazine-1,4-diyl)diethanamine (57.0 mg, 331 µmol, 1 equiv) as starting materials, and NaBH₃CN (187 mg, 2.98 mmol, 9 equiv) as reducing agent, with 0.17 vol% AcOH (473 µL, 8.27 mmol, 25 equiv) in MeOH (276 mL). The product was purified by normal phase flash chromatography to afford ((piperazine-1,4-diylbis(ethane-2,1-diyl))bis(azanetriyl))tetrakis(butane-4,1-diyl) tetrakis(2-hexyldecanoate) (223 mg, 158 µmol, yield 48%) as a transparent oil. MS (ESI) m/z 707.9 [M+2H]²⁺ |
| | **¹H NMR (700MHz, CDCl₃):** δ 0.86 (12H, t, *J* = 10.58 Hz), 0.86 (12H, t, *J* = 10.57 Hz), 1.18-1.32 (80H, m), 1.37-1.51 (16H, m), 1.53-1.65 (16H, m), 2.30 (4H, distortet sept, *J* = 4.75 Hz), 2.35 - 2.65 (24H, m), 4.06 (8H, t, *J* = 6.68 Hz). |
| | **¹³C NMR (176MHz, CDCl₃):** δ 14.09, 14.12, 22.61, 22.68, 23.7, 26.7, 27.44, 27.48, 29.24, 29.27, 29.46, 29.58, 31.7, 31.9, 32.5 (2C), 45.8, 51.5, 53.7, 54.1, 56.7, 64.0, 176.6. |
| 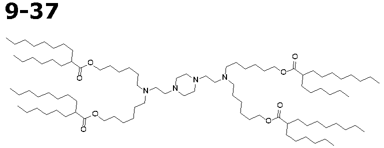 | Step 1 (1.2.4.1.) was followed using **9-16** (500 mg, 1 mmol, 5 equiv) and 2,2-(Piperazine-1,4-diyl)diethanamine (50.7 mg, 294 µmol, 1 equiv) as starting materials, and NaBH₃CN (166 mg, 2.65 mmol, 9 equiv) as reducing agent, with 0.17 vol% AcOH (421 µL, 7.36 mmol, 25 equiv) in MeOH (245 mL). The product was purified by normal phase flash chromatography to afford ((piperazine-1,4-diylbis(ethane-2,1-diyl))bis(azanetriyl))tetrakis(hexane-6,1-diyl) tetrakis(2-hexyldecanoate) (261 mg, 171 µmol, yield 58.1%) as a transparent oil. |
| | MS (ESI) m/z 763.9 [M+2H]²⁺ |
| | **¹H NMR (400MHz, CDCl₃):** δ 0.87 (24H, distorted t, *J* = 6.58 Hz), 1.26 (96H, m), 1.39-1.52 (16H, m), 1.60 (16H, m), 2.31 (4, tt, *J* = 5.29, 8.95 Hz), 2.36-2.86 (24H, br), 4.06 (8H, t, *J* = 6.67 Hz). |
| | **¹³C NMR (176MHz, CDCl₃):** δ 14.09, 14.12, 22.60, 22.68, 26.0, 27.1, 27.4, 27.5, 28.7, 29.2, 29.27, 29.5, 29.6, 31.7, 31.9, 32.5, 45.8, 53.6, 54.5, 64.0, 176.7 |
| | Remark: Not all of the carbons of the 2,2-(Piperazine-1,4-diyl)diethanamino core are detected. |

### 1.3.5. Preparation of Compound 9-40

### 1.3.5.1. Step 1:

| **Sample code** | **Experimental procedure** |
|---|---|
| 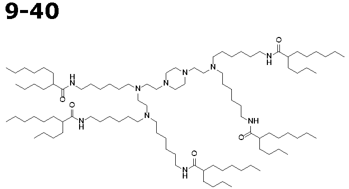 | Step 1 (1.2.5.1.) was followed using **9-21** (425 mg, 1.43 mmol, 5.6 equiv), N1-(2-(4-(2-aminoethyl)piperazin-1-yl)ethyl)ethane-1,2-diamine (55.0 mg, 255 µmol, 1 equiv) as starting material, and NaBH₃CN (160 mg, 2.55 mmol, 10.0 equiv) as reducing agent, with 0.17 vol% AcOH (439 µL, 7.66 mmol, 30 equiv) in MeOH (256 mL). The product was purified by normal phase flash chromatography to afford N,N'-(((2-(4-(2-((2-(bis(6-(2-butyloctanamido)hexyl)amino) ethyl)(6-(2-butyloctanamido)hexyl)amino) ethyl)piperazin-1-yl)ethyl)azanediyl) bis(hexane-6,1-diyl))bis(2-butyloctanamide) (322 mg, 198 µmol, yield 78%) as light yellow gummy liquid. |
| | MS (ESI) m/z 541.7 [M+3H]³⁺ |
| | **¹H NMR (700MHz, CDCl₃):** δ 0.87 (15H, t, *J* = 6.86 Hz), 0.88 (15H, t, *J* = 7.08 Hz), 1.18-1.27 (64H, m), 1.33-1.37 (28H, m), 1.48-1.54 (24H, m), 1.67-1.68 (8H, m), 1.98-2.03 (5H, m), 2.54-2.56 (2H, m), 2.80-2.87 (8H, m), 3.02 (10H, br s), 3.15-3.23 (16H, m), 6.08 (2H, t, *J* = 5.6 Hz), 6.20 (3H, t, *J* = 5.36 Hz). |
| | **¹³C NMR (176MHz, CDCl₃):** δ 14.08, 14.1, 22.6, 22.8, 23.2, 23.6, 25.3, 26.1, 26.2, 26.3, 26.7, 26.8, 27.6, 29.3, 29.34, 29.4, 29.41, 29.5, 29.8, 31.8, 32.8, 32.82, 33.1, 38.9, 39.0, 39.1, 47.8, 47.84, 48.6, 48.9, 49.7, 50.5, 50.9, 51.5, 52.4, 53.3, 53.35, 53.8, 54.3, 176.4, 176.4. |

### Preparation of Compounds 9-41

### 1.3.5.2. Step 1:

| **Sample code** | **Experimental procedure** |
|---|---|
| 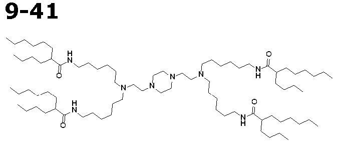 | Step 1 (1.2.6.1.) was followed using **9-21** (0.475 g, 1.60 mmol, 5.0 equiv), 2,2-(Piperazine-1,4-diyl)diethanamine (55.0 mg, 319 µmol, 1 equiv) as starting materials, and NaBH₃CN (181 mg, 2.87 mmol, 9 equiv) as reducing agent, with 0.12 vol% AcOH (457 µL, 7.98 mmol, 25 equiv) in MeOH (364 mL). The product was purified by normal phase flash chromatography to afford N,N',N",N‴-(((piperazine-1,4-diylbis(ethane-2,1-diyl))bis(azanetriyl))tetrakis(hexane-6,1-diyl))tetrakis(2-butyloctanamide) (354 mg, 273 µmol, yield 85%) as light yellow gummy liquid. |
| | MS (ESI) m/z 649.9 [M+2H]²⁺ |
| | **¹H NMR (400MHz, CDCl₃):** δ 0.87 (12H, t, *J* = 6.80 Hz), 0.88 (12H, t, *J* = 7.08 Hz), 1.25-.29 (48H, m), 1.37 - 1.42 (24H, m), 1.52-1.55 (14H, m), 1.76 (8H, br s), 2.05-2.06 (4H, m), 2.92-2.99 (8H, m), 3.15-3.32 (18H, m), 5.72 (8H, br s), 6.38 (4H, br s). |
| | **¹³C NMR (101MHz, CDCl₃):** δ 14.08, 14.1, 22.6, 22.8, 23.6, 26.1, 26.2, 27.6, 29.2, 29.4, 29.8, 31.8, 32.7, 33.0, 39.2, 47.6, 49.4, 51.4, 52.0, 53.8, 176.8. |

### 2. LNP FORMULATION AND TESTING

### 2.1 LNP formulation

Cationic lipids, DOPE, Cholesterol and DMG-PEG lipid were solubilized in ethanol or DMSO at a molar ratio of 35:20:43.5:1.5 and a concentration of 100:25:20:25 µg/µL for C12-200 or 170:25:20:25 µg/µL for the comparator lipids. Lipid nanoparticles (LNP) were prepared by targeting an N/P ratio of approximately 40/1 with saRNA. Briefly, the firefly luciferase saRNA of approximately 9659 nucleotides was diluted to 0.5 mg/mL in 5 mM citrate buffer, pH 4.5. Microfluidic mixer (Ignite from Precision Nanosystems, California, USA) was used to mix the lipid solution with the saRNA aqueous solution at a ratio of about 1:3 (vol/vol) with total flow rates above 10 ml/min. The ethanol was then removed, and the external buffer replaced with 10 mM TRIS-HCL , pH 7.4, by dialysis. Finally, the lipid nanoparticles were filtered through a 0.2 µm pore sterile filter. Lipid nanoparticle particle size was 50-150 nm diameter and polydispersity index (PI) of 0.1-0.4 as determined by Zeta Sizer (Malvern Panalytical, UK). The charge of the formulation was also measured by Zeta Sizer and ranged between 20 to -20 mV.

See Table 3 below for the results obtained. Comparator compounds 1 and 2 have the following structures C1 and C2 respectively:

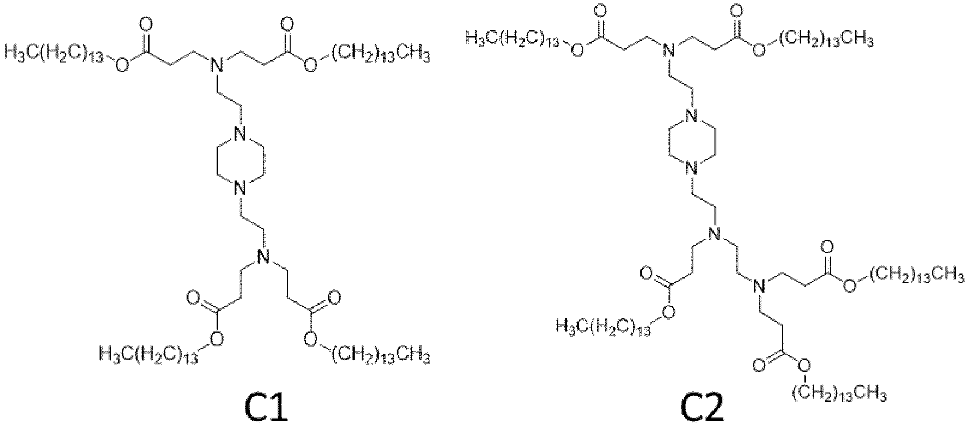

### 2.2 Encapsulation

The saRNA loading in LNP formulations was quantified using a Quant-iT RiboGreen assay (Thermo Fisher Scientific, Waltham, Massachusetts, USA) as previously described. 14 samples were diluted tenfold in 1× Tris HCL - EDTA (TE) buffer (10 mM Tris-HCL, 1mM EDTA, pH 7.5) with or without 2% (v/v) Triton X-100 (Sigma-Aldrich, Saint Louis, Missouri, USA). Standard solutions were also prepared in 1×TE with or without 2% (v/v) Triton X-100 to account for any variation in fluorescence. The assay was performed according to the manufacturer's protocol. Samples were loaded on a black 96-well plate and analysed for fluorescence on a microplate reader (Tecan Infinite^{®} 200 PRO) at an excitation of 485 nm and emission at 528 nm. Encapsulation efficiency was calculated between 80-100%.

See Table 3 below for the results obtained.

### 2.3 In vivo Study

Female SWISS mice (6 weeks old) were purchased from Janvier Laboratories (Paris, France) and kept in individually ventilated cages with access to food and water *ad libitum.* Mice were anesthetized with isoflurane (Zoetis, Louvain-La-Neuve, Belgium) (5% for induction and 2% for maintenance) and intramuscularly injected with a total of 1 µg in 100 µL Tris-HCL (50 µL per leg) LNP-formulated luciferase saRNA (for each group *n*=7). The expression of the luciferase induced bioluminescence was measured via non-invasive *In vivo* bioluminescent imaging (*In vivo* Imaging System (IVIS) Lumina III, Perkin Elmer, Waltham, Massachusetts, USA), 10 minutes after subcutaneous injection of 200 µL of D-luciferin (GoldBio, Saint Louis, Missouri, USA, #LUCK-1G) were measured in day 0 (before injection), day 1, day 3, day 5, day 7, day 10, day 15 and day 20.

See Tables 4 and Figure 1 for the results showing higher expression of the saRNA when administered in LNPs comprising compounds of the invention compared to prior art compounds. Table 5 supports good tolerability of the compounds according to formula I.

**TABLE 3**

| **Lipid** | **Characterisation of LNPs** | | | | | | | |
|---|---|---|---|---|---|---|---|---|
| | **Z-average (nm)** | | **Polydispersity Index (PI)** | | **Encapsulation (%)** | | **Zetapotential (mV)** | |
| | | **± SD (%)** | | **± S_{D} (%)** | | **± SD (%)** | | **± SD (%)** |
| **C12-200** | 94.03 | 1.57 | 0.19 | 0.02 | 97.46 | 6.24 | 9.59 | 0.90 |
| **Comparator 1 (C1)** | 70.96 | 0.7 | 0.16 | 0.02 | 99.03 | 7.77 | 9.07 | 0.46 |
| **Comparator 2 (C2)** | 96.53 | 0.75 | 0.16 | 0.02 | 97.61 | 3.85 | 8.70 | 0.94 |
| **9-26** | 100.07 | 13.76 | 0.28 | 0.05 | 92.78 | 3.60 | -18.67 | 4.30 |
| **9-27** | 65.27 | 1.10 | 0.18 | 0.03 | 93.93 | 2.98 | -10.65 | 1.19 |
| **9-28** | 71.49 | 1.24 | 0.19 | 0.04 | 93.04 | 6.06 | -7.52 | 0.85 |
| **9-29** | 102.48 | 1.35 | 0.15 | 0.02 | 94.31 | 2.04 | -10.51 | 0.88 |
| **9-30** | 115.36 | 1.45 | 0.17 | 0.01 | 94.22 | 5.15 | -8.56 | 1.03 |
| **9-31** | 73.81 | 1.36 | 0.19 | 0.02 | 94.33 | 4.06 | -16.58 | 1.46 |
| **9-32** | 54.53 | 0.48 | 0.17 | 0.03 | 93.33 | 2.16 | -16.38 | 2.86 |
| **9-33** | 86.24 | 1.45 | 0.22 | 0.02 | 92.26 | 2.95 | -13.70 | 1.84 |
| **9-34** | 71.78 | 1.08 | 0.16 | 0.04 | 94.93 | 6.93 | -7.92 | 0.35 |
| **9-35** | 87.91 | 1.06 | 0.19 | 0.01 | 92.96 | 2.89 | -5.54 | 1.46 |
| **9-36** | 105.70 | 0.96 | 0.20 | 0.02 | 96.25 | 3.02 | -10.52 | 1.44 |
| **9-37** | 89.43 | 11.43 | 0.27 | 0.07 | 92.32 | 3.82 | -9.93 | 0.76 |

**TABLE 4**

| **Lipid** | ***Bioluminescence expression of LNPs*** | | | | | | | |
|---|---|---|---|---|---|---|---|---|
| | **Day 0 (Before injection)** | **Day 1** | **Day 3** | **Day 5** | **Day 7** | **Day 10** | **Day 15** | **Day 20** |
| ***Control*** | 1.21E+04 | 8.08E+05 | 6.28E+05 | 2.05E+06 | 8.53E+05 | 6.52E+04 | 3.66E+04 | 4.08E+04 |
| ***C12-200*** | 2.39E+04 | 3.36E+08 | 6.19E+08 | 4.79E+08 | 2.47E+08 | 2.20E+07 | 4.80E+05 | 9.12E+04 |
| ***Comparator 1 (C1)*** | 3.32E+04 | 2.76E+07 | 1.28E+08 | 7.19E+07 | 8.88E+07 | 5.07E+06 | 8.46E+04 | 4.32E+04 |
| ***Comparator 2 (C2)*** | 2.15E+04 | 2.19E+07 | 8.21E+07 | 2.21E+08 | 1.62E+08 | 2.27E+07 | 2.04E+05 | 9.42E+04 |
| ***9-26*** | 5.87E+03 | 8.16E+06 | 2.76E+08 | 6.92E+08 | 6.31E+08 | 2.23E+07 | 4.91E+05 | 7.78E+04 |
| ***9-27*** | 7.41E+03 | 4.09E+07 | 1.51E+09 | 1.35E+09 | 9.33E+08 | 2.88E+06 | 2.77E+05 | 1.87E+05 |
| ***9-28*** | 4.44E+03 | 1.01E+08 | 8.67E+08 | 9.45E+08 | 5.65E+08 | 2.08E+06 | 8.15E+04 | 3.35E+04 |
| ***9-29*** | 1.64E+04 | 4.13E+05 | 1.68E+07 | 4.25E+07 | 6.13E+07 | 3.92E+07 | 1.44E+06 | 5.43E+05 |
| ***9-30*** | 1.29E+04 | 2.31E+06 | 2.87E+07 | 6.18E+07 | 9.09E+07 | 2.84E+07 | 1.67E+05 | 1.67E+05 |
| ***9-31*** | 9.46E+03 | 3.10E+07 | 2.74E+08 | 5.31E+08 | 2.16E+08 | 2.22E+06 | 2.46E+05 | 2.71E+04 |
| ***9-32*** | 1.13E+04 | 5.13E+06 | 1.31E+08 | 2.76E+08 | 3.71E+08 | 1.08E+07 | 6.35E+05 | 1.29E+05 |
| ***9-33*** | 3.16E+04 | 3.79E+07 | 6.90E+08 | 1.45E+09 | 1.02E+09 | 5.76E+06 | 5.96E+05 | 1.21E+05 |
| ***9-34*** | 1.16E+04 | 4.79E+07 | 7.26E+08 | 7.42E+08 | 9.12E+08 | 4.70E+07 | 1.43E+05 | 3.37E+04 |
| ***9-35*** | 2.11E+04 | 8.83E+05 | 9.99E+07 | 5.92E+07 | 6.45E+07 | 5.62E+07 | 6.82E+05 | 2.44E+05 |
| ***9-36*** | 1.02E+04 | 4.28E+06 | 1.92E+08 | 8.44E+08 | 6.10E+08 | 5.56E+06 | 2.57E+05 | 1.16E+05 |
| ***9-37*** | 1.00E+04 | 1.07E+07 | 2.06E+08 | 4.17E+08 | 3.54E+08 | 2.11E+06 | 7.50E+04 | 2.65E+04 |
| ***9-40*** | 1.21E+04 | 6.07E+07 | 6.79E+08 | 1.14E+09 | 8.88E+08 | 2.00E+07 | 1.32E+06 | 2.64E+05 |

**TABLE 5 - weight evolution of the animals following administration of LNPs comprising compounds according to formula I**

| **Lipid** | ***Weight (g)*** | | | | | | | | | | | | | | | |
|---|---|---|---|---|---|---|---|---|---|---|---|---|---|---|---|---|
| | **Day 0** | | **Day 1** | | **Day 3** | | **Day 5** | | **Day 7** | | **Day 10** | | **Day 15** | | **Day 20** | |
| | | **± SD (%)** | | **± SD (%)** | | **± SD (%)** | | **± SD (%)** | | **± SD (%)** | | **± SD (%)** | | **± SD (%)** | | **± SD (%)** |
| *Control* | 28.89 | 2.63 | 29.07 | 3.01 | 29.63 | 2.19 | 29.89 | 2.69 | 30.41 | 2.91 | 30.09 | 2.74 | 31.45 | 3.42 | 32.56 | 3.05 |
| *C12-200* | 28.61 | 2.25 | 27.17 | 2.15 | 28.62 | 1.94 | 28.98 | 1.57 | 28.97 | 1.98 | 29.23 | 2.55 | 30.16 | 2.40 | 31.23 | 2.88 |
| ***Comparator 1 (C1)*** | 26.56 | 1.14 | 25.99 | 1.13 | 27.21 | 1.45 | 27.59 | 1.61 | 27.09 | 1.69 | 27.17 | 1.21 | 29.43 | 1.60 | 30.11 | 1.82 |
| ***Comparator 2 (C2)*** | 27.80 | 0.86 | 27.00 | 1.25 | 27.70 | 1.24 | 27.44 | 1.29 | 27.84 | 1.31 | 27.31 | 1.57 | 29.54 | 1.48 | 29.79 | 1.84 |
| ***9-26*** | 27.91 | 1.66 | 27.31 | 1.24 | 28.51 | 1.15 | 28.46 | 1.15 | 28.44 | 1.64 | 29.13 | 1.91 | 30.50 | 1.94 | 32.44 | 2.43 |
| ***9-27*** | 27.51 | 1.30 | 26.21 | 1.24 | 28.67 | 1.61 | 28.56 | 1.62 | 29.01 | 1.13 | 29.61 | 1.50 | 30.77 | 1.64 | 31.87 | 2.38 |
| ***9-28*** | 28.36 | 1.85 | 27.19 | 1.72 | 29.44 | 1.56 | 29.17 | 1.71 | 29.37 | 1.44 | 29.39 | 1.98 | 31.43 | 2.60 | 32.46 | 1.96 |
| ***9-29*** | 29.59 | 2.10 | 29.53 | 2.08 | 29.74 | 2.22 | 30.04 | 2.41 | 30.93 | 2.30 | 30.63 | 2.74 | 30.89 | 2.65 | 32.69 | 2.91 |
| ***9-30*** | 29.39 | 1.40 | 28.23 | 1.63 | 29.73 | 1.39 | 29.77 | 1.64 | 30.46 | 1.80 | 31.20 | 2.04 | 30.96 | 1.77 | 32.00 | 2.59 |
| ***9-31*** | 27.70 | 1.77 | 26.17 | 2.09 | 28.46 | 1.37 | 27.66 | 1.07 | 28.29 | 0.83 | 28.27 | 0.76 | 29.76 | 1.34 | 30.90 | 1.06 |
| ***9-32*** | 27.00 | 1.56 | 26.94 | 1.52 | 28.46 | 1.47 | 28.26 | 1.26 | 28.11 | 1.33 | 27.91 | 2.22 | 29.69 | 2.45 | 30.94 | 2.13 |
| ***9-33*** | 26.83 | 1.88 | 25.36 | 1.15 | 27.37 | 1.87 | 26.96 | 1.62 | 27.29 | 1.71 | 27.20 | 2.05 | 28.79 | 2.36 | 29.60 | 2.06 |
| ***9-34*** | 27.77 | 1.55 | 26.14 | 1.73 | 28.33 | 1.62 | 28.51 | 1.54 | 28.49 | 1.90 | 28.97 | 1.69 | 30.24 | 2.22 | 31.51 | 3.58 |
| ***9-35*** | 30.01 | 1.70 | 30.06 | 1.82 | 30.43 | 1.40 | 29.87 | 1.60 | 30.30 | 2.29 | 31.23 | 2.52 | 32.13 | 2.30 | 32.23 | 2.73 |
| ***9-36*** | 29.09 | 2.37 | 29.49 | 1.62 | 31.49 | 3.40 | 30.09 | 2.60 | 30.36 | 1.83 | 30.56 | 2.52 | 31.33 | 2.82 | 32.30 | 2.55 |
| ***9-37*** | 28.21 | 2.25 | 26.73 | 1.66 | 28.81 | 1.66 | 28.84 | 1.93 | 28.84 | 1.41 | 29.36 | 1.92 | 30.63 | 2.78 | 31.61 | 2.36 |
| ***9-40*** | 30.11 | 2.62 | 29.54 | 3.23 | 29.41 | 4.29 | 29.19 | 3.22 | 30.31 | 3.41 | 30.51 | 3.87 | 31.09 | 3.76 | 32.37 | 4.20 |
| ***9-41*** | 29.19 | 1.22 | 29.19 | 1.27 | 29.43 | 1.02 | 29.21 | 1.65 | 30.44 | 1.97 | 31.06 | 1.81 | 31.26 | 2.02 | 31.77 | 1.70 |

### Example 3. Formulation of LNPs according to an embodiment of the current invention

Several LNPs are obtained comprising at least one of the compounds as shown in Table 1 an saRNA. These LNPs were tested and were shown to efficiently encapsulate the RNA and deliver the RNA *in vivo.*

Examples of LNPs are:

| **Compound** | **LNP A (concentrati on %)** | **LNP B (concentrati on %)** | **LNP C (concentrati on %)** | **LNP D (concentrati on %)** |
|---|---|---|---|---|
| **C12-200** | 0 | 17.5 | 0 | 0 |
| **Dlin-KC2-DMA** | 0 | 0 | 0 | 17.5 |
| **DOPE** | 20 | 15 | 0 | 15 |
| **DMG-PEG2000** | 1.5 | 1.5 | 1.5 | 1.5 |
| **Cholesterol** | 43.5 | 48.5 | 50 | 48.5 |
| **Compound according to Formula I** | 35 | 17.5 | 48.5 | 17.5 |

## Claims

1. A compound according to Formula (I) or pharmaceutically acceptable salt, tautomer, or stereoisomer thereof, 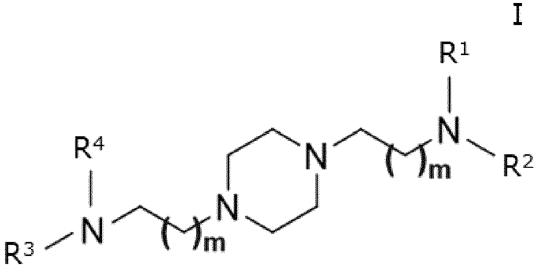 wherein:
each m is, independently, 1, 2, 3, 4 or 5; and
R¹ is -L¹N[L²F¹R^{A}]₂ or -L³F²R^{B} and R², R³ and R⁴ are each -L⁴F³R^{C},
wherein:
each L¹, L², L³ and L⁴ are, independently, C2-C10 alkylene, each F¹, F² and F³ are, independently, ester or amide functional group and each R^{A}, R^{B} and R^{C} are, independently, branched C4-C30 alkyl.

2. A compound according to claim 1, wherein each R^{A}, R^{B} and R^{C} are, independently, selected from undecan-5-yl, tridecan-6-yl, pentadecan-7-yl, heptadecan-8-yl, and nonadecan-9-yl.

3. A compound according to claim 1 or 2, wherein each L², L³ and L⁴ are, independently, selected from C4-C10 alkylene, or, from C4-C8 alkylene, or, from C4, C5 or C6 alkylene, or, each L², L³ and L⁴ are butylene or hexylene.

4. A compound according to any of the previous claims wherein -L²F¹R^{A}, -L³F²R^{B} and -L⁴F³R^{C}, are, independently, ester groups.

5. A compound according to any of the previous claims wherein F¹, F² and F³ are ester groups positioned according to -L-O-C(=O)-R, wherein -L-represents any one of L², L³ or L⁴, and, -R represents any one of R^{A}, R^{B} and R^{C}

6. A compound according to any of the previous claims, wherein the compound is -((2-(4-(2-((2-(bis(2-((2- hexyldecanoyl)oxy)ethyl)amino)ethyl)(2-((2-hexyldecanoyl)oxy)ethyl)amino)ethyl)piperazin-1-yl)ethyl)azanediyl)bis(ethane-2,1-diyl)bis(2-hexyldecanoate)
- ((piperazine-1,4-diylbis(ethane-2,1- diyl))bis(azanetriyl))tetrakis(ethane-2,1-diyl)tetrakis(2-hexyldecanoate)
- ((piperazine-1,4-diylbis(ethane-2,1- diyl))bis(azanetriyl))tetrakis(butane-4,1-diyl)tetrakis(2-octyldodecanoate)
- ((piperazine-1,4-diylbis(ethane-2,1- diyl))bis(azanetriyl))tetrakis(ethane-2,1-diyl)tetrakis(2-butyloctanoate)
- ((piperazine-1,4-diylbis(ethane-2,1- diyl))bis(azanetriyl))tetrakis(hexane-6,1-diyl)tetrakis(2-hexyldecanoate)
- ((2-(4-(2-((2-(bis(2-((2- butyloctanoyl)oxy)ethyl)amino)ethyl)(2-((2-butyloctanoyl)oxy)ethyl)amino)ethyl)piperazin-1-yl)ethyl)azanediyl)bis(ethane-2,1-diyl)bis(2-butyloctanoate)
- ((2-(4-(2-((2-(bis(4-((2- butyloctanoyl)oxy)butyl)amino)ethyl)(4-((2-butyloctanoyl)oxy)butyl)amino)ethyl)piperazin-1-yl)ethyl)azanediyl)bis(butane-4,1-diyl)bis(2-butyloctanoate)
- ((2-(4-(2-((2-(bis(6-((2- butyloctanoyl)oxy)hexyl)amino)ethyl)(6-((2-butyloctanoyl)oxy)hexyl)amino)ethyl)piperazin-1-yl)ethyl)azanediyl)bis(hexane-6,1-diyl)bis(2-butyloctanoate)
- ((2-(4-(2-((2-(bis(4-((2- hexyldecanoyl)oxy)butyl)amino)ethyl)(4-((2-hexyldecanoyl)oxy)butyl)amino)ethyl)piperazin-1-yl)ethyl)azanediyl)bis(butane-4,1-diyl)bis(2-hexyldecanoate)
- ((2-(4-(2-((2-(bis(6-((2- hexyldecanoyl)oxy)hexyl)amino)ethyl)(6-((2-hexyldecanoyl)oxy)hexyl)amino)ethyl)piperazin-1-yl)ethyl)azanediyl)bis(hexane-6,1-diyl)bis(2-hexyldecanoate)
- ((2-(4-(2-((2-(bis(2-((2- octyldodecanoyl)oxy)ethyl)amino)ethyl)(2-((2-octyldodecanoyl)oxy)ethyl)amino)ethyl)piperazin-1-yl)ethyl)azanediyl)bis(ethane-2,1-diyl) bis(2-octyldodecanoate)
- ((2-(4-(2-((2-(bis(4-((2- octyldodecanoyl)oxy)butyl)amino)ethyl)(4-((2-octyldodecanoyl)oxy)butyl)amino)ethyl)piperazin-1-yl)ethyl)azanediyl)bis(butane-4,1-diyl) bis(2-octyldodecanoate)
- ((2-(4-(2-((2-(bis(6-((2- octyldodecanoyl)oxy)hexyl)amino)ethyl)(6-((2-octyldodecanoyl)oxy)hexyl)amino)ethyl)piperazin-1-yl)ethyl)azanediyl)bis(hexane-6,1-diyl) bis(2-octyldodecanoate)
- ((piperazine-1,4-diylbis(ethane-2,1- diyl))bis(azanetriyl))tetrakis(butane-4,1-diyl)tetrakis(2-butyloctanoate)
- ((piperazine-1,4-diylbis(ethane-2,1- diyl))bis(azanetriyl))tetrakis(hexane-6,1-diyl)tetrakis(2-butyloctanoate)
- ((piperazine-1,4-diylbis(ethane-2,1- diyl))bis(azanetriyl))tetrakis(butane-4,1-diyl)tetrakis(2-hexyldecanoate)
- ((piperazine-1,4-diylbis(ethane-2,1- diyl))bis(azanetriyl))tetrakis(ethane-2,1-diyl)tetrakis(2-octyldodecanoate)
- ((piperazine-1,4-diylbis(ethane-2,1- diyl))bis(azanetriyl))tetrakis(hexane-6,1-diyl)tetrakis(2-octyldodecanoate)
- di(nonadecan-9-yl)3,3'-((2-(4-(2-((2-(bis(3-(nonadecan-9-yloxy)-3-oxopropyl)amino)ethyl)(3-(nonadecan-9-yloxy)-3-oxopropyl)amino)ethyl)piperazin-1-yl)ethyl)azanediyl)dipropionate
- di(nonadecan-9-yl)7,7'-((2-(4-(2-((2-((3-(nonadecan-9-yloxy)-3-oxopropyl) (7-(nonadecan-9-yloxy)-7-oxoheptyl)amino)ethyl)(7-(nonadecan-9-yloxy)-7-oxoheptyl)amino)ethyl)piperazin-1- yl)ethyl)azanediyl)diheptanoate

7. A lipid nanoparticle (LNP) comprising at least one compound according to any of the preceding claims and one or more oligonucleotides.

8. Lipid nanoparticle according to claim 6, wherein said oligonucleotide is RNA or DNA having a length of at least 5000 nt.

9. Lipid nanoparticle according to any of the previous claims 7 or 8, wherein said lipid nanoparticle further comprises:
- at least a PEG or a PEG conjugate,
- at least a sterol, and
- at least a phospholipid and/or at least a second ionizable lipid.

10. Lipid nanoparticle according to any of claims 7 to 9, wherein said compound according to Formula (I) or pharmaceutically acceptable salt, tautomer, or stereoisomer thereof is present in said LNP in a concentration of 12.5-60 mol%, and/or said phospholipid is present in a concentration of 0.5-35 mol% in said LNP, and/or said sterol is present in a concentration of 30-50 mol% in said LNP and/or said PEG conjugate is present in said LNP in a concentration of 0.5-5 mol%.

11. Lipid nanoparticle according to any of the previous claims 7 to 10, wherein said lipid nanoparticle comprises at least one second ionizable lipid, wherein the overall concentration of said ionizable lipids or lipid-like compounds is between 12.5 and 60 mol%.

12. A pharmaceutical composition or vaccine comprising one or more lipid nanoparticles as defined in any of the previous claims 7 to 11.

13. Pharmaceutical composition or vaccine according to claim 12 for use in the treatment and/or prophylaxis of a disease.

14. The compound according to any of claims 1 to 6 for use in the preparation of a pharmaceutical composition for therapeutic use comprising an RNA encoding a gene of interest.

## Patentansprüche

1. Verbindung gemäß der Formel (I) oder pharmazeutisch unbedenkliches Salz, Tautomer oder Stereoisomer davon, 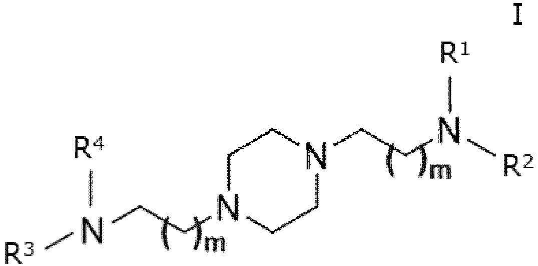 wobei:
m jeweils unabhängig 1, 2, 3, 4 oder 5 ist und
R¹ -L¹N[L²F¹R^{A}]₂ oder -L³F²R^{B} ist und R², R³ und R⁴ jeweils -L⁴F³R^{C}, sind, wobei:
L¹, L², L³ und L⁴ jeweils unabhängig C2-C10-Alkylen sind, F¹, F² und F³ jeweils unabhängig eine funktionale Ester- oder Amidgruppe sind und R^{A}, R^{B} und R^{C} jeweils unabhängig verzweigtes C4-C30-Alkyl sind.

2. Verbindung nach Anspruch 1, wobei R^{A}, R^{B} und R^{C} jeweils unabhängig aus Undecan-5-yl, Tridecan-6-yl, Pentadecan-7-yl, Heptadecan-8-yl und Nonadecan-9-yl ausgewählt sind.

3. Verbindung nach Anspruch 1 oder 2, wobei L², L³ und L⁴ jeweils unabhängig aus C4-C10-Alkylen oder aus C4-C8-Alkylen oder aus C4, C5 oder C6-Alkylen ausgewählt sind oder L², L³ und L⁴ jeweils Butylen oder Hexylen sind.

4. Verbindung nach einem der vorhergehenden Ansprüche, wobei -L²F¹R^{A}, -L³F²R^{B} und -L⁴F³R^{C}, unabhängig Estergruppen sind.

5. Verbindung nach einem der vorhergehenden Ansprüche, wobei F¹, F² und F³ Estergruppen sind, die gemäß -L-O-C(=O)-R positioniert sind, wobei -L- ein beliebiges von L², L³ und L⁴ darstellt und -R ein beliebiges von R^{A}, R^{B} und R^{C} darstellt.

6. Verbindung nach einem der vorhergehenden Ansprüche, wobei die Verbindung Folgendes ist:
- ((2-(4-(2-((2-(bis(2-((2-Hexyldecanoyl)oxy)ethyl)amino)ethyl)(2-((2-hexyldecanoyl)oxy)ethyl)amino)ethyl)piperazin-1-yl)ethyl)azandiyl)bis (ethan-2,1-diyl)bis(2-hexyldecanoat)
- ((Piperazin-1,4-diylbis(ethan-2,1-diyl))bis(azantriyl))tetrakis(ethan-2,1-diyl)tetrakis(2-hexyldecanoat)
- ((Piperazin-1,4-diylbis(ethan-2,1-diyl))bis(azantriyl))tetrakis(butan-4,1-diyl)tetrakis(2-octyldodecanoat)
- ((Piperazin-1,4-diylbis(ethan-2,1-diyl))bis(azantriyl))tetrakis(ethan-2,1-diyl)tetrakis(2-butyloctanoat)
- ((Piperazin-1,4-diylbis(ethan-2,1-diyl))bis(azantriyl))tetrakis(hexan-6,1-diyl)tetrakis(2-hexyldecanoat)
- ((2-(4-(2-((2-(bis(2-((2-Butyloctanoyl)oxy)ethyl)amino)ethyl)(2-((2-butyloctanoyl)oxy)ethyl)amino)ethyl)piperazin-1-yl)ethyl)azandiyl)bis (ethan-2,1-diyl)bis(2-butyloctanoat)
- ((2-(4-(2-((2-(bis(4-((2-Butyloctanoyl)oxy)butyl)amino)ethyl)(4-((2-butyloctanoyl)oxy)butyl)amino)ethyl)piperazin-1-yl)ethyl)azandiyl)bis (butan-4,1-diyl)bis(2-butyloctanoat)
- ((2-(4-(2-((2-(bis(6-((2-Butyloctanoyl)oxy)hexyl)amino)ethyl)(6-((2-butyloctanoyl)oxy)hexyl)amino)ethyl)piperazin-1-yl)ethyl)azandiyl) bis(hexan-6,1-diyl)bis(2-butyloctanoat)
- ((2-(4-(2-((2-(bis(4-((2-Hexyldecanoyl)oxy)butyl)amino)ethyl)(4-((2-hexyldecanoyl)oxy)butyl)amino)ethyl)piperazin-1-yl)ethyl)azandiyl)bis (butan-4,1-diyl)bis(2-hexyldecanoat)
- ((2-(4-(2-((2-(bis(6-((2-Hexyldecanoyl)oxy)hexyl)amino)ethyl)(6-((2-hexyldecanoyl)oxy)hexyl)amino)ethyl)piperazin-1-yl)ethyl)azandiyl)bis (hexan-6,1-diyl)bis(2-hexyldecanoat)
- ((2-(4-(2-((2-(bis(2-((2-Octyldodecanoyl)oxy)ethyl)amino)ethyl)(2-((2-octyldodecanoyl)oxy)ethyl)amino)ethyl)piperazin-1-yl)ethyl)azandiyl)bis(ethan-2,1-diyl)bis(2-octyldodecanoat)
- ((2-(4-(2-((2-(bis(4-((2-Octyldodecanoyl)oxy)butyl)amino)ethyl)(4-((2-octyldodecanoyl)oxy)butyl)amino)ethyl)piperazin-1-yl)ethyl)azandiyl)bis(butan-4,1-diyl)bis(2-octyldodecanoat)
- ((2-(4-(2-((2-(bis(6-((2-Octyldodecanoyl)oxy)hexyl)amino)ethyl)(6-((2-octyldodecanoyl)oxy)hexyl)amino)ethyl)piperazin-1-yl)ethyl)azandiyl)bis(hexan-6,1-diyl)bis(2-octyldodecanoat)
- ((Piperazin-1,4-diylbis(ethan-2,1-diyl))bis(azantriyl))tetrakis(butan-4,1-diyl)tetrakis(2-butyloctanoat)
- ((Piperazin-1,4-diylbis(ethan-2,1-diyl))bis(azantriyl))tetrakis(hexan-6,1-diyl)tetrakis(2-butyloctanoat)
- ((Piperazin-1,4-diylbis(ethan-2,1-diyl))bis(azantriyl))tetrakis(butan-4,1-diyl)tetrakis(2-hexyldecanoat)
- ((Piperazin-1,4-diylbis(ethan-2,1-diyl))bis(azantriyl))tetrakis(ethan-2,1-diyl)tetrakis(2-octyldodecanoat)
- ((Piperazin-1,4-diylbis(ethan-2,1-diyl))bis(azantriyl))tetrakis(hexan-6,1-diyl)tetrakis(2-octyldodecanoat)
- Di(nonadecan-9-yl)3,3'-((2-(4-(2-((2-(bis(3-(nonadecan-9-yloxy)-3-oxopropyl)amino)ethyl)(3-(nonadecan-9-yloxy)-3-oxopropyl)amino)ethyl)piperazin-1-yl)ethyl)azandiyl)dipropionat
- Di(nonadecan-9-yl)7,7'-((2-(4-(2-((2-((3-(nonadecan-9-yloxy)-3-oxopropyl)(7-(nonadecan-9-yloxy)-7-oxoheptyl)amino)ethyl)(7-(nonadecan-9-yloxy)-7-oxoheptyl)amino)ethyl)piperazin-1-yl)ethyl)azandiyl)diheptanoat.

7. Lipidnanopartikel (LNP), mindestens eine Verbindung nach einem der vorhergehenden Ansprüche und ein oder mehrere Oligonukleotide umfassend.

8. Lipidnanopartikel nach Anspruch 6, wobei das Oligonukleotid RNA oder DNA mit einer Länge von mindestens 5000 nt ist.

9. Lipidnanopartikel nach einem der vorhergehenden Ansprüche 7 oder 8, wobei der Lipidnanopartikel ferner Folgendes umfasst:
- mindestens ein PEG oder ein PEG-Konjugat,
- mindestens ein Sterin und
- mindestens ein Phospholipid und/oder mindestens ein zweites ionisierbares Lipid.

10. Lipidnanopartikel nach einem der Ansprüche 7 bis 9, wobei die Verbindung gemäß der Formel (I) oder das pharmazeutisch unbedenkliche Salz, Tautomer oder Stereoisomer davon in dem LNP in einer Konzentration von 12,5 bis 60 Mol-% vorhanden ist und/oder das Phospholipid in dem LNP in einer Konzentration von 0,5 bis 35 Mol-% vorhanden ist und/oder das Sterin in dem LNP in einer Konzentration von 30 bis 50 Mol-% vorhanden ist und/oder das PEG-Konjugat in dem LNP in einer Konzentration von 0,5 bis 5 Mol-% vorhanden ist.

11. Lipidnanopartikel nach einem der vorhergehenden Ansprüche 7 bis 10, wobei der Lipidnanopartikel mindestens ein zweites ionisierbares Lipid umfasst, wobei die Gesamtkonzentration der ionisierbaren Lipide oder der lipidähnlichen Verbindungen zwischen 12,5 und 60 Mol-% beträgt.

12. Pharmazeutische Zusammensetzung oder Impfstoff, einen oder mehrere Lipidnanopartikel nach einem der vorhergehenden Ansprüche 7 bis 11 umfassend.

13. Pharmazeutische Zusammensetzung oder Impfstoff nach Anspruch 12 zur Verwendung bei der Behandlung und/oder Prophylaxe einer Erkrankung.

14. Verbindung nach einem der Ansprüche 1 bis 6 zur Verwendung bei der Herstellung einer pharmazeutischen Verbindung zur therapeutischen Verwendung, die eine RNA umfasst, die für ein Gen von Interesse codiert.

## Revendications

1. Composé selon la Formule (I) ou sel, tautomère ou stéréo-isomère pharmaceutiquement acceptable de celui-ci, 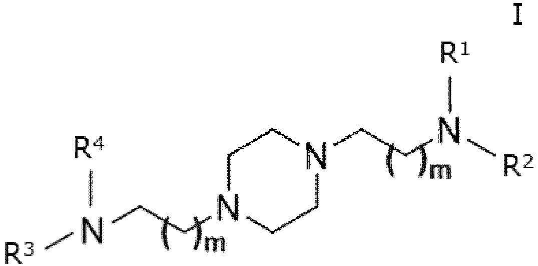 dans lequel :
chaque m est, indépendamment, 1, 2, 3, 4 ou 5 ; et
R¹ est -L¹N[L²F¹R^{A}]₂ ou -L³F²R^{B} et R², R³ et R⁴ sont chacun -L⁴F³R^{C},
dans lequel :
chaque L¹, L², L³ et L⁴ sont, indépendamment, alkylène en C2-C10, chaque F¹, F² et F³ sont, indépendamment, un groupe fonctionnel ester ou amide et chaque R^{A}, R^{B} et R^{C} sont, indépendamment, alkyle ramifié en C4-C30.

2. Composé selon la revendication 1, dans lequel chaque R^{A}, R^{B} et R^{C} sont, indépendamment, choisis parmi undécan-5-yl, tridécan-6-yl, pentadécan-7-yl, heptadécan-8-yl, et nonadécan-9-yl.

3. Composé selon la revendication 1 ou 2, dans lequel chaque L², L³ et L⁴ sont, indépendamment, choisis parmi alkylène en C4-C10, ou alkylène en C4-C8, ou alkylène en C4, C5 ou C6, ou chaque L², L³ et L⁴ sont butylène ou hexylène.

4. Composé selon l'une quelconque des revendications précédentes, dans lequel -L²F¹R^{A}, -L³F²R^{B} et -L⁴F³R^{C} sont, indépendamment, des groupes ester.

5. Composé selon l'une quelconque des revendications précédentes, dans lequel F¹, F² et F³ sont des groupes ester positionnés selon -L-O-C(=O)-R, où -L-représente l'un quelconque parmi L², L³ ou L⁴, et, -R représente l'un quelconque parmi R^{A}, R^{B} et R^{C}.

6. Composé selon l'une quelconque des revendications précédentes, dans lequel le composé est
- ((2-(4-(2-((bis(2-((2-hexyldécanoyl)oxy)éthyl)amino)éthyl)(2-((2-hexyldécanoyl)oxy)éthyl)amino)éthyl)pipérazin-1-yl)éthyl)azanediyl)bis (éthane-2,1-diyl) bis(2-hexyldécanoate))
- ((pipérazine-1,4-diylbis(éthane-2,1-diyl))bis(azanetriyl))tétrakis(éthane -2,1-diyl)tétrakis(2-hexyldécanoate)
- ((pipérazine-1,4-diylbis(éthane-2,1-diyl))bis(azanetriyl))tétrakis(butane -4,1-diyl)tétrakis(2-octyldodécanoate)
- ((pipérazine-1,4-diylbis(éthane-2,1-diyl))bis(azanetriyl))tétrakis(éthane -2,1-diyl)tétrakis(2-butyloctanoate))
- ((pipérazine-1,4-diylbis(éthane-2,1-diyl))bis(azanetriyl))tétrakis(hexane-6,1-diyl)tétrakis(2-hexyldécanoate)
- ((2-(4-(2-((bis(2-((2-butyloctanoyl)oxy)éthyl)amino)éthyl)(2-((2-butyloctanoyl)oxy)éthyl)amino)éthyl)pipérazin-1-yl)éthyl)azanediyl)bis (éthane-2,1-diyl) bis(2-butyloctanoate))
- ((2-(4-(2-((bis(4-((2-butyloctanoyl)oxy)butyl)amino)éthyl)(4-((2-butyloctanoyl)oxy)butyl)amino)éthyl)pipérazin-1-yl)éthyl)azanediyl)bis (butane-4,1-diyl) bis(2-butyloctanoate))
- ((2-(4-(2-((bis(6-((2-butyloctanoyl)oxy)hexyl)amino)éthyl)(6-((2-butyloctanoyl)oxy)hexyl)amino)éthyl)pipérazin-1-yl)éthyl)azanediyl) bis(hexane-6,1-diyl) bis(2-butyloctanoate))
- ((2-(4-(2-((bis(4-((2-hexyldécanoyl)oxy)butyl)amino)éthyl)(4-((2-hexyldécanoyl)oxy)butyl)amino)éthyl)pipérazin-1-yl)éthyl)azanediyl)bis (butane-4,1-diyl) bis(2-hexyldécanoate))
- ((2-(4-(2-((bis(6-((2-hexyldécanoyl)oxy)hexyl)amino)éthyl)(6-((2-hexyldécanoyl)oxy)hexyl)amino)éthyl)pipérazin-1-yl)éthyl)azanediyl)bis (hexane-6,1-diyl) bis(2-hexyldécanoate))
- ((2-(4-(2-((bis(2-((2-octyldodécanoyl)oxy)éthyl)amino)éthyl)(2-((2-octyldodécanoyl)oxy)éthyl)amino)éthyl)pipérazin-1-yl)éthyl)azanediyl) bis(éthane-2,1-diyl) bis(2-octyldodécanoate))
- ((2-(4-(2-((bis(4-((2-octyldodécanoyl)oxy)butyl)amino)éthyl)(4-((2-octyldodécanoyl)oxy)butyl)amino)éthyl)pipérazin-1-yl)éthyl)azanediyl) bis(butane-4,1-diyl) bis(2-octyldodécanoate)))
- ((2-(4-(2-((bis(6-((2-octyldodécanoyl)oxy)hexyl)amino)éthyl)(6-((2-octyldodécanoyl)oxy)hexyl)amino)éthyl)pipérazin-1-yl)éthyl)azanediyl) bis(hexane-6,1-diyl) bis(2-octyldodécanoate))
- ((pipérazine-1,4-diylbis(éthane-2,1-diyl))bis(azanetriyl))tétrakis(butane -4,1-diyl) tétrakis(2-butyloctanoate)
- ((pipérazine-1,4-diylbis(éthane-2,1-diyl))bis(azanetriyl))tétrakis(hexane-6,1-diyl)tétrakis(2-butyloctanoate)
- ((pipérazine-1,4-diylbis(éthane-2,1-diyl))bis(azanetriyl))tétrakis(butane -4,1-diyl)tétrakis(2-hexyldécanoate))
- ((pipérazine-1,4-diylbis(éthane-2,1-diyl))bis(azanetriyl))tétrakis(éthane -2,1-diyl)tétrakis(2-octyldodécanoate))
- ((pipérazine-1,4-diylbis(éthane-2,1-diyl))bis(azanetriyl))tétrakis(hexane -6,1-diyl)tétrakis(2-octyldodécanoate)
- di(nonadécan-9-yl)3,3'-((2-(4-(2-(bis(3-(nonadécan-9-yloxy)-3-oxopropyl)amino)éthyl)(3-(nonadécan-9-yloxy)-3-oxopropyl)amino) éthyl)pipérazin-1-yl)éthyl)azanediyl)dipropionate
- di(nonadécan-9-yl)7,7'-((2-(4-(2-((3-(nonadécan-9-yloxy)-3-oxopropyl)(7-(nonadécan-9-yloxy)-7-oxoheptyl)amino)éthyl)(7-(nonadécan-9-yloxy)-7-oxoheptyl)amino)éthyl)pipérazin-1-yl)éthyl) azanediyl)diheptanoate.

7. Nanoparticule lipidique (LNP) comprenant au moins un composé selon l'une quelconque des revendications précédentes et un ou plusieurs oligonucléotides.

8. Nanoparticule lipidique selon la revendication 6, dans laquelle ledit oligonucléotide est un ARN ou un ADN d'une longueur d'au moins 5000 nt.

9. Nanoparticule lipidique selon l'une des quelconque des revendications précédentes 7 ou 8, dans laquelle ladite nanoparticule lipidique comprend en outre :
- au moins un PEG ou un conjugué de PEG,
- au moins un stérol, et
- au moins un phospholipide et/ou au moins un second lipide ionisable.

10. Nanoparticule lipidique selon l'une quelconque des revendications 7 à 9, dans laquelle ledit composé selon la Formule (I) ou un sel, un tautomère, ou un stéréo-isomère pharmaceutiquement acceptable de celui-ci est présent dans ladite LNP à une concentration de 12,5 à 60 % mol, et/ou ledit phospholipide est présent à une concentration de 0,5 à 35 % mol dans ladite LNP, et/ou ledit stérol est présent à une concentration de 30 à 50 % mol dans ladite LNP et/ou ledit conjugué PEG est présent dans ladite LNP à une concentration de 0,5 à 5 % mol.

11. Nanoparticule lipidique selon l'une quelconque des revendications précédentes 7 à 10, dans laquelle ladite nanoparticule lipidique comprend au moins un second lipide ionisable, dans laquelle la concentration globale desdits lipides ionisables ou composés de type lipide est comprise entre 12,5 et 60 % mol.

12. Composition pharmaceutique ou vaccin comprenant une ou plusieurs nanoparticules lipidiques telles que définies dans l'une quelconque des revendications précédentes 7 à 11.

13. Composition pharmaceutique ou vaccin selon la revendication 12, destinés à être utilisés dans le traitement et/ou la prophylaxie d'une maladie.

14. Composé selon l'une quelconque des revendications 1 à 6, destiné à être utilisé dans la préparation d'une composition pharmaceutique à usage thérapeutique comprenant un ARN codant pour un gène d'intérêt.
